(19) Europäisches Patentamt
European Patent Office
Office européen des brevets

(11) Veröffentlichungsnummer: 0 416 373 A2

(12) **EUROPÄISCHE PATENTANMELDUNG**

(21) Anmeldenummer: 90116088.7

(22) Anmeldetag: 22.08.90

(51) Int. Cl.5: C07D 233/64, A61K 31/415, C07D 233/90, C07K 7/00

(30) Priorität: 04.09.89 CH 3192/89
12.07.90 CH 2336/90

(43) Veröffentlichungstag der Anmeldung: 13.03.91 Patentblatt 91/11

(84) Benannte Vertragsstaaten: AT BE CH DE DK ES FR GB GR IT LI LU NL SE

(71) Anmelder: F. HOFFMANN-LA ROCHE AG
Postfach 3255
CH-4002 Basel(CH)

(72) Erfinder: Branca, Quirico, Dr.
Lenzgasse 2
CH-4056 Basel(CH)
Erfinder: Neidhart, Werner, Dr.
Merzhauserstrasse 159A
W-7800 Freiburg im Breisgau(DE)
Erfinder: Ramuz, Henri, Prof. Dr.
Rheinparkstrasse 3
CH-4127 Birsfelden(CH)
Erfinder: Stadler, Heinz, Dr.
Waldhofstrasse 37
CH-4310 Rheinfelden(CH)
Erfinder: Wostl, Wolfgang, Dr.
Im Strick 2
W-7889 Grenzach-Wyhlen(DE)

(74) Vertreter: Kellenberger, Marcus, Dr. et al
Grenzacherstrasse 124 Postfach 3255
CH-4002 Basel(CH)

(54) **Aminosäurederivate.**

(57) Die Verbindungen der Formel

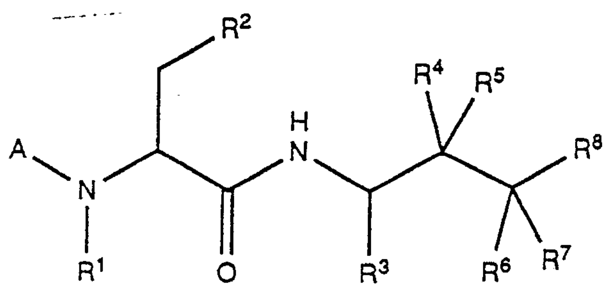

I

worin A, $R^1$, $R^2$, $R^3$, $R^4$, $R^5$, $R^6$, $R^7$ und $R^8$ die in Anspruch 1 angegebene Bedeutung besitzen, in Form von optisch reinen Diastereomeren, Diastereomerengemischen, diastereomeren Racematen oder Gemischen von diastereomeren Racematen sowie pharmazeutisch verwendbare Salze davon hemmen die Wirkung des natürlichen Enzyms Renin und können demnach in Form pharmazeutischer Präparate bei der Bekämpfung bzw. Verhütung von Bluthochdruck und Herzinsuffizienz verwendet werden. Sie können nach verschiedenen, an sich bekannten Verfahren hergestellt werden.

EP 0 416 373 A2

## AMINOSÄUREDERIVATE

Die vorliegende Erfindung betrifft Aminosäurederivate. Im speziellen betrifft sie Aminosäurederivate der allgemeinen Formel

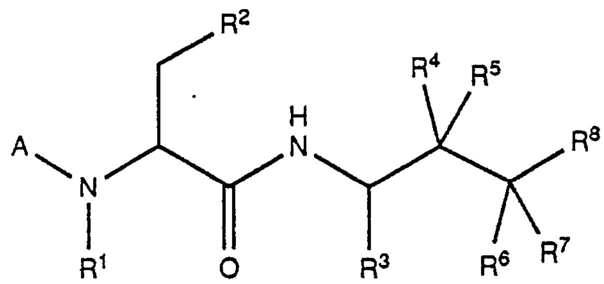

worin $R^1$ Wasserstoff oder Methyl, $R^2$ Aethyl, Propyl, Isopropyl, Thioalkyl, Imidazol-2-yl, Imidazol-4-yl, 5-Jodimidazol-4-yl, 5-Cyanimidazol-4-yl, N-Methylimidazol-2-yl, N-Methylimidazol-4-yl, C-methyliertes Imidazol-2-yl, C-methyliertes Imidazol-4-yl, N-substituiertes Imidazol-2-yl, N-substituiertes Imidazol-4-yl, Pyrazol-3-yl, Thiazol-4-yl, Thien-2-yl, Aethoxycarbonyl, Aminocarbonyl, Aminocarbonylmethyl, t-Butoxycarbonylmethyl, Benzyloxycarbonylmethyl oder t-Butoxy, $R^3$ Isobutyl, Cyclohexylmethyl, substituiertes Cyclohexylmethyl, Cyclohexenylmethyl, Cyclohexanonylmethyl, Bicyclo[3.1.0]hexylmethyl, Bicyclo[4.1.0] heptylmethyl, Cycloalkylalkylthiomethyl, 1,3-Dithiolan-2-ylmethyl, 1,3-Dithian-2-ylmethyl, Halobenzyl oder Benzyl, $R^4$ Wasserstoff und $R^6$ Wasserstoff oder Alkyl und $R^5$ und $R^7$ unabhängig voneinander je Hydroxy, gegebenenfalls durch Amino, Monoalkylamino, Dialkylamino, Alkanoylamino, Alkoxycarbonylamino, Alkylcarbonyloxy, Carboxy, Alkoxy oder Hydroxy ein- oder mehrfach substituiertes Alkylcarbonyloxy, oder Arylcarbonyloxy, Arylalkylcarbonyloxy, Cycloalkylcarbonyloxy, Heteroarylalkylcarbonyloxy, die Gruppe $-OSO_3H$ oder $-PO(OR)_2$, worin R Alkyl bedeutet, oder mit einer O-Schutzgruppe geschütztes Hydroxy, oder $R^5$ Amino oder unter physiologischen Bedingungen leicht spaltbares, durch eine Schutzgruppe substituiertes Amino und $R^7$ Hydroxy, Amino, unter physiologischen Bedingungen leicht spaltbares, durch eine Schutzgruppe substituiertes Amino oder Azido oder $R^5$ und $R^7$ zusammen mit einer cyclischen O-Schutzgruppe geschütztes Hydroxy, oder $R^4$ und $R^5$ zusammen eine Oxogruppe und $R^6$ Wasserstoff oder Fluor und $R^7$ Fluor, oder $R^4$ Wasserstoff, $R^5$ Hydroxy, $R^6$ Wasserstoff und $R^7$ Amino, unter physiologischen Bedingungen leicht spaltbares, durch eine Schutzgruppe substituiertes Amino, Azido oder Fluor oder $R^6$ und $R^7$ je Fluor oder zusammen Oximino oder eine Oxogruppe, $R^8$ Hydroxymethyl, Alkylhydroxymethyl, Cycloalkylhydroxymethyl, Cycloalkylaminomethyl, Cycloalkylcarbonyl, oder eine der Gruppen

$$\begin{array}{ccc} R^9 & & R^{10} \\ | & & | \\ -C & = & D \end{array}$$

(a)

und

$$\begin{array}{c} R^{11} \\ | \\ -C-R^{12} \\ | \\ R^{13} \end{array}$$

(b)

oder $R^7$ und $R^8$ zusammen 2-Oxo-3-cycloalkyl-oxazolidin-5-yl und A eine der Gruppen

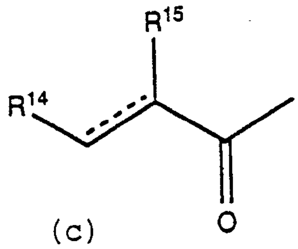

(c)

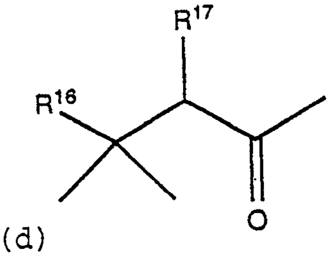

(d)

und $-(Y)_nZ$ (e)

bedeuten, worin D eine Methingruppe oder ein Stickstoffatom, $R^9$ Alkyl, Aryl oder Arylalkyl, $R^{10}$ Wasserstoff, Alkyl, Aryl oder Arylalkyl, oder $R^9$ und $R^{10}$ zusammen mit den beiden sie verbindenden Atomen, Aryl, Heteroaryl, Cycloalkenyl oder Heterocycloalkenyl, $R^{11}$ Wasserstoff oder Alkyl und $R^{12}$ und $R^{13}$ unabhängig voneinander je Alkyl, Aryl, Arylalkyl, Cycloalkyl oder die Gruppe

$-CH_2-X-R^{18}$ (f)

oder zusammen mit dem Kohlenstoffatom, an das sie gebunden sind, Cycloalkyl oder Heterocycloalkyl bedeuten, mit der Massgabe, dass, falls $R^{11}$ Alkyl bedeutet, $R^{12}$ und $R^{13}$ ebenfalls Alkyl bedeuten, die gestrichelte Linie eine zusätzliche Bindung bedeuten kann, $R^{14}$ und $R^{16}$ je Phenyl, substituiertes Phenyl, Benzyl, Naphthyl, Cyclohexyl, Thienyl oder Furyl und $R^{15}$ und $R^{17}$ je Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonyl hydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Cycloalkylsulfonylalkyl, Cycloalkylalkylsulfonylalkyl, substituiertes Phenylsulfonylalkyl, substituiertes Aminocarbonyloxy, Aminoalkylcarbonylalkyl, substituiertes Aminoalkylcarbonylalkyl, Heterocycloalkylcarbonyloxy, Guanidinium-methylsulfonat, substituiertes Aminoalkylsulfonylalkyl oder substituiertes Aminosulfonylalkyl bedeuten, mit der Massgabe, dass $R^{15}$ nicht Alkoxycarbonylamino oder Arylalkoxycarbonylamino bedeuten kann, wenn $R^{14}$ Phenyl, Halophenyl, Hydroxyphenyl, Methoxyphenyl, Benzyl, $\alpha$-Naphthyl, Cyclohexyl, Thienyl oder Furyl bedeutet, Y den N-terminal mit Z verbundenen, bivalenten Rest von gegebenenfalls N- und/oder $\alpha$-methyliertem Phenylalanin, Halophenylalanin, Cyclohexylalanin, Thienylalanin, Furylalanin, Pyridylalanin, Tyrosin, O-Methyltyrosin, $\alpha$-Naphthylalanin, Homophenylalanin oder 2-Hydroxy-3-amino-4-phenylbuttersäure, Z Wasserstoff, Acyl oder 1-Azabicyclo[2.2.2]octan-3-yl, n die Zahl 0 oder 1, ein Sauerstoff- oder Schwefelatom oder die Gruppe -NH- und $R^{18}$ Wasserstoff, Alkyl, Cycloalkyl, Arylalkyl, Cycloalkylalkyl, Alkylcarbonyl, Arylcarbonyl oder Arylalkylcarbonyl bedeuten, mit den Massgaben, dass

(i) falls $R^4$ und $R^6$ je Wasserstoff und $R^5$ und $R^7$ unabhängig voneinander je Hydroxy, gegebenenfalls durch Amino, Monoalkylamino, Dialkylamino, Alkanoylamino, Alkoxycarbonylamino, Carboxy, Alkoxy oder Hydroxy ein- oder mehrfach substituiertes Alkylcarbonyloxy oder eine O-Schutzgruppe oder zusammen eine cyclische O-Schutzgruppe bedeuten, dann A die Gruppe (c), worin R Cyclohexyl, Thienyl oder Furyl und/oder R Cycloalkylsulfonylalkyl, Cycloalkylalkylsulfonylalkyl, substituiertes Phenylsulfonylalkyl, substituiertes Aminocar bonyloxy, Aminoalkylcarbonylalkyl, substituiertes Aminocarbonylalkyl, substituiertes Aminoalkylsulfonylalkyl oder substituiertes Aminosulfonylalkyl bedeuten, die Gruppe (d) oder (e) bedeutet, worin n die Zahl 0 oder Y Thienylalanin, Furylalanin oder Pyridylalanin und/oder Z 1-Azabicyclo-[2.2.2]octan-3-yl und/oder $R^2$ Thioalkyl, N-substituiertes Imidazol-2-yl oder N-substituiertes Imidazol-4-yl und/oder $R^3$ substituiertes Cyclohexylmethyl, Cyclohexenylmethyl, Cyclohexanonylmethyl, Bicyclo[3.1.0]-hexylmethyl, Bicyclo[4.1.0]heptylmethyl, Cycloalkylalkylthiomethyl, 1,3-Dithiolan-2-ylmethyl oder 1,3-Dithian-2-ylmethyl und/oder $R^8$ Hydroxymethyl, Cycloalkylhydroxymethyl, Cycloalkylaminomethyl oder Cycloalkylcarbonyl bedeuten,

(ii) falls $R^4$ und $R^6$ Wasserstoff, $R^5$ Amino und $R^7$ Hydroxy, Amino oder Azido oder $R^4$ Wasserstoff, $R^5$ Hydroxy, $R^6$ Wasserstoff und $R^7$ Amino, Azido oder Fluor oder $R^6$ und $R^7$ je Fluor oder zusammen eine Oxogruppe oder $R^4$ und $R^5$ zuammen eine Oxogruppe und $R^6$ Wasserstoff oder Fluor und $R^7$ Fluor bedeuten, dann $R^8$ die Gruppe (a) oder (b) bedeutet,

(iii) falls $R^8$ Hydroxymethyl, Alkylhydroxymethyl, Cycloalkylhydroxymethyl, Cycloalkylaminomethyl oder Cycloalkylcarbonyl bedeutet, dann $R^4$ und $R^6$ je Wasserstoff und $R^5$ und $R^7$ je Hydroxy bedeuten, oder falls $R^7$ und $R^8$ zusammen 2-Oxo-3-cycloalkyl-oxazolidin-5-yl bedeuten, dann $R^4$ und $R^6$ je Wasserstoff und $R^5$ Hydroxy bedeuten, und

(iv) falls $R^8$ Cycloalkylaminomethyl bedeutet, dann $R^{15}$ von Alkylthioalkyl, Alkylsulfinylalkyl, Alkylsulfonylalkyl, substituiertem Aminoalkylsulfonylalkyl oder substituiertem Aminosulfonylalkyl verschieden ist, in

Form von optisch reinen Diastereomeren, Diastereomerengemischen, diastereomeren Racematen oder Gemischen von diastereomeren Racematen sowie pharmazeutisch verwendbare Salze dieser Verbindungen.

Diese Verbindungen sind neu und zeichnen sich durch wertvolle pharmakodynamische Eigenschaften aus.

Gegenstand der vorliegenden Erfindung sind die Verbindungen der Formel I und ihre pharmazeutisch verwendbaren Salze als solche und zur Anwendung als therapeutische Wirkstoffe, die Herstellung dieser Verbindungen, ferner diese enthaltende Arzneimittel und die Herstellung solcher Arzneimittel, sowie die Verwendung von Verbindungen der Formel I und ihrer pharmazeutisch verwendbaren Salze bei der Bekämpfung bzw. Verhütung von Krankheiten, bzw. bei der Verbesserung der Gesundheit, insbesondere bei der Bekämpfung bzw. Verhütung von Bluthochdruck und Herzinsuffizienz.

Die nachstehenden Definitionen der in der vorliegenden Beschreibung verwendeten allgemeinen Ausdrücke besitzen ihre Gültigkeit unabhängig davon, ob die fraglichen Ausdrücke allein oder in Kombination aufscheinen.

Der in der vorliegenden Beschreibung verwendete Ausdruck "Alkyl" bedeutet geradkettige und verzweigte, gesättigte Kohlenwasserstoffreste mit 1-8, vorzugsweise 1-4, Kohlenstoffatomen, wie Methyl, Aethyl, n-Propyl, Isopropyl, n-Butyl, Isobutyl, sec.-Butyl, t-Butyl, Pentyl, Hexyl und dergleichen. Der Ausdruck "Alkoxy" bedeutet Alkyläthergruppen, worin der Ausdruck "Alkyl" die obige Bedeutung hat, wie Methoxy, Aethoxy, Propoxy, Isopropoxy, Butoxy, Iso butoxy, sec.-Butoxy, t-Butoxy und dergleichen. Der Ausdruck "Cycloalkyl" bedeutet gesättigte, cyclische Kohlenwasserstoffreste mit 3-8, vorzugsweise 3-6, Kohlenstoffatomen, wie Cyclopropyl, Cyclobutyl, Cyclopentyl, Cyclohexyl und dergleichen. Der Ausdruck "Alkanoyl" bedeutet den Säurerest einer geradkettigen oder verzweigten Alkansäure mit 1-8, vorzugsweise 1-4, Kohlenstoffatomen, wie Formyl, Acetyl, Propionyl, Butyryl, Valeryl, Isovaleryl und dergleichen. Der Ausdruck "Heterocycloalkyl" betrifft in ähnlicher Weise gesättigte, 3-8-gliedrige, vorzugsweise 5- oder 6-gliedrige, cyclische Kohlenwasserstoffreste, in welchen eine oder zwei Methylengruppen durch ein oder zwei Sauerstoff-, Schwefel- oder gegebenenfalls durch Alkyl, Phenylalkyl, Alkylcarbonyl oder Alkylcarbonyloxy substituierte Stickstoffatome ersetzt sind, wie Piperidinyl, Pyrazinyl, N-Benzylpyrazinyl, Morpholinyl, N-Methylpiperidinyl, N-Benzylmorpholinyl und dergleichen. Der Ausdruck "Cycloalkenyl" betrifft einen ungesättigten Kohlenwasserstoffrest mit 3-8, vorzugsweise 3-6, Kohlenstoffatomen, wie 1-Cyclohexenyl, 1,4-Cyclohexadienyl und dergleichen. Der Ausdruck "Heterocycloalkenyl" betrifft in ähnlicher Weise ungesättigte, 3-8-gliedrige, vorzugsweise 5- oder 6-gliedrige, cyclische Kohlenwasserstoffreste, in welchen eine oder zwei Methylengruppen durch ein oder zwei Sauerstoff-, Schwefel- oder gegebenenfalls durch Alkyl, Phenylalkyl, Alkylcarbonyl oder Alkylcarbonyloxy substituierte Stickstoffatome ersetzt sind, wie Dihydropyranyl, Dihydropyridyl, Dihydrothienyl und dergleichen. Der Ausdruck "Aryl" bezeichnet einen gegebenenfalls durch Alkyl, Alkoxy, Alkylcarbonyloxy, Amino, Alkylamino, Dialkylamino, Alkylcarbonylamino, Hydroxy, Halogen, Trifluormethyl oder Nitro ein- oder mehrfach substituierten mono- oder bicyclischen aromatischen Kohlenwasserstoffrest mit 6-14 Kohlenstoffatomen, wie Phenyl, α- oder β-Naphthyl, Indenyl, Anthryl oder Phenanthryl und dergleichen. Der Ausdruck "Heteroaryl" bezeichnet einen gegebenenfalls an einem Stickstoffatom durch Alkyl, Phenyl oder Phenylalkyl und/oder an einem oder mehreren Kohlenstoffatomen durch Alkyl, Phenyl, Phenylalkyl, Halogen, Hydroxy, Alkoxy, Phenylalkoxy oder Oxo substituierten und teilweise gesättigten mono- oder bicyclischen aromatischen Kohlenwasserstoffrest, in welchem ein oder mehrere Kohlenstoffatome durch ein bis zwei Stickstoffatome und/oder ein Sauerstoff- oder Schwefelatom ersetzt sind, wie Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzanneliertes cyclopenta-, cyclohexa- oder cyclohepta-anneliertes Derivat davon, z.B. 2- oder 3-Pyrrolyl, Phenylpyrrolyl, z.B. 4- oder 5-Phenyl-2-pyrrolyl, 2-Furyl, 2-Thienyl, 2-Imidazolyl, 2-, 3- oder 4-Pyridyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2-indolyl, 1-Benzyl-3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl, β-Carbolin-3-yl und dergleichen. Der Ausdruck "Arylalkyl" bezeichnet geradkettige oder verzweigte Alkylgruppen, worin ein oder mehrere Wasserstoffatome durch Arylgruppen ersetzt sind, wie Benzyl, Diphenylmethyl, Trityl, α- oder β-Naphthylmethyl, 2-Phenyläthyl, 3-Phenyl-2-propyl, 4-Phenyl-3-butyl, 2- (α- oder β-Naphthyl)äthyl, 3-α-Naphthyl-2-propyl, 4-α-Naphthyl-3-butyl und dergleichen, wobei der aromatische Rest jeweils wie oben angegeben ein- oder mehrfach substituiert sein kann. Der Ausdruck "N-substituiertes Imidazol-2-yl" bzw. "N-substituiertes Imidazol-4-yl" bezeichnet entsprechende Imidazolylreste, die an einem der Stickstoffatome durch Alkoxycarbonyl, Alkenyloxycarbonyl, Arylalkoxycarbonyl, Arylsulfonyl, Alkylcarbonyloxyalkoxycarbonyl, Alkylcarbonyloxyalkyl, Alkoxycarbonylalkylidenalkyl, Arylalkyl oder 2,4-Dinitrophenyl und dergleichen, substituiert

sind, wie Aethoxycarbonyl-, t-Butoxycarbonyl-, Allyloxycarbonyl-, Benzyloxycarbonyl-, 9-fluorenylmethoxycarbonyl-, p-Toluolsulfonyl, 1-Methylcar bonyloxyäthoxycarbonyl-, t-Butylcarbonyloxymethyl, Methoxycarbonylmethylidenäthyl-, Triphenylmethyl oder 2,4-Dinitrophenyl substituiertes Imidazol-2-yl. Der Ausdruck "substituiertes Cyclohexylmethyl" bezeichnet durch Hydroxy oder Halogen, insbesondere Fluor, und dergleichen substituiertes Cyclohexylmethyl, wie 4-Hydroxycyclohexylmethyl, 4-Fluorcyclohexylmethyl, 4,4-Difluorcyclohexylmethyl und dergleichen. Der Ausdruck "substituiertes Phenyl" bezeichnet gegebenenfalls durch Alkyl, Alkoxy, Alkoxycarbonyl, Alkylcarbonyloxy, Hydroxy, Halogen oder Trifluormethyl ein- oder mehrfach substituiertes Phenyl, wie 4-Hydroxyphenyl, 4-Methoxyphenyl, 4-Methylphenyl, 4-Chlorphenyl und dergleichen. Der Ausdruck "substituiertes Amino" bedeutet eine durch Alkyl, Arylalkyl, Alkylcarbonyl, Alkoxycarbonyl oder Arylalkoxycarbonyl mono- oder di-substituierte oder eine gegebenenfalls durch ein Sauerstoff-, Schwefel- oder gegebenenfalls Alkyl-, Phenylalkyl-, Alkylcarbonyl- oder Alkylcarbonyloxy-substituiertes Stickstoffatom unterbrochene $C_3$-$C_6$-Alkylen disubstituierte Aminogruppe. Der Ausdruck $C_3$-$C_6$-Alkylen" bezeichnet geradkettige oder verzweigte Reste mit 3-6 Kohlenstoffatomen, wie Trimethylen, Propylen, Tetramethylen, Pentamethylen, Hexamethylen und dergleichen. Der Ausdruck "Acyl" betrifft die Acylgruppe einer Carbonsäure, einer gegebenenfalls N-substituierten Carbaminsäure, einer Sulfonsäure oder einer gegebenenfalls N-substituierten Amidosulfonsäure, insbesondere solche mit den Teilformeln $R^a$-CO-, $(R^a)(R^a)$N-CO-, $R^a$-$SO_2$-, oder $(R^a)(R^a)$N-$SO_2$, worin $R^a$ Wasserstoff, einen unsubstituierten oder substituierten, gesättigten, gegebenenfalls mit Hydroxy und/oder Amino, Monoalkylamino, Dialkylamino, Alkanoylamino, Alkoxycarbonylamino, Arylalkoxycarbonylamino oder substituiertem Aminocarbonyl funktionalisierten aliphatischen, cycloaliphatischen, cycloaliphatisch-aliphatischen Kohlenwasserstoffrest mit bis zu 10, vorzugsweise 6, Kohlenstoffatomen, einen unsubstituierten oder substituierten, gegebenenfalls mit Hydroxy und/oder Amino, Monoalkylamino, Dialkylamino, Alkanoylamino, Alkoxycarbonyl amino, Arylalkoxycarbonylamino oder substituiertem Aminocarbonyl funktionalisierten aromatischen, heteroaromatischen, aromatisch-aliphatischen oder heteroaromatisch-aliphatischen Kohlenwasserstoffrest mit bis zu 18, vorzugsweise 10, Kohlenstoffatomen oder einen unsubstituierten oder substituierten, gesättigten 5- oder 6-gliedrigen Heterocyclus bedeutet. Der Ausdruck "Acyl" betrifft auch den einwertigen, über die $\alpha$- oder gegebenenfalls $\omega$-Carboxylgruppe gebundenen Rest einer Aminosäure. Der Ausdruck "O-Schutzgruppe" bedeutet eine mit Base oder vorzugsweise mit Säure abspaltbare Schutzgruppe, wie der Tetrahydropyranyl- oder Methoxymethylrest, ein Alkylcarbonyloxymethyl- oder Alkoxycarbonyloxymethylrest und dergleichen. Beispiele von "cyclischen O-Schutzgruppen" sind Acetale, Ketale und cyclische Ester, wie das Ketal von Aceton, das Acetal von Pivalinaldehyd oder Benzaldehyd oder das cyclische Carbonat. Der Ausdruck "unter physiologischen Bedingungen leicht spaltbares, durch eine Schutzgruppe substituiertes Amino" bedeutet Formylamino, Alkylcarbonylamino, wie Acetylamino oder Pivaloylamino, Hydroxyalkylcarbonylamino, wie Hydroxyacetamino, Aminoalkylcarbonylamino, wie Aminoacetylamino, Arylcarbonylamino, wie Benzoylamino, Alkoxycarbonylamino, wie Methoxy-oder tert-Butoxycarbonylamino, Arylalkoxycarbonylamino, wie Benzyloxycarbonylamino, Arylmethylamino, wie Diphenylmethylamino oder Tritylamino, und dergleichen.

Ein unsubstituierter oder substituierter, gesättigter, aliphatischer, cycloaliphatischer oder cycloaliphatisch-aliphatischer Kohlenwasserstoffrest $R^a$ ist beispielsweise unsubstituiertes oder substituiertes Alkyl, Mono-, Bi- oder Tricycloalkyl oder Cycloalkylalkyl. "Substituiertes Alkyl" bedeutet einen Alkylrest, in welchem ein oder mehrere Wasserstoffatome durch Hydroxy, Alkoxy, Alkylcarbonyloxy, Halogen, Amino oder Oxo substituiert sein können, wobei die Substituenten nur dann in 1-Stellung des Alkylrestes stehen, wenn dieser in der Teilformel $R^a$-CO- vorhanden ist.

Beispiele von substituiertem Alkyl sind 2-Hydroxyäthyl, Methoxymethyl, 2-Methoxyäthyl, Acetoxymethyl, 2-Acetoxyäthyl, Chlormethyl, Brommethyl, 2-Chlor- oder 2-Bromäthyl, 2-Oxopropyl, 2-Oxobutyl.

Der Ausdruck "Bicycloalkyl" betrifft bicyclische gesättigte Kohlenwasserstoffreste mit 5-10, vorzugsweise 6-9, Kohlenstoffatomen, wie Bicyclo-[3.1.O]hex-1-yl, Bicyclo[3.1.O]hex-2-yl, Bicyclo[3.1.O]hex-3-yl, Bicyclo[4.1.O]hept-1-yl, Bicyclo[4.1.O]hept-4-yl, Bicyclo[2.2.1]hept-2-yl, Bicyclo[3.2.]oct-2-yl, Bicyclo[3.3.O]-oct-3-yl, Bicyclo[3.3.1]non-9-yl, $\alpha$- oder $\beta$-Decahydronaphthyl und dergleichen.

Der Ausdruck "Tricycloalkyl" betrifft einen tricyclischen gesättigten Kohlenwasserstoffrest mit 8-10 Kohlenstoffatomen, wie 1-Adamantyl.

Beispiele für Cycloalkylalkyl sind Cyclopropylmethyl, Cyclobutylmethyl, Cyclopentylmethyl, Cyclohexylmethyl und dergleichen.

Die genannten cycloaliphatischen und cycloaliphatisch-aliphatischen Reste können durch die gleichen Substituenten wie Alkyl substituiert sein.

Ein gegebenenfalls substituierter aromatischer oder aromatisch-aliphatischer Kohlenwasserstoffrest ist beispielsweise unsubstituiertes oder substituiertes Aryl oder Arylalkyl.

In einem heteroaromatischen oder heteroaromatisch-aliphatischen Kohlenwasserstoffrest ist der Heterocyclus mono-, bi- oder tricyclisch und enthält ein bis zwei Stickstoffatome und/oder ein Sauerstoff- oder

Schwefelatom und ist mit einem seiner Ring-Kohlenstoffatome mit der Gruppe -CO-, >N-CO-, $-SO_2$ oder $>N-SO_2-$ verknüpft. Beispiele solcher heteroaromatischer Kohlenwasserstoffreste sind Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzanneliertes cyclopenta-, cyclohexa- oder cyclohepta-anneliertes Derivat dieser Reste. Der heteroaromatische Rest kann an einem Stickstoffatom durch Alkyl, Phenyl oder Phenylalkyl, z.B. Benzyl, und/oder an einem oder mehreren Kohlenstoffatomen durch Alkyl, Phenyl, Phenylalkyl, Halogen, Hydroxy, Alkoxy, Phenylalkoxy oder Oxo substituiert und teilweise gesättigt sein. Beispiele solcher heteroaromatischer Reste sind 2- oder 3-Pyrrolyl, Phenylpyrrolyl, z.B. 4- oder 5-Phenyl-2-pyrrolyl, 2-Furyl, 2-Thienyl, 2-Imidazolyl, 2-, 3- oder 4-Pyridyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2-indolyl, 1-Benzyl-3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl, β-Carbolin-3-yl und dergleichen.

Beispiele heteroaromatisch-aliphatischer Kohlenwasserstoffreste sind 2- oder 3-Pyrrolylmethyl, 2-, 3- oder 4-Pyridylmethyl, 2- (2-, 3- oder 4-Pyridyl)äthyl, 4-Imidazolylmethyl, 2-(4-imidazolyl)äthyl, 2-Indolylmethyl, 3-Indolylmethyl, 2-(3-Indolyl)äthyl, 2-Chinolylmethyl und dergleichen.

Ein gesättigter 5- oder 6-gliedriger Heterocyclus hat mindestens ein Kohlenstoffatom, 1-3 Stickstoffatome und/oder 1-2 Sauerstoff- und/oder Schwefelatome als Ringglieder und ist mit einem seiner Ringkohlenstoffatome mit der Gruppe -CO-, >N-CO-, $-SO_2-$ oder $>N-SO_2-$ verknüpft. Der Heterocyclus kann an einem seiner Kohlenstoffatome oder an einem Ringstickstoffatom durch Alkyl, z.B. Methyl oder Aethyl, Phenyl oder Phenylalkyl, z.B. Benzyl, oder an einem seiner Kohlenstoffatome durch Hydroxy oder Oxo substituiert und/oder an zwei benachbarten Kohlenstoffatomen benzanneliert sein. Beispiele solcher Heterocyclen sind Pyrrolidin-3-yl, 4-Hydroxypyrrolidin-2-yl, 5-Oxopyrrolidin-2-yl, Piperidin-2-yl, Piperidin-3-yl, 1-Methylpiperidin-2-yl, 1-Methylpiperidin-3-yl, 1-Methylpiperidin-4-yl, Morpholin-2-yl, Morpholin-3-yl, Thiomorpholin-2-yl, Thiomorpholin-3-yl, 1,4-Dimethylpiperazin-2-yl, 2-Indolinyl, 3-Indolinyl, 1,2,3,4-Tetrahydrochinol-2-, -3- oder -4-yl, 1,2,3,4-Tetrahydroisochinol-1-, -3- oder -4-yl, 1-Oxo-1,2,3,4-tetrahydroisochinol-3-yl und dergleichen.

Als Rest einer über die α- oder gegebenenfalls ω-Carboxylgruppe gebundenen Aminosäure kommen ein- und zweibasische α- und β-Aminosäuren mit der L- oder D-Konfiguration in Frage, welche gegebenenfalls in α-Stellung alkyliert und/oder am N-Atom aminoalkyliert sind, und deren Aminogruppe(n) und/oder zweite Carboxylgruppe gegebenenfalls geschützt sind. Geeignete Schutzgruppen sind in beiden Fällen solche, die in der Peptidchemie üblich sind, wie tert-Butoxycarbonyl, Benzyloxycarbonyl, tert-Butylester, Benzylester und dergleichen. Im weiteren kann bei Vorhandensein einer zweiten Carboxylgruppe diese intramolekular amidiert sein. Beispiele solcher Aminosäuren sind Prolin, Pyroglutaminsäure, Aminobuttersäure, Aminoäthylglycin, Aminoäthylasparaginsäureester und dergleichen.

Der Ausdruck "pharmazeutisch verwendbare Salze" umfasst Salze mit anorganischen oder organischen Säuren, wie Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfonsäure, p-Toluolsulfonsäure und dergleichen, oder, für den Fall, dass $R^5$ und/oder $R^7$ die Gruppe $-OSO_3H$ bedeuten, auch mit anorganischen oder organischen Basen, wie Natrium- oder Kaliumhydroxyd, Ammoniak, Triäthylamin, Diisopropyläthylamin, Pyridin und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Die Verbindungen der Formel I besitzen mindestens drei asymmetrische Kohlenstoffatome und liegen deshalb in Form von optisch reinen Diastereomeren, Diastereomerengemischen, diastereomeren Racematen oder Gemischen von diastereomeren Racematen vor. Die vorliegende Erfindung umfasst alle Formen. Diastereomerengemische, diastereomere Racemate oder Gemische von diastereomeren Racematen können nach üblichen Methoden aufgetrennt werden, z.B. durch Säulenchromatographie, Dünnschichtchromatographie, HPLC und dergleichen.

Eine spezielle Gruppe von Verbindungen der Formel I sind solche, worin $R^2$ von Thioalkyl, Aminocarbonyl und Aminocarbonylmethyl, $R^3$ von Cycloalkylalkylthiomethyl, $R^{15}$ und $R^{17}$ von Cycloalkylsulfonylalkyl, Cycloalkylalkylsulfonylalkyl, substituiertem Phenylsulfonylalkyl und Aminoalkylcarbonylalkyl, Y von Pyridylalanin und Z von 1-Azabicyclo[2.2.2]octan-3-yl verschieden sind.

Bevorzugt sind solche Verbindungen der Formel I, worin $R^1$ Wasserstoff bedeutet. $R^2$ bedeutet vorzugsweise Imidazol-2-yl, Imidazol-4-yl, Thiazol-4-yl, Aminocarbonyl oder Aminocarbonylmethyl, besonders bevorzugt Imidazol-4-yl. Weiter sind solche Verbindungen der Formel I bevorzugt, worin $R^3$ Cyclohexylmethyl, substituiertes Cyclohexylmethyl oder Cyclohexenylmethyl, besonders bevorzugt Cyclohexylmethyl oder 4,4-Difluorcyclohexylmethyl, bedeutet. $R^4$ und $R^6$ bedeuten vorzugsweise je Wasserstoff. $R^5$ bedeutet

vorzugsweise Hydroxy, Amino oder durch Amino monosubstitu iertes Alkylcarbonyloxy, besonders bevorzugt Hydroxy oder Aminomethylcarbonyloxy. Weiter sind auch solche Verbindungen der Formel I bevorzugt, worin $R^7$ Hydroxy, Amino, durch Amino monosubstituiertes Alkylcarbonyloxy, Azido oder Fluor, besonders bevorzugt Hydroxy oder Aminomethylcarbonyloxy, bedeutet. Auch bevorzugt sind solche Verbindungen der Formel I, worin $R^8$ Alkylhydroxymethyl, Cycloalkylhydroxymethyl oder die Gruppe (b), besonders bevorzugt die Gruppe (b), bedeutet. Ebenfalls bevorzugt sind die Verbindungen der Formel I, worin $R^7$ und $R^8$ zusammen 2-Oxo-3-cycloalkyl-oxazolidin-5-yl bedeuten. Im weiteren sind auch solche Verbindungen der Formel I bevorzugt, worin A die Gruppe (c) oder (e), besonders bevorzugt die Gruppe (c), bedeutet. Die bevorzugte Bedeutung von $R^{11}$ ist Wasserstoff. $R^{12}$ und $R^{13}$ bedeuten vorzugsweise je Alkyl oder zusammen mit dem Kohlenstoffatom, an das sie gebunden sind, Cycloalkyl, besonders bevorzugt Cyclopropyl oder Cyclobutyl. $R^{14}$ bedeutet vorzugsweise Phenyl oder substituiertes Phenyl, besonders bevorzugt Phenyl. Die bevorzugte Bedeutung von $R^{15}$ ist Alkylcarbonylalkyl, Heterocycloalkylcarbonylalkyl, substituiertes Aminocarbonylalkyl, Alkylsulfonylalkyl, Cycloalkylsulfonylalkyl, Cycloalkylalkylsulfonylalkyl, substituiertes Aminoalkylcarbonylalkyl, Heterocycloalkylcarbonyloxy, Aminoalkylsulfonylalkyl oder substituiertes Aminosulfonylalkyl, vorzugsweise $C_1$-$C_4$-Alkylcarbonylmethyl, Heterocycloalkylcarbonylmethyl, substituiertes Aminocarbonylmethyl, $C_1$-$C_4$- -Alkylsulfonylmethyl, $C_3$-$C_6$-Cycloalkylsulfonylmethyl, $C_3$-$C_6$-Cycloalkyl-$C_1$-$C_4$-alkylsulfonylmethyl, substituiertes Amino-$C_1$-$C_4$-alkylcarbonylmethyl, Heterocycloalkylcarbonyloxy, Amino-$C_1$-$C_4$-alkylsulfonylmethyl oder substituiertes Amino-$C_1$-$C_4$-alkylsulfonylmethyl. Falls A die Gruppe (e) bedeutet, so sind diejenigen Verbindungen der Formel I bevorzugt, worin Y den N-terminal mit Z verbundenen, bivalenten Rest von Phenylalanin oder O-Methyltyrosin bedeutet. Z bedeutet vorzugsweise die Gruppe $R^a$-CO-, worin $R^a$ einen unsubstituierten oder substituierten, gesättigten, gege benenfalls mit Hydroxy und/oder Amino, Monoalkylamino, Dialkylamino, Alkanoylamino, Alkoxycarbonylamino, Arylalkoxycarbonylamino oder substituiertem Aminocarbonyl funktionalisierten aliphatischen Kohlenwasserstoffrest mit bis zu 10 Kohlenstoffatomen oder einen unsubstituierten oder substituierten heterаromatischen Kohlenwasserstoffrest mit bis zu 18 Kohlenstoffatomen bedeutet, ganz besonders bevorzugt die Gruppe $R^a$-CO-, worin $R^a$ einen gesättigten, gegebenenfalls mit Hydroxy und/oder Amino, Alkanoylamino, Alkoxycarbonylamino, Arylalkoxycarbonylamino oder substituiertem Aminocarbonyl funktionalisierten aliphatischen Kohlenwasserstoffrest mit bis zu 6 Kohlenstoffatomen oder einen gegebenenfalls durch Alkyl, Halogen, Hydroxy oder Alkoxy substituierten heteroaromatischen Rest mit bis 10 Kohlenstoffatomen bedeutet. Eine bevorzugte Bedeutung von Z ist auch der einwertige, über die Carboxylgruppe gebundene Rest von Prolin, Pyroglutaminsäure, α-Methylalanin, Aminoäthylglycin, D-Alanin, β-Alanin oder β,β-Dimethylalanin.

Aus dem obigen folgt, dass solche Verbindungen der Formel I ganz besonders bevorzugt sind, worin A, $R^1$ , $R^2$ , $R^3$ $R^4$, $R^5$, $R^6$, $R^7$ und $R^8$ die oben angegebenen bevorzugten Bedeutungen besitzen, insbesondere solche, worin $R^1$ Wasserstoff, $R^2$ Imidazol-4-yl, $R^3$ Cyclohexylmethyl oder 4,4-Difluorcyclohexylmethyl, $R^4$ und $R^6$ je Wasserstoff, $R^5$ und $R^7$ je Hydroxy oder Aminomethylcarbonyloxy, $R^8$ die Gruppe (b), R Wasserstoff, $R^{12}$ und $R^{13}$ zusammen mit dem Kohlenstoffatom, an das sie gebunden sind, Cyclopropyl oder Cyclobutyl, $R^{14}$ Phenyl und $R^{15}$ $C_1$-$C_4$-Alkylcarbonylmethyl, Heterocycloalkylcarbonylmethyl, substituiertes Aminocarbonylmethyl, $C_1$-$C_4$-Alkylsulfonylmethyl, $C_3$-$C_6$-Cycloalkylsulfonylmethyl, $C_3$-$C_6$-Cycloalkyl-$C_1$-$C_4$-Alkylsulfonylmethyl, substituiertes Amino-$C_1$-$C_4$-alkylcarbonylmethyl, Heterocycloalkylcarbonyloxy, Amino-$C_1$-$C_4$-alkylsulfonylmethyl oder substituiertes Amino-$C_1$-$C_4$-alkylsulfonylmethyl bedeuten.

Speziell bevorzugte Verbindungen der Formel I sind:

- (S)-α-[(S)-α-[[[(2-Amino-1,1-dimethyläthyl)sulfonyl]methyl]hydrocinnamamido]-N-[(1S,2R,3S)-1-(cyclohexylmethyl)-3-cyclopropyl-2,3-dihydroxypropyl]imidazol-4-propionamid-dihydrochlorid,
- (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]-5-cyanimidazol-4-propionamid,
- (S)-α-[(S)-α-[(tert-Butylsulfonyl)methyl]hydrocinnamamido]-N-[(1S,2R,3S)-3-cyclopropyl-1-[(4,4-difluorcyclohexyl)methyl]-2,3-dihydroxypropyl]imidazol-4-propionamid,
- (S)-α-[(S)-α-[(tert-Butylsulfonyl)methyl]hydrocinnamamido]-N-[(1S,2R,3S)3-cyclopropyl-1-(p-fluorbenzyl)-2,3-dihydroxypropyl]imidazol-4-propionamid und
- (S)-α-[(S)-α-[[(2-Amino-2-methylpropyl)sulfonyl]methyl]hydrocinnamamido]-N-[(1S,2R,3S)-1-(cyclohexylmethyl)-3-cyclopropyl-2,3-dihydroxypropyl]imidazol-4-propionsäureamiddiacetat.

Weitere speziell bevorzugte Verbindungen der Formel I sind:

- (S)-N-[(1S,2R,3S)-3-Azido-1-(cyclohexylmethyl)-3-cyclopropyl-2-hydroxypropyl]-α-[(S)-α-(tert-butylsulfonyl)-methyl]hydrocinnamamido] imidazol-4-propionamid,
- (S)-α-[(S)-α-[(tert-Butylsulfonyl)methyl]hydrocinnamamido]-N-[(1S,2R)-1-(cyclohexylmethyl)-2-[(R oder S)-3-cyclopropyl-2-oxo-5-oxazolidinyl]-2-hydroxyäthyl]imidazol-4-propionamid,
- (S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]phenäthyl 1-piperidincarboxylat,

- (S)-α-[[(S)-1-[[(1S,2R,3S)-1-(cyclohexylmethyl)-3-cyclopropyl-2,3-dihydroxypropyljcarbamoyl]-2- imidazol-4-yläthyl]carbamoyl]phenäthyl 4-morpholincarboxylat,
- (R)-2-[[(S)-α-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]phenäthyl]carbamoyl]-1-pyrrolidincarbonsäure-tert-butylester,
- (S)-α-[(S)-α-[(tert-Butylsulfonyl)methyl]hydrocinnamamido]-N-[(1S,2R,3S)-1-(cyclohexylmethyl)-3-cyclopropyl-3-fluor-2-hydroxypropyl]imidazol-4-propionamid,
- tert-Butyl [1-[(S)-α-[[(S)-1-[[(1S,2R,3S)-1-(cyclohexylmethyl)-3-cyclopropyl-2,3-dihydropropyl]carbamoyl]-2-imidazol-4-yläthyl]carbamoyl]phenäthyl]carbamoyl]-1-methyläthyl]carbamat,
- Di-tert-butyl N-[(S)-1-(tert-butoxycarbonyl)-2-[[(S)-α-[[(S)-1-[[(1S,2R,3S)-1-(cyclohexylmethyl)-3-cyclopropyl2,3-dihydroxypropyl]carbamoyl]-2-imidazol-4-yläthyl]carbamoyl]phenäthyl]carbamoyl]äthyl]-äthylendicarbamat,
- (S)-α-[(S)-α-[(tert-Butylsulfonyl)methyl]hydrocinnamamido]-N-[(1S,2R,3RS)-1-(cyclohexylmethyl)-2,3,4-trihydroxybutyl]imidazol-4-propionamid,
- (S oder R)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3-phenylpropansulfonsäure-guanidinsalz,
- Di-tert-butyl N-[[[(S)-α-[[(S)-1-[[(1S,2R,3S)-1-(cyclohexylmethyl)-3-cyclopropyl-2,3-dihydroxypropyl]-carbamoyl]-2-imidazol-4-yläthyl]carbamoyl]phenäthyl]carbamoyl]methyl]äthylendicarbamat,
- N-[(S)-1-[[(1S,2R,3S)-1-(Cyclohexylmethyl)-3-cyclopropyl-2,3-dihydroxypropyl]carbamoyl]-2-imidazol-4-yläthyl]indol-2-carboxamid,
- (S)-N-[(1S,2R,3S)-1-(Cyclohexylmethyl)-3-cyclopropyl2,3-dihydroxypropyl]-α-(2,2-dibenzylacetamido)-imidazol-4-propionamid,
- (S)-α-[(S)-α-[2-[(2-Aminoäthyl)amino]acetamido]hydrocinnamamido]-N-[(1S,2R,3S)-1-(cyclohexylmethyl)-3-cyclopropyl-2,3-dihydroxypropyl]imidazol-4-propionamid,
- N-[(S)-1-[[(1S,2R,3S)-1-(Cyclohexylmethyl)-3-cyclo propyl-2,3-dihydroxypropyl]carbamoyl]-2-imidazol-4-yl-äthyl]-2-benzimidazolcarboxamid und
- (S)-α-[(S)-α-[(tert-Butylsulfonyl)methyl]hydrocinnamamido]-N-[(1S,2R,3R oder S,4R oder S)-1-(cyclohexylmethyl)-2,3,4-trihydroxyhexyl]imidazol-4-propionamid.

Verbindungen der Formel I in Form von optisch reinen Diastereomeren, Diastereomerengemischen, diastereomeren Racematen oder Gemischen von diastereomeren Racematen sowie pharmazeutisch verwendbare Salze davon können hergestellt werden, indem man

a) eine Verbindung der allgemeinen Formel

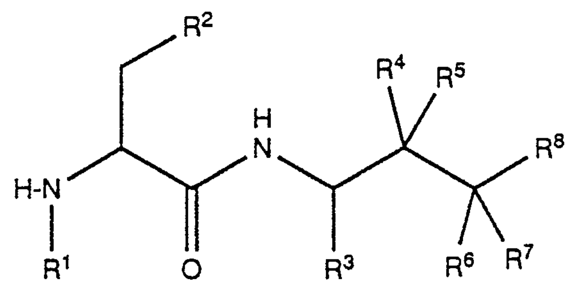

II

worin $R^1$, $R^2$, $R^3$, $R^4$, $R^5$, $R^6$, $R^7$ und $R^8$ die oben angegebene Bedeutung besitzen, mit einem die Gruppe

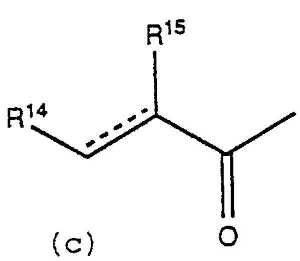

(c)

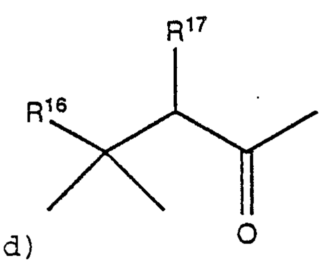

(d)

oder $-(Y)_nZ$ (e)

worin $R^{14}$, $R^{15}$, $R^{16}$, $R^{17}$ Y, Z, n und die gestrichelte Linie die oben angegebene Bedeutung besitzen,

abgebenden Acylierungsmittel umsetzt, oder
b) eine Verbindung der allgemeinen Formel

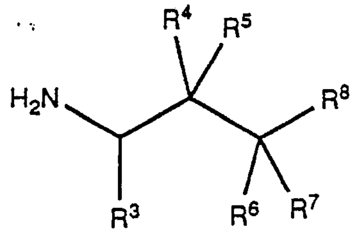

III

worin $R^3$, $R^4$, $R^5$, $R^6$, $R^7$ und $R^8$ die oben angegebene Bedeutung besitzen, mit einer Verbindung der allgemeinen Formel

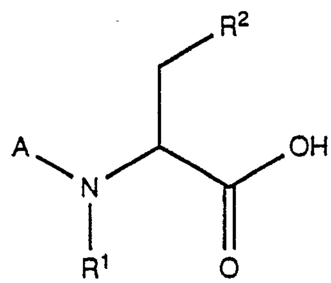

IV

worin $R^1$, $R^2$ und A die oben angegebene Bedeutung besitzen, oder einem aktivierten Derivat davon umsetzt, oder
c) zur Herstellung einer Verbindung der Formel I, worin A die Gruppe (e) und Z den einwertigen, über die Carboxylgruppe gebundenen Rest einer Aminosäure bedeuten und die übrigen Symbole die oben angegebene Bedeutung besitzen, eine Verbindung der Formel I, worin Z Wasserstoff bedeutet und die übrigen Symbole die oben angegebene Bedeutung besitzen, mit einer Aminosäure umsetzt, oder
d) zur Herstellung einer Verbindung der Formel I, worin $R^5$ und/oder $R^7$ gegebenenfalls durch Amino, Monoalkylamino, Dialkylamino, Alkanoylamino, Alkoxycarbonylamino, Alkylcarbonyloxy, Carboxy, Alkoxy oder Hydroxy ein- oder mehrfach substituiertes Alkylcarbonyloxy, oder Arylcarbonyloxy, Arylalkylcarbonyloxy, Cycloalkylcarbonyloxy, Heteroarylalkylcarbonyloxy, die Gruppe $-OSO_3H$ oder $-PO(OR)_2$, worin R Alkyl bedeutet, oder mit einer O-Schutzgruppe geschütztes Hydroxy oder unter physiolo gischen Bedingungen leicht spaltbares, durch eine Schutzgruppe substituiertes Amino oder zusammen mit einer cyclischen O-Schutzgruppe geschütztes Hydroxy bedeuten und die übrigen Symbole die oben angegebene Bedeutung besitzen, eine Verbindung der Formel I, worin $R^5$ und/oder $R^7$ Hydroxy oder Amino bedeuten und die übrigen Symbole die oben angegebene Bedeutung besitzen, mit einem gegebenenfalls durch Amino, Monoalkylamino, Dialkylamino, Alkanoylamino, Alkoxycarbonylamino, Alkylcarbonyloxy, Carboxy, Alkoxy oder Hydroxy ein- oder mehrfach substituierten Alkanoylierungsmittel oder einem Aroylierungs-, Arylalkanoylierungs-, Cycloalkanoylierungs- oder Heteroarylalkanoylierungsmittel, einem Sulfatierungs- oder Phosphorylierungsmittel oder mit einem eine N- und/oder O-Schutzgruppe oder cyclische O-Schutzgruppe bildenden Mittel umsetzt, oder
e) zur Herstellung einer Verbindung der Formel I, worin $R^2$ N-substituiertes, gegebenenfalls C-methyliertes Imidazol-2-yl oder N-substituiertes, gegebenenfalls C-methyliertes Imidazol-4-yl bedeutet und die übrigen Symbole die oben angegebene Bedeutung besitzen, eine Verbindung der Formel I, worin $R^2$ gegebenenfalls C-methyliertes Imidazol-2-yl oder gegebenenfalls C-methyliertes Imidazol-4-yl bedeutet und die übrigen Symbole die oben angegebene Bedeutung besitzen, mit einem geeigneten Acylierungs-, Alkylierungs-, Arylalkylierungs- bzw. Arylierungsmittel umsetzt, oder
f) zur Herstellung einer Verbindung der Formel I, worin A eine freie Aminogruppe enthält und/oder $R^2$ Imidazol-2-yl, Imidazol-4-yl, C-methyliertes Imidazol-2-yl, C-methyliertes Imidazol-4-yl oder Pyrazol-3-yl und/oder $R^5$ und/oder $R^7$ Amino bedeuten und/oder $R^8$ eine primäre oder sekundäre Aminogruppe enthält, aus einer Verbindung der allgemeinen Formel

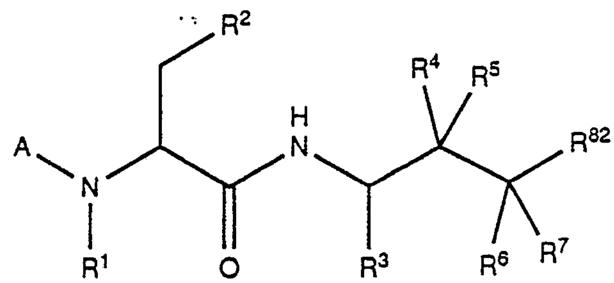

Ia

worin $R^{82}$ die gleiche Bedeutung wie $R^8$ hat, jedoch noch zusätzlich N-geschütztes Cycloalkylaminomethyl bedeutet und die übrigen Symbole die oben angegebene Bedeutung besitzen, mit der Massgabe, dass mindestens einer von A, $R^2$, $R^5$, $R^7$ und $R^{82}$ eine N-Schutzgruppe enthält, die N-Schutzgruppe(n) abspaltet, und

g) erwünschtenfalls ein Gemisch diastereomerer Racemate in die diastereomeren Racemate oder optisch reinen Diastereomeren auftrennt, und/oder

h) erwünschtenfalls ein Diastereomerengemisch in die optisch reinen Diastereomeren auftrennt, und/oder

i) erwünschtenfalls eine erhaltene Verbindung in ein pharmazeutisch verwendbares Salz überführt.

Die Acylierung einer Verbindung der Formel II erfolgt nach an sich bekannten Methoden. Geeignete Acylierungsmittel sind insbesondere aktivierte Säurederivate, wie Ester, gemischte Ester, Säurehalogenide und Säureanhydride oder gemischte Säureanhydride. Die Reaktion wird in einem unter den Reaktionsbedingungen inerten organischen Lösungsmittel oder Lösungsmittelgemisch bei einer Temperatur zwischen etwa 0° C und der Raumtemperatur durchgeführt. Als Lösungsmittel kommen insbesondere aromatische Kohlenwasserstoffe, wie Benzol, Toluol oder Xylol, chlorierte Kohlenwasserstoffe, wie Methylenchlorid oder Chloroform, Aether, wie Diäthyläther, Tetrahydrofuran oder Dioxan, und dergleichen in Frage. Falls es sich beim Acylierungsmittel um ein Peptid handelt, erfolgt die Reaktion unter in der Peptidchemie üblichen Reaktionsbedingungen, d.h. vorzugsweise in Gegenwart eines Kondensationsmittels wie HBTU (O-Benzotriazolyl-N,N,N′,N′-tetramethyluronium-hexafluorophosphat), HOBTU (1,1,3,3-Tetramethyl-2-[4-oxo-1,2,3-benzotriazin-3(4H)-yl]uronium-hexafluorophosphat), BOP (Benzotriazol-1-yloxy-tris(dimethylamino)-phosphonium-hexafluorophosphat), BOPC (Bis(2-oxo-2-oxozolidinyl)phosphinchlorid), HOBT (N-Hydroxybenzotriazol), HOOBT (3,4-Dihydro-3-hydroxy-4-oxo-1,2,3-benzotriazin), DBU (1,8-Diazabicyclo[5.4.0]-undec-7-en), DCC (Dicyclohexylcarbodiimid), EDC (N-Aethyl-N′(3-dimethylaminopropyl)carbodiimid-hydrochlorid), Hünigbase (Aethyldiisopropylamin), und dergleichen. Die Reaktion wird zweckmässigerweise in einem unter den Reaktionsbedingungen inerten organischen Lösungsmittel oder Lösungsmittelgemisch bei einer Temperatur zwischen etwa 0° und 50° C, vorzugsweise bei etwa Raumtemperatur durchgeführt. Als Lösungsmittel kommen insbesondere Dimethylformamid, Methylenchlorid, Acetonitril, Tetrahydrofuran, und dergleichen in Frage.

Die Umsetzung einer Verbindung der Formel III mit einer Verbindung der Formel IV erfolgt ebenfalls nach an sich in der Peptid-Chemie bekannten Methoden, d.h. unter den gleichen Bedingungen wie oben für die Umsetzung einer Verbindung der Formel II mit einem Peptid angegeben wurde. Beispiele geeigneter aktivierter Derivate einer Verbindung der Formel IV sind Säurehalogenide, Säureanhydride, gemischte Anhydride, Ester, gemischte Ester, und dergleichen.

Die Umsetzung einer Verbindung der Formel I, worin Z Wasserstoff bedeutet, mit einer Aminosäure gemäss Verfahrensvariante c) erfolgt ebenfalls nach an sich in der Peptid-Chemie bekannten Methoden, d.h. unter den oben für die Umsetzung einer Verbindung der Formel II mit einem Peptid angegebenen Bedingungen.

Die Umsetzung einer Verbindung der Formel I, worin $R^5$ und/oder $R^7$ Hydroxy oder Amino bedeuten, mit einem gegebenenfalls durch Amino, Monoalkylamino, Dialkylamino, Alkanoylamino,Alkoxycarbonylamino, Alkylcarbonyloxy, Carboxy, Alkoxy oder Hydroxy ein- oder mehrfach substituierten Alkanoylierungsmittel oder einem Aroylierungs-, Arylalkanoylierungs-, Cycloalkanoylierungs- oder Heteroarylalkanoylierungsmittel erfolgt ebenfalls nach an sich bekannten Methoden. Geeignete Acylierungsmittel sind Säureanhydride und Säurehalogenide, vorzugsweise Säurechloride. Die Umsetzung erfolgt in einem unter den Reaktionsbedingungen inerten organischen Lösungsmittel oder Lösungsmittelgemisch, wie Methylenchlorid, Dimethylformamid und dergleichen, bei einer Temperatur zwischen etwa Raumtemperatur und der Rückflusstemperatur des Reaktionsgemisches, vorzugsweise bei etwa Raumtemperatur. Die Reaktion kann in Gegenwart oder Abwesenheit eines Säurebindemittels, wie Natrium- oder Kaliumcarbonat, Pyridin, Triäthylamin und dergleichen, durchgeführt werden. Die Umsetzung mit einem Sulfatisierungs- oder Phosphorylierungsmittel erfolgt gleichfalls in an sich bekannter Weise. Geeignete Sulfatisierungsmittel sind

Komplexe von Schwefeltrioxid mit einer organischen Base, wie der Schwefeltrioxid-Dimethylformamid-Komplex, der Schwefeltrioxid-Triäthylamin-Komplex, der Schwefeltrioxid-Aethyldiisopropylamin-Komplex, der Schwefeltrioxid-Pyridin-Komplex und dergleichen. Die Reaktion erfolgt in einem unter den Reaktionsbedingungen inerten organischen Lösungsmittel, wie Dimethylformamid und dergleichen, bei einer Temperatur zwischen etwa 0° und 50° C, zweckmässigerweise bei Raumtemperatur. Geeignete Phosphorylierungsmittel sind beispielsweise Dialkylchlorophosphate in Pyridin, wie Diäthylchlorophosphat in Pyridin. Die Umsetzung erfolgt wiederum bei einer Temperatur zwischen etwa 0° und 50° C, vorzugsweise bei Raumtemperatur. Die Umsetzung einer Verbindung der Formel I, worin $R^5$ und/oder $R^7$ Hydroxy oder Amino bedeuten, mit einem eine N- und/oder O-Schutzgruppe bildenden Mittel erfolgt ebenfalls nach an sich bekannten Methoden. So können beispielsweise die Tetrahydropyranyläther durch Umsetzen mit Dihydropyran in Gegenwart eines Säurekatalysators, wie p-Toluolsulfonsäure und dergleichen, und das Acetonketal durch Umsetzen mit 2,2-Dimethoxypropan in Gegenwart eines Säurekatalysators, wie p-Toluolsulfonsäure, hergestellt werden., Die Umsetzung mit einem eine N-Schutzgruppe bildenden Mittel erfolgt in Abhängigkeit von der Natur des eingesetzten Mittels. Handelt es sich beim Mittel um ein Acylierungsmittel, so erfolgt die Umsetzung in analoger Weise wie die O-Acylierung, d.h. mit Säureanhydriden, Säurechloriden und dergleichen, unter denselben Reaktionsbedingungen. Hingegen wird die Umsetzung mit einem Arylmethylierungsmittel, z.B. Tritylchlorid und dergleichen, in Gegenwart einer organischen oder anorganischen Base, wie Triäthylamin, Aethyldiisopropylamin, Kaliumcarbonat und dergleichen, bei einer Temperatur zwischen etwa 0° und 50° C, vorzugsweise bei Raumtemperatur, in einem unter den Reaktionsbedingungen inerten organischen Lösungsmittel, wie Methylenchlorid, Dimethylformamid, Methanol und dergleichen, durchgeführt.

Die N-Substitution am Imidazolring gemäss Verfahrensvariante e) erfolgt ebenfalls nach an sich bekannten Methoden. So wird beispielsweise eine Verbindung der Formel I, worin $R^2$ Imidazol-2-yl, Imidazol-4-yl, C-methyliertes Imidazol-2-yl oder Imidazol-4-yl bedeutet, mit einem geeigneten Acylierungs-, Alkylierungs-, Arylalkylierungs-oder Arylierungsmittel in Gegenwart einer organischen oder anorganischen Base, wie Triäthylamin, Kaliumcarbonat und dergleichen, bei einer Temperatur zwischen etwa 0° und 50° C, vorzugsweise bei Raumtemperatur, in einem unter den Reaktionsbedingungen inerten organischen Lösungsmittel oder Lösungsmittelgemisch, wie Methylenchlorid, Dimethylformamid, oder für die Alkylierung oder Arylalkylierung auch Methanol, und dergleichen durchgeführt. Beispiele geeigneter Acylierungs-, Alkylierungs-, Arylalkylierungs- und Arylierungsmittel sind Säurehalogenide, wie z.B. Chlorameisen säurealIylester, Carbonate, wie Di-tert-butyl-dicarbonat, Halogenide, wie Tritylchlorid, und 2,4-Dinitrofluorbenzol, und dergleichen.

Die Abspaltung der N-Schutzgruppe(n) gemäss Verfahrensvariante f) erfolgt ebenfalls nach an sich bekannten Methoden in Abhängigkeit von der Art der abzuspaltenden N-Schutzgruppe. Die Abspaltung erfolgt jedoch zweckmässig durch saure oder basische Hydrolyse. Für die saure Hydrolyse verwendet man vorteilhafter Weise eine Lösung einer Mineralsäure, wie Chlorwasserstoffsäure, Bromwasserstoffsäure, Trifluoressigsäure, Schwefelsäure, Phosphorsäure und dergleichen, in einem inerten Lösungsmittel oder Lösungsmittelgemisch. Geeignete Lösungsmittel sind Alkohole, wie Methanol oder Aethanol, Aether, wie Tetrahydrofuran oder Dioxan, chlorierte Kohlenwasserstoffe, wie Methylenchlorid, und dergleichen. Für die basische Hydrolyse können Alkalimetallhydroxyde und -carbonate, wie Kalium- oder Natriumhydroxyd oder Kalium- oder Natriumcarbonat, organische Amine, wie Piperidin, und dergleichen verwendet werden. Inerte organische Lösungsmittel, wie sie oben für die saure Hydrolyse genannt sind, können als Lösungsvermittler zugegeben werden. Die Reaktionstemperatur kann für die saure und basische Hydrolyse in einem Bereich von etwa 0° C bis Rückflusstemperatur variiert werden, wobei man vorzugsweise zwischen etwa 0° C und der Raumtemperatur arbeitet. Der t-Butoxycarbonylrest wird zweckmässig mit Trifluoressigsäure oder Ameisensäure in Gegenwart oder Abwesenheit eines inerten Lösungsmittels abgespalten. Die Fmoc-Schutzgruppe wird zweckmässig mit Piperidin bei etwa Raumtemperatur abgespalten. Die Benzyloxycarbonylgruppe kann in bekannter Weise durch saure Hydrolyse wie weiter oben beschrieben oder hydrogenolytisch abgespalten werden.

Die Ausgangsstoffe der Formel II sind teilweise neu und teilweise bekannt. Diese Verbindungen können hergestellt werden, indem man eine Verbindung der Formel III mit einer der Formel IV entsprechenden Verbindung, worin A jedoch Wasserstoff bedeutet, umsetzt. Diese Umsetzung erfolgt ebenfalls nach in der Peptid-Chemie bekannten Methoden, d.h. unter den Reaktionsbedingungen, wie sie weiter oben für die Umsetzung einer Verbindung der Formel II mit einem Acylierungsmittel beschrieben sind.

Die Ausgangsstoffe der Formel III sind ebenfalls teilweise neu und teilweise bekannt. So können beispielsweise diejenigen Verbindungen der Formel III, worin $R^4$ und $R^6$ je Wasserstoff und $R^5$ und $R^7$ je Hydroxy bedeuten, hergestellt werden, indem man in einer Verbindung der allgemeinen Formel

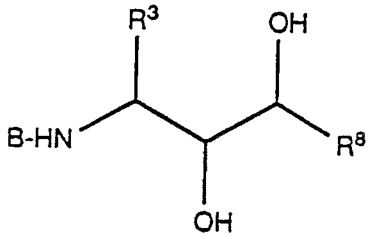

V

oder

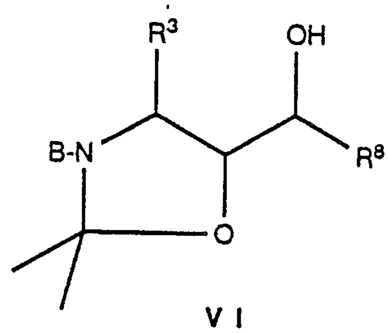

V I

worin B eine Aminoschutzgruppe bedeutet, vorzugsweise t-Butoxycarbonyl oder Benzyloxycarbonyl, und $R^3$ und $R^8$ die oben angegebene Bedeutung besitzen,
die Aminoschutzgruppe und gegebenenfalls gleichzeitig auch die O-Schutzgruppe abspaltet oder indem man eine Verbindung der allgemeinen Formel

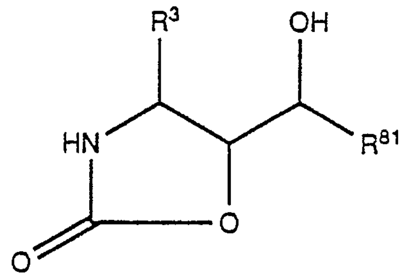

V I I

worin $R^3$ die oben angegebene Bedeutung besitzt und $R^{81}$ die Gruppe (a) oder (b) bedeutet,
mit einer Base behandelt.

Die Abspaltung der N-Schutzgruppe und gegebenenfalls O-Schutzgruppe erfolgt ebenfalls nach an sich bekannten Methoden, beispielsweise in einem unter den Reaktionsbedingungen inerten organischen Lösungsmittel oder Lösungsmittelgemisch bei einer Temperatur zwischen etwa 0° C und der Raumtemperatur mit einer Säure, wie Chlorwasserstoffsäure, Trifluoressigsäure, und dergleichen. Geeignete Lösungsmittel sind Aether, wie Tetrahydrofuran oder Dioxan, Alkohole, wie Methanol, oder chlorierte Kohlenwasserstoffe, wie Methylenchlorid, und dergleichen. Unter diesen Reaktionsbedingungen wird in einer Verbindung der Formel VI - wie bereits erwähnt - gleichzeitig der Oxazolidinring aufgespalten. Ist allerdings ausschliesslich die Aufspaltung des Oxazolidinrings erwünscht, so sind andere Reaktionsbedingungen zu wählen: die Reaktion muss bei tieferen Temperaturen und in wässrigen Lösungsmitteln oder beispielsweise mit Eisentrichlorid/Kieselgel durchgeführt werden.

Die Umsetzung einer Verbindung der Formel VII mit einer Base erfolgt ebenfalls nach an sich bekannten Methoden in einem unter den Reaktionsbedingungen inerten Lösungsmittel oder Lösungsmittelgemisch bei einer Temperatur zwischen etwa Raumtemperatur und der Rückflusstemperatur. Geeignete Lösungsmittel sind beispielsweise Methanol, Aethanol, Dioxan, Tetrahydrofuran, Wasser oder Gemische davon. Als Basen kommen zweckmässigerweise Natrium-, Kalium- oder Bariumhydroxyd und dergleichen in Frage.

Die übrigen Verbindungen der Formel III können in Analogie zur Herstellung derjenigen Verbindungen der Formel III erhalten werden, worin $R^4$ und $R^6$ je Wasserstoff und $R^5$ und $R^7$ je Hydroxy bedeuten. Als Ausgangsstoffe werden den Formeln V und VI entsprechende Verbindungen eingesetzt, worin $R^4$, $R^5$, $R^6$ und $R^7$ die übrigen möglichen Bedeutungen besitzen. In den nachstehenden Schemata I-III sind verschiedene Verfahren zur Herstellung von Verbindungen der Formeln V, VI und VII sowie der übrigen zur Herstellung der Verbindungen der Formel III notwendigen Ausgangsstoffe formelmässig darstellt. Die Formeln V und VI scheinen in den Schemata nicht auf; hingegen fällt die Formel Xa unter die Formel V und fallen die Formeln Xb, XV, XIX ,und XXII unter die Formel VI. Die in den Schemata verwendeten Symbole B, $R^3$ und $R^{81}$ besitzen die oben angegebene Bedeutung, während $R^{19}$ Alkyl oder Cycloalkyl, B′ eine Aminoschutzgruppe, P eine O-Schutzgruppe und Q eine Abgangsgruppe, wie Alkylsulfonyl oder Arylsulfonyl, beispielsweise Methylsulfonyl, Trifluormethylsulfonyl, p-Toluolsulfonyl und dergleichen, bedeuten.

Bei den in den Schemata I-III dargestellten Schritten handelt es sich durchwegs um in der synthetischen Chemie übliche Reaktionen, die alle nach an sich bekannten Methoden durchgeführt werden. Bezüglich der genauen Reaktionsbedingungen wird auf den Beispielteil verwiesen. Der Vollständigkeit halber sei erwähnt, dass gewisse, in den Schemata I-III aufgezeichnete Reaktionsschritte auch auf einer späteren Stufe der Synthese, beispielsweise als Schlussstufe zur Herstellung der Verbindungen der Formel I, durchgeführt werden können, wie beispielsweise der Oxazolidinringschluss gemäss Stufe XVII → XVI, die Abspaltung einer O-Schutzgruppe gemäss Stufe XXXIII → XXXIV oder die Oximbildung gemäss Stufe XLII → XLVI und dergleichen. Die selbstverständlich nur dann, wenn das Molekül keine anderen Gruppen enthält, welche dabei in Mitleidenschaft gezogen werden könnten.

Die Ausgangsstoffe der Formel IV sind bekannt oder können in Analogie zur Herstellung der bekannten Verbindungen erhalten werden.

Schema I

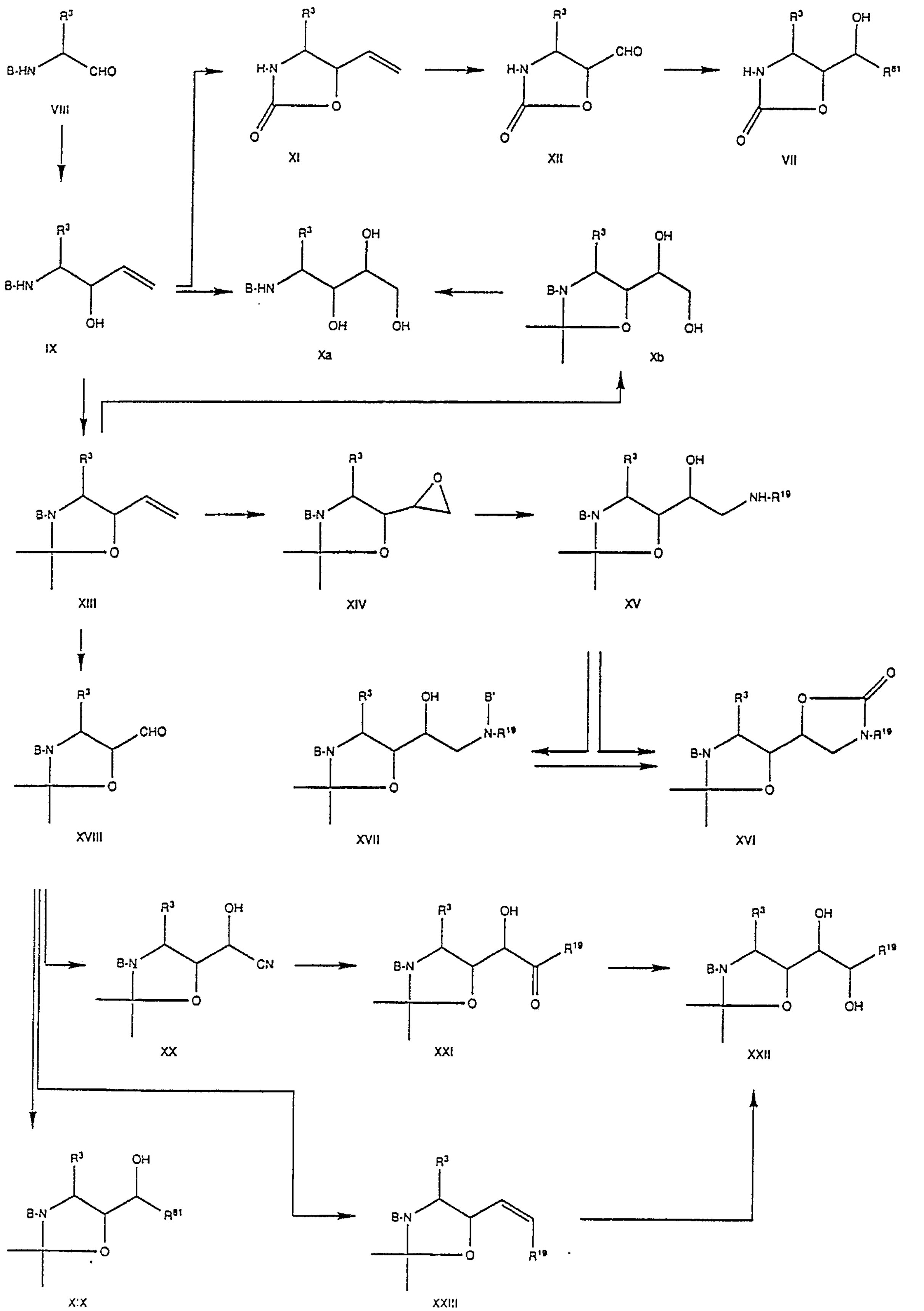

VIII, XI, XII, VII, IX, Xa, Xb, XIII, XIV, XV, XVIII, XVII, XVI, XX, XXI, XXII, XIX, XXIII

Schema II

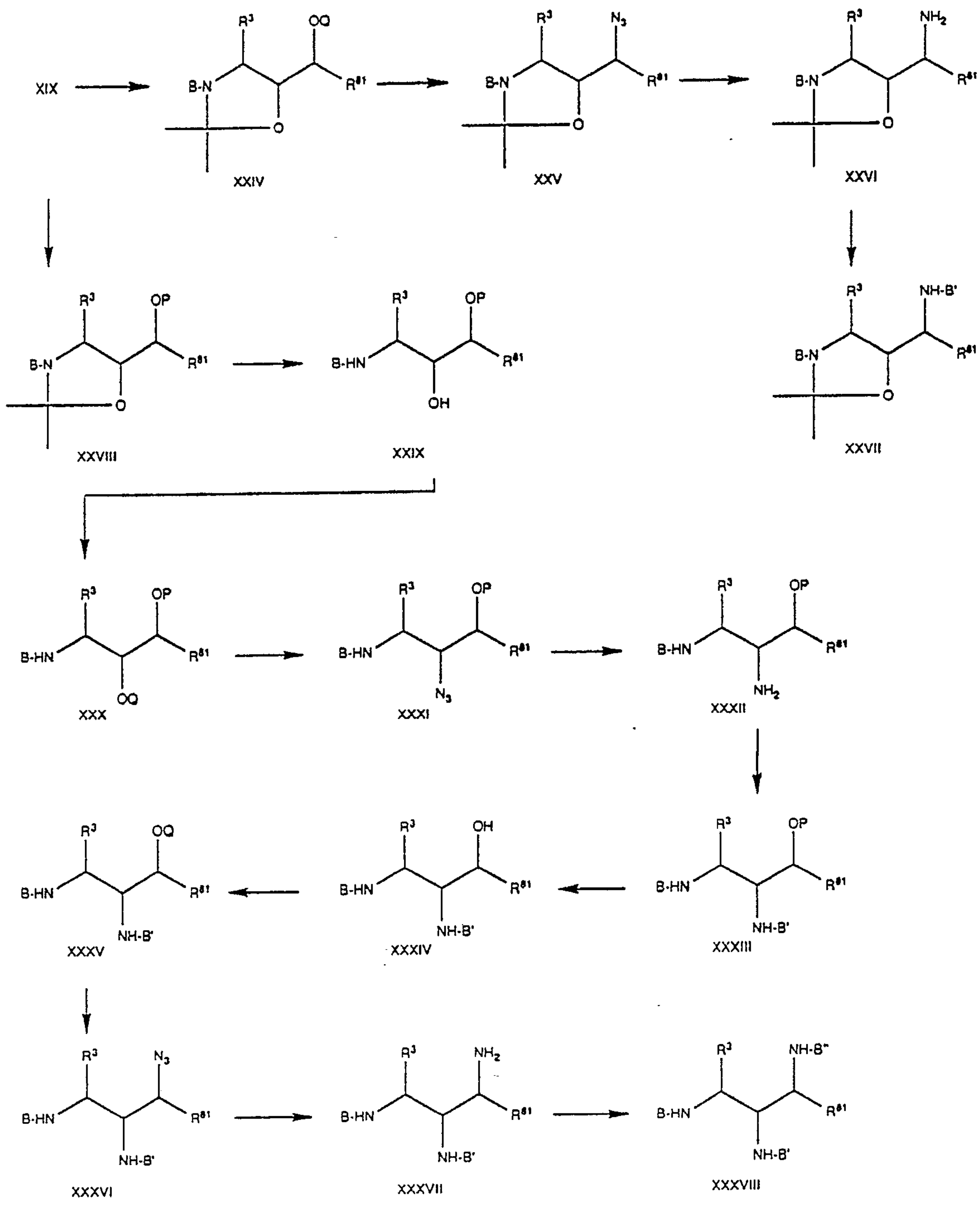

XIX XXIV XXV XXVI XXVIII XXIX XXVII XXX XXXI XXXII XXXV XXXIV XXXIII XXXVI XXXVII XXXVIII

Schema III

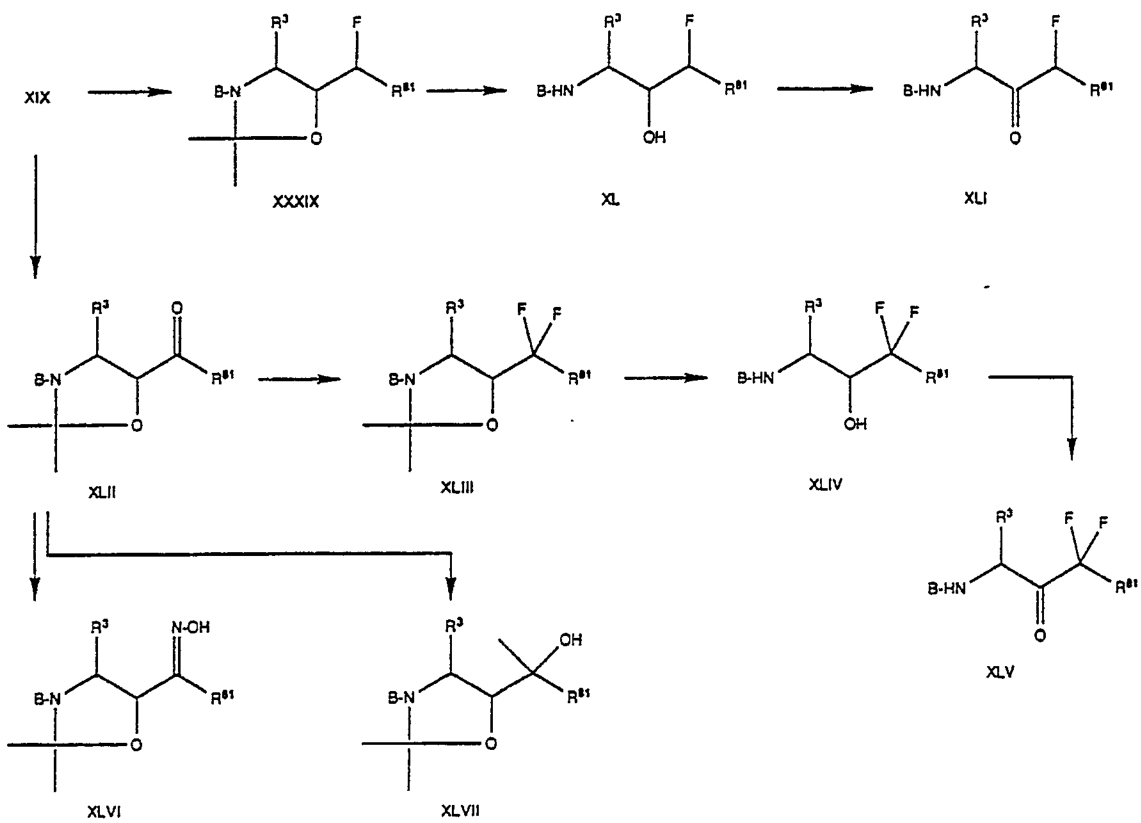

XIX, XXXIX, XL, XLI, XLII, XLIII, XLIV, XLV, XLVI, XLVII

Die noch neuen Ausgangsstoffe der Formel II und III sowie der in den Schemata I-III angegebenen, Formeln sind ebenfalls Gegenstand der vorliegenden Erfindung.

Die Verbindungen der Formel I und ihre pharmazeutisch verwendbaren Salze weisen hemmende Wirkung des natürlichen Enzyms Renin auf. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Angiotensin II erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des daraus als Spaltprodukt gebildeten Heptapeptids Angiotensin III zurückzuführen. Hemmer der enzymatischen Aktivität von Renin bewirken eine Verringerung der Bildung von Angiotensin I und als Folge davon die Bildung einer geringeren Menge von Angiotensin II. Die verminderte Konzentration dieses aktiven Peptid-Hormons ist die unmittelbare Ursache für die blutdrucksenkende Wirkung von Renin-Hemmern.

Die Wirkung von Renin-Hemmern kann experimentell mittels des nachstehend beschriebenen In vitro-Tests gezeigt werden:

<u>In vitro-Test mit reinem Human-Renin</u>

Der Test wird in Eppendorf Röhrchen durchgeführt. Die Inkubationsmischung besteht aus (1) 100 µl Human-Renin in Puffer A (0,1M Natriumphosphatlösung, pH 7,4, enthaltend 0,1% Rinderserumalbumin, 0,1 % Natriumazid und 1 mM Aethylendiamintetraessigsäure), genügend für eine Renin-Aktivität von 2-3 ng Angiotensin I/ ml/Std.: (2) 145 µl Puffer A; (3) 30 µl von 10 µM humanem Tetradekapeptid-Reninsubstrat (hTD) in 10 mM Salzsäure; (4) 15 µl Dimethyl sulfoxid mit bzw. ohne Hemmer und (5) 10 µl einer 0,03 molaren Lösung von Hydroxychinolinsulfat in Wasser.

Die Proben werden drei Stunden bei 37° C bzw. 4° C in Triplikaten inkubiert. 2 x 100 µl Proben pro Versuchsröhrchen werden dann verwendet, um die Produktion von Angiotensin I via RIA (standard

radioimmunoassay; Clinical Assay solid phase kit) zu messen. Kreuzreaktivitäten der verwendeten Antikörper im RIA sind: Angiotensin I 100 %; Angiotensin II 0,0013 %; hTD (Angiotensin I-Val-Ile-His-Ser-OH) 0,09 %. Die Produktion von Angiotensin I wird durch die Differenz zwischen dem Versuch bei 37°C und demjenigen bei 4°C bestimmt.

Folgende Kontrollen werden mitgeführt:

(a) Inkubation von hTD-Proben ohne Renin und ohne Hemmer bei 37°C und 4°C. Die Differenz zwischen diesen beiden Werten ergibt den Grundwert der Angiotensin I-Produktion.

(b) Inkubation von hTD-Proben mit Renin, jedoch ohne Hemmer bei 37°C und 4°C. Die Differenz zwischen diesen Werten ergibt den Maximalwert der Angiotensin I-Produktion.

In jeder Probe wird von der ermittelten Angiotensin I-Produktion der Grundwert der Angiotensin I-Produktion abgezogen. Die Differenz zwischen dem Maximalwert und dem Grundwert ergibt den Wert der maximalen Substrathydrolyse ( = 100%) durch Renin.

Die Resultate werden als $IC_{50}$-Werte angegeben, welche diejenige Konzentration des Hemmers bezeichnen, bei welcher die enzymatische Aktivität um 50% gehemmt wird. Die $IC_{50}$-Werte werden aus einer linearen Regressionskurve aus einem logit-log plot ermittelt.

Die in diesem Test erhaltenen Resultate sind in der folgenden Tabelle zusammengefasst:

Tabelle

| Verbindung | $IC_{50}$-Werte in μMol/lt. |
|---|---|
| A | 0,0018 |
| B | 0,0170 |
| C | 0,0009 |
| D | 0,0012 |
| E | 0,0023 |
| F | 0,0210 |

A = (S)-α-[(S)-α-[[[(2-Amino-1,1-dimethyläthyl)sulfonyl]methyl]hydrocinnamamido]-N-[(1S,2R,3S)-1-(cyclohexylmethyl)-3-cyclopropyl-2,3-dihydroxypropyl]imidazol-4-propionamiddihydrochlorid,

B = (S)-α-[(S)-α-[[(2-Amino-2-methylpropyl)sulfonyl]methyl]hydrocinnamamido]-N-[(1S,2R,3S)-1-(cyclohexylmethyl)-3-cyclopropyl-2,3-dihydroxypropyl]imidazol-4-propionsäureamiddiacetat,

C = (S)-α-[(S)-α-[(tert-Butylsulfonyl)methyl]hydrocinnamamido]-N-[(1S,2R)-2-(cyclohexylmethyl)-2-[(R oder S)-3-cyclopropyl-2-oxo-5-oxazolidinyl]-2-hydroxyäthyl]imidazol-4-propionamid,

D = (S)-α-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]phenäthyl 1-piperidincarboxylat,

E = (S)-α-[(S)-α-[(tert-Butylsulfonyl)methyl]hydrocinnamamido]-N-[(1S,2R,3S)-1-(cyclohexylmethyl)-3-cyclopropyl-3-fluor-2-hydroxypropyl]imidazol-4-propionamid und

F = N-[(S)-1-[[(1S,2R,3S)-1-(Cyclohexylmethyl)-3-cyclopropyl-2,3-dihydroxypropyl]carbamoyl]-2-imidazol-4-yläthyl]indol-2-carboxamid.

Die Verbindungen der Formel I sowie deren pharmazeutisch verwendbare Salze können als Heilmittel, z.B. in Form pharmazeutischer Präparate, Verwendung finden. Die pharmazeutischen Präparate können enteral, wie oral, z.B. in Form von Tabletten, Lacktabletten, Dragees, Hart- und Weichgelatinekapseln, Lösungen, Emulsionen oder Suspensionen, nasal, z.B. in Form von Nasensprays, oder rektal, z.B. in Form von Suppositorien, verabreicht werden. Die Verabreichung kann aber auch parenteral, wie intramuskulär oder intravenös, z.B. in Form von Injektionslösungen, erfolgen.

Zur Herstellung von Tabletten, Lacktabletten, Dragees und Hartgelatinekapseln können die Verbindungen der Formel I sowie ihre pharmazeutisch verwendbaren Salze mit pharmazeutisch inerten, anorganischen oder organischen Excipientien verarbeitet werden. Als solche Excipientien kann man z.B. für Tabletten, Dragees und Hartgelatinekappeln, Laktose, Maisstärke oder Derivate davon, Talk, Stearinsäure oder deren Salze etc. verwenden.

Für Weichgelatinekappeln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose etc.

Für Injektionslösungen eignen sich als Excipientien z.B. Wasser, Alkohole, Polyole, Glycerin, vegetabile

Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, viskositätserhöhende Stoffe, Stabilisierungsmittel, Netzmittel, Emulgiermit tel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der allgemeinen Formel I sowie deren pharmazeutisch verwendbare Salze bei der Bekämpfung bzw. Verhütung von Bluthochdruck und Herzinsuffizienz verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 3 mg bis etwa 3 g, vorzugsweise etwa 10 mg bis etwa 1 g, z.B. ungefähr 300 mg pro Person, verteilt auf vorzugsweise 1-3 Einzeldosen, die z.B. gleich gross sein können, angemessen sein, wobei aber die soeben angegebene obere Grenze auch überschritten werden kann, wenn sich dies als angezeigt erweisen sollte. Ueblicherweise erhalten Kinder die halbe Dosis von Erwachsenen.

Die folgenden Beispiele sollen die vorliegende Erfindung erläutern, sie jedoch in keiner Weise einschränken. Alle Temperaturen sind in Celsiusgraden angegeben. Es werden die folgenden Abkürzungen verwendet:

H-His-OH = L-Histidin
H-Phe-OH = L-Phenylalanin
H-Pro-OH = L-Prolin
Boc = t-Butoxycarbonyl
Fmoc = 9-Fluorenylmethoxycarbonyl

## Beispiel 1

Ein Gemisch von 200 mg (0.5 mMol) (S)-α-Amino-N-[(1S,2R,3S)-1-(cyclohexylmethyl)-3-cyclopropyl2,3-dihydroxypropyl]imidazol-4-propionamid, 197 mg (0.5 mMol) (S)-α-[[[2-(1-tert-Butoxyformamido)-1,1-di methyläthyl]sulfonyl]methyl]hydrozimtsäure, 56 mg (0.5 mMol) Triäthylamin, 89 mg (0.5 mMol) HOBT und 209mg (0.5 mMol) HBTU in 15 ml Dimethylformamid wird 4 Stunden lang bei Raumtemperatur gerührt. Danach wird das Reaktionsgemisch im Hochvakuum zur Trockene eingedampft, der Rückstand in 50 ml Essigester aufgenommen und zweimal mit 20 ml gesättigter Natriumbicarbonatlösung gewaschen. Nach dem Trocknen der organischen Phase über Natriumsulfat wird das Lösungsmittel unter vermindertem Druck abgedampft. Der Rückstand (420 mg) wird zur Reinigung an 20 g Kieselgel unter Verwendung eines 95:5:0.1-Gemisches von Methylenchlorid, Methanol und Ammoniak als Eluierungsmittel chromatographiert. Nach dem Lyophilisieren aus Dioxan/Wasser wird tert-Butyl [2-[[(S)-2-[[(S)-1-[[(1S,2R,3S)-1-(cyclohexylmethyl)-3-cyclopropyl-2,3-dihydroxypropyl)carbamoyl]-2-imidazol-4-yläthyl]carbamoyl]-3-phenylpropyl]sulfonyl]-2-methylpropyl]carbamat als farbloses, amorphes Pulver erhalten; MS : 746 $(M+H)^+$.

Die als Ausgangsstoff eingesetzte (S)-α-[[[2-(1-tert-Butoxyformamido)-1,1-dimethyläthyl]sulfonyl]methyl]-hydrozimtsäure wurde wie folgt hergestellt:

(a) tert-Butyl [2-(benzylthio-2-methylpropyl]carbamat:

Zu einem Gemisch von 3.17 g (13.7 mMol) 2-(Benzylthio)-2-methylpropylamin-hydrochlorid [B.J.Sweetmann et al., J.Med.Chem. 14 , 868 (1971)] und 1.45 g (14.3 mMol) Triäthylamin in 30 ml Dimethylformamid werden bei 0° 3.28 g (15.1 mMol) Di-tert-butyldicarbonat getropft. Anschliessend wird das Reaktionsgemisch über Nacht bei Raumtemperatur gerührt und danach im Hochvakuum eingedampft. Der Rückstand wird dreimal mit je 50 ml Aether extrahiert. Die organischen Lösungen werden vereinigt, mit 50 ml Wasser gewaschen, über Natriumsulfat getrocknet und eingedampft. Chromatographie des so erhaltenen Rohprodukts (4.18 g) an Kieselgel mit einem 6:1-Gemisch von Hexan und Aether als Eluierungsmittel liefert 3.5 g tert-Butyl [2-(benzylthio)-2-methylpropyl]carbamat als farbloses Oel; MS : 239 $(M - C_4H_8)^+$.

(b) tert Butyl [2-methyl-2-mercaptopropyl]carbamat:

3.49 g (11.8 mMol) tert-Butyl [2-(benzylthio)-2-methylpropyl]carbamat, gelöst in 10 ml Aether werden in einem Kolben vorgelegt, der mit einem Ammoniakkühler und einem Gaseinlass ausgestattet ist. Die Lösung wird auf -78° abgekühlt, danach werden ca. 35 ml Ammoniak einkondensiert. Zu dieser Lösung wird so lange Natrium (ca. 1 g) in kleinen Stücken gegeben, bis die blaue Farbe der Lösung bestehen bleibt. Man lässt das Reaktionsgemisch langsam erwärmen, zerstört das überschüssige Natrium durch Zusatz von Ammoniumchlorid (ca. 3.5 g) und bläst den Ammoniak mittels Stickstoff ab. Der Rückstand wird zwischen 50 ml Wasser und 250 ml Aether verteilt. Die organische Phase wird abtgetrennt, über Natriumsulfat getrocknet und eingedampft. Man erhält 2.29 g tert-Butyl [2-methyl-2-mercaptopropyl]carbamat als farblosen Festkörper; MS : 149 $(M - C_4H_8)^+$.

(c) tert-Butyl [2-[[(RS)-2-(äthoxycarbonyl)-3-phenylpropyl]sulfonyl]-2-methylpropyl]carbamat:

Zu einem Gemisch von 1.99 g (10.47 mMol) α-Benzylacrylsäureäthylester und 2.26 g (11 mMol) tert-Butyl [2-methyl-2-mercaptopropyl]carbamat in 5 ml Dioxan werden bei 0° 10.2 ml (11 mMol) 1.08 N Natriumäthylat-Lösung getropft. Danach wird 0.5 Stunden bei 0° und 2.5 Stunden bei Raumtemperatur gerührt. Das Reaktionsgemisch wird unter Eiskühlung mit 8.96 g (14.6 mMol) Kaliummonopersulfat Tripelsalz, suspendiert in 15 ml Wasser, versetzt und 1 Stunde bei Raumtemperatur gerührt. Danach wird auf 0° abgekühlt und nochmals eine Suspension von 8.96 g (14.6 mMol) Kaliummonopersulfat Tripelsalz in 15 ml Wasser zugegeben. Anschliessend wird 15 Stunden bei Raumtemperatur gerührt. Das Reaktionsgemisch wird mit 50 ml Wasser verdünnt, und die wässrige Lösung dreimal mit je 50 ml Aether extrahiert. Die vereinigten organischen Lösungen werden über Natriumsulfat getrocknet und eingedampft. Man erhält 3.25 g tert-Butyl [2-[[(RS)-2-(äthoxycarbonyl)-3-phenylpropyl]sulfonyl]-2-methylpropyl]carbamat als farbloses Oel; MS : 445 $(M + NH_4)^+$.

(d) (S)-α-[[[2-(1-tert-Butoxyformamido)-1,1-dimethyläthyl]sulfonyl]methyl]hydrozimtsäure und (R)-α-[[[2-(1-tert-Butoxyformamido)-1,1-dimethyläthyl]sulfonyl]methyl]hydrozimtsäureäthylester:

Zu einem Gemisch von 1.75 g (4.1 mMol) tert-Butyl [2-[[(RS)-2-(äthoxycarbonyl)-3-phenylpropyl]-sulfonyl]-2-methylpropyl]carbamat und 70 mg α-Chymotrypsin in 5 ml Aethanol und 250 ml Wasser wird unter Rühren eine 0.039 N Calciumhydroxid-Lösung so zugegeben, dass der pH-Wert bei 7.5 gehalten wird. Wenn kein Calciumhydroxid mehr verbraucht wird, erfolgt die Aufarbeitung des Reaktionsgemisches, indem die wässrige Lösung zweimal mit je 50 ml Essigester extrahiert wird. Die vereinigten organischen Phasen werden mit 25 ml gesättigter Natriumbicarbonat-Lösung gewaschen, über Natriumsulfat getrocknet und unter vermindertem Druck eingedampft. Man erhält 0.86 g (R)-α-[[[2-(1-tert-Butoxyformamido)-1,1-dimethyläthyl]sulfonyl]methyl]hydrozimtsäureäthylester als gelbliches Oel; MS: 445 $(M + NH_4)^+$.

Die wässrige Phase und die Natriumbicarbonat-Lösung werden vereinigt, mit 1 N Salzsäure auf pH 3.5 gestellt und, nach Zusatz von festem Natriumchlorid, zweimal mit je 50 ml Essigester extrahiert. Nach dem Trocknen über Natriumsulfat und Eindampfen unter vermindertem Druck werden 0.81 g (S)-α-[[[2(1-tert-Butoxyformamido)-1,1-dimethyläthyl]sulfonyl]methyl]hydrozimtsäure als gelblicher Schaum erhalten; MS : 343 $(M - C_4H_8)^+$.

Das als Ausgangsstoff eingesetzte (S)-α-Amino-N-[(1S,2R,3S)-1-(cyclohexylmethyl)-3-cyclopropyl-2,3-dihydroxypropyl]imidazol-4-propionamid wurde wie folgt hergestellt:

(e) tert-Butyl (4S,5R)-4(cyclohexylmethyl)-5-[(S)-cyclopropylhydroxmethyl]-2,2-dimethyl-3-oxazolidincarboxylat und tert-Butyl (4S,5R)-4-(cyclohexylmethyl)-5-[(R)-cyclopropylhydroxymethyl]-2,2-dimethyl-3-oxazolidincarboxylat:

Zu einer Lösung der aus 3.94 ml (49 mMol) Cyclopropylbromid und 1.2 g (0.049 Grammatom) Magnesiumspänen in 22 ml Tetrahydrofuran hergestellten Grignardverbindung wird bei ca. 15° eine Lösung von 3.21 g (9,8 mMol) tert-Butyl (4S, SR)-4-(cyclohexylmethyl)-5-formyl-2,2-dimethyl-3-oxazolidincarboylat (WO 87/05302) in 25 ml Tetrahydrofuran getropft, und das Reaktionsgemisch anschliessend während 16 Stunden bei Raumtemperatur unter Argon gerührt. Danach giesst man das Reaktionsgemisch auf 40 ml einer eiskalten, gesättigten Ammoniumchloridlösung und extrahiert zweimal mit je 50 ml

Essigester. Die Essigesterextrakte werden mit 40 ml eiskalter, gesättigter Ammoniumchloridlösung gewaschen, vereinigt, über Natriumsulfat getrocknet und unter vermindertem Druck eingedampft. Zur Reinigung wird der Rückstand (4.33 g) über eine mit Toluol und 1% Triäthylamin aufgezogene Säule von 110 g Kieselgel mit einem 95:5-Gemisch von Toluol und Essigester als Eluierungsmittel chromatographiert. Man erhält 1.9 g tert-Butyl (4S,5R)-4-(cyclohexylmethyl)-5-[(S)-cyclopropylhydroxymethyl]-2,2-dimethyl-3-oxazolidincarboxylat, MS: 368 $(M+H)^+$, und 0.5 g tert-Butyl (4S,5R)-4-(cyclohexylmethyl)-5-[(R)-cyclopropylhydroxymethyl]-2,2-dimethyl-3-oxazolidincarboxylat, MS : 368 $(M+H)^+$, jeweils als farbloses Oel.

(f) (1S,2R,3S)-3-Amino-4-cyclohexyl-1-cyclopropy-1,2-butandiol:

1.42 g (3.86 mMol) tert-Butyl (4S,5R)-4-(cyclohexylmethyl)-5-[(S)-cyclopropylhydroxymethyl]-2,2-dimethyl-3-oxazolidincarboxylat, gelöst in 15 ml Methanol und 10 ml Wasser, werden mit 4 ml 7.5 N Salzsäure versetzt und 3 Stunden bei 50° gerührt. Die Reaktionslösung wird im Eisbad auf 3° abgekühlt, mit 4 ml 7.5 N Natronlauge tropfenweise verset/t und 1 Stunde nachgerührt. Die erhaltene Suspension wird unter vermindertem Druck eingedampft, zweimal mit 10 ml Toluol azeotrop entwässert, und der Rückstand dreimal mit 10 ml eines 95:5-Gemisches von Methylenchlorid und Methanol ausgerührt. Der unlösliche Rückstand wird abfiltriert, und das Filtrat unter vermindertem Druck eingedampft. Das erhaltene Rohprodukt (1.14 g) wird in 15 ml Aether suspendiert und danach abfiltriert. Man erhält 0.58 g (1S,2R,3S)-3-Amino-4-cyclohexyl-1-cyclopropyl-1,2-butandiol als farblose Kristalle, Smp. 141-142°.

(g) (S)-α-Amino-N-[1S,2R,3S)-1-cyclohexylmethyl)-3-cyclopropyl-2,3-dihydroxypropyl]imidazol-4-propionamid:

Ein Gemisch von 343 mg (1.51 mMol) (1S,2R,3S)-3-Amino-4-cyclohexyl-1-cyclopropyl-1,2-butandiol, 995 mg (1.66 mMol) $(Fmoc)_2$His-OH, 0.21 ml (1.61 mMol) 4-Aethylmorpholin, 449 mg (3.22 mMol) HOBT und 347 mg (1.81 mMol) EDC in 20 ml Dimethylformamid wird über Nacht bei Raumtemperatur stehen gelassen. Danach wird das Reaktionsgemisch im Hochvakuum eingedampft, der Rückstand auf ein Gemisch von Eis und 90 ml Natriumbicarbonat-Lösung gegossen und dreimal mit je 150 ml Essigester extrahiert. Die drei Essigesterextrakte werden nacheinander mit 70 ml gesättigter Ammoniumchloridlösung, 70 ml 2N Natriumbicarbonat-Lösung und 70 ml gesättigter Natriumchlorid-Lösung gewaschen, vereinigt, über Magnesiumsulfat getrocknet, filtriert und eingedampft. Das erhaltene Rohprodukt wird in 60 ml Methylenchlorid und 2 ml Piperidin 3 Stunden bei Raumtemperatur gerührt. Dann wird das Reaktionsgemisch eingedampft, und der Rückstand mit 50 ml Hexan angerieben und abfiltriert. Das Filtrat wird mit einem 65:10:1-Gemisch von Methylenchlorid, Methanol und Ammoniak als Eluierungsmittel an 70 g Kieselgel chromatographiert, wobei man 390 mg (S)-α-Amino-N-[(1S,2R,3S)-1-(cyclohexylmethyl)-3-cyclopropyl-2,3-dihydroxypropyl]imidazol-4-propionamid als farblosen Schaum erhält; MS : 365 $(M+H)^+$.

## Beispiel 2

In analoger Weise wie in Beispiel 1 beschrieben, wurden die folgenden Verbindungen hergestellt:

- Aus (S)-α-Amino-N-[(1S,2R,3S)-1-(cyclohexylmethyl)-3-cyclopropyl-2,3-dihydroxypropyl]imidazol-4-propionamid und (S)-α-[[[2-(Dimethylamino)-1,1-dimethyläthyl]sulfonyl]methyl]hydrozimtsäure-hydrochlorid das (S)-N-[(1s,2R,3S)-1-(Cyclohexylmethyl)-3-cyclopropyl-2,3-dihydroxypropyl]-α-[(S)-α-[[[2-(dimethylamino)-1,1-dimethyläthyl]sulfonyl]methyl]hydrocinnamamido]imidazol-4-propionamid als farbloser, amorpher Festkörper, MS : 674 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und (S)-α-[[1,1-Dimethyl-2-morpholinoäthyl)sulfonyl]methyl]hydrozimtsäurehydrochlorid das (S)-N-[-(1S,2R, 3S)-1-(Cyclohexylmethyl)-3-cyclopropyl-2,3-dihydroxypropyl]-α-[(S)-α-[[(1,1-dimethyl-2-morpholinoäthyl)sulfonyl]methyl]hydrocinnamamido]imidazol-4-propionamid als farbloser, amorpher Festkörper, MS : 716 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und (S)-α-[[[2[1(Benzyloxy)formamido]-2-methylpropyl]sulfonyl]methyl]hydrozimtsäure das Benzyl [2-[[(S)-2-[[(S)-1-[[(1S,2R,3S)-1-(cyclohexylmethyl)-3- cyclopropyl-2,3-dihydroxypropyl]carbamoyl]-2-imidazol-4-yläthyl]carbamoyl]-3-phenylpropyl]sulfonyl]-1,1-dimethyläthyl]carbamat als farbloser, amorpher Festkörper, MS : 780 $(M+H)^+$;
- aus (S)-α-Amino-N-[(1S,2R,3S)-1(cyclohexylmethyl)-3-cyclopropyl-2,3-dihydroxypropyl]imidazol-4-propionamid und (S)-α[[[2-[1-(Benzyloxy)formamido]-2-methylpropyl]sulfonyl]methyl]-p-fluorhydrozimtsäure das Benzyl [2-[[(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]-p-fluorphenäthyl]sulfonyl]-1,1-dimethyläthyl]carbamat als farbloser, amorpher Festkörper, MS : 798 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und (S)-α-[(tert-Butylsulfonyl)methyl]cyclohexanpropionsäure das (S)-α-[(S)-α-[(tert-Butylsulfonyl)-methyl]cyclohexanpropionamido]-N-[(1S,2R,3S)-1-(cyclohexylmethyl)-3-cyclopropyl-2,3-dihydroxypropyl]-imidazol-4-propionamid als farbloser, amorpher Festkörper, MS : 637 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und (S)-α-[(tert-Butylsulfonyl)methyl]-2-thiophenpropionsäure das (S)-α-[(S)-α-[(tert-Butylsulfonyl)-methyl]-2-thiophencarboxamido]-N-[(1S,2R,3S)-1-(cyclohexylmethyl)-3-cyclopropyl-2,3-dihydroxypropyl]-imidazol-4-propionamid als farbloser, amorpher Festkörper, MS : 637 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und (RS)-α-[(tert-Butylsulfonyl)methyl]-β,β-dimethylhydrozimtsäure nach chromatographischer Trennung der Epimeren das (S)-α-[(R oder S)-α-[(tert-Butylsulfonyl)methyl]-β,β-dimethylhydrocinnamamido]-N-[-(1S,2R,3S)-1-cyclohexylmethyl-3-cyclopropyl-2,3-dihydroxypropyl]-3-(4-imidazolyl)propionamid, MS : 659 $(M+H)^+$, und das (S)-α-[(S oder R)-α-[(tert-Butylsulfonyl)methyl]-β,β-dimethylhydrocinnamamido]-N-[-(1S,2R,3S)-1-cyclohexylmethyl-3-cyclopropyl-2,3-dihydroxypropyl]-3-(4-imidazolyl)propionamid, MS : 659 $(M+H)^+$, jeweils als farbloser, amorpher Festkörper.

Die als Ausgangsstoffe eingesetzten Säuren wurden wie folgt hergestellt:

(S)-α-[[[2-(Dimethylamino)-1,1-dimethyläthyl]sulfonyl]methyl]hydrozimtsäure-hydrochlorid

In analoger Weise wie in Beispiel 1(c) beschrieben, wird α-Benzylacrylsäureäthylester mit Natriumäthylat und 1-(Dimethylamino)-2-methyl-2-propanthiol [J.L.Corbin et. al., Inorg.Chem. 1984, 23(21), 3404] zu (RS)-α-[[[2-(dimethylamino)-1,1-dimethyläthyl]sulfonyl]methyl]hydrozimtsäureäthylester umgesetzt, welcher nach in zu Beispiel 1(d) analoger Weise durchgeführten enzymatischen Hydrolyse mittels α-Chymotrypsin das (S)-α-[[[2-(Dimethylamino)-1,1-dimethyläthyl]sulfonyl]methyl]hydrozimtsäure-hydrochlorid liefert; MS : 328 $(M+H)^+$.

(S)-α-[[1,1-Dimethyl-2-morpholinoäthyl)sulfonyl]methyl]hydrozimtsäure-hydrochlorid

Diese Verbindung wird, ebenfalls in analoger Weise wie in Beispiel 1(c) und (d) beschrieben, durch Umsetzung von α-Benzylacrylsäureäthylester mit Natriumäthylat und α,α-Dimethyl-4-morpholinoäthanthiol (Japan.Kokai 78 32,736) und enzymatischer Hydrolyse des (RS)-α-[[1,1-Dimethyl-2-morpholinäthyl)sulfonyl]-methyl]hydrozimtsäureäthylesters mittels α-Chymotrypsin erhalten. MS : 370 $(M+H)^+$.

(S)-α-[[[2-[1-(Benzyloxy)formamido]-2-methylpropyl]sulfonyl]methyl]hydrozimtsäure

In analoger Weise wie in Beispiel 1(c) beschrieben, wird α-Benzylacrylsäureäthylester mit Natriummethylat und tert-Butyl (2-mercapto-1,1-dimethyläthyl)carbamat zu tert-Butyl [2-[[2-(äthoxycarbonyl)-3-phenylpropyl]sulfonyl]-1,1-dimethyl]carbamat umgesetzt. Nach Abspaltung der Boc-Schutzgruppe mit Salzsäure in Dioxan und Einführung der Benzylyloxycarbonyl-Schutzgruppe mit Benzyloxycarbonyloxysuccinimid/Triäthylamin und anschliessender enzymatischer Hydrolyse mittels α-Chymotrypsin, wie in Beispiel 1(d) beschrieben, erhält man die (S)-α-[[[2-[1-(Benzyloxy)formamido]-2-methylpropyl]sulfonyl]-methyl]hydrozimtsäure als farbloses Oel; MS : 389 $(M - CO_2)^+$.

Das als Ausgangsstoff eingesetzte tert-Butyl (2-mercapto-1,1-dimethyläthyl)carbamat wurde wie folgt hergestellt:

In analoger Weise wie in Beispiel 1(a) und (b) beschrieben, wurde diese Verbindung aus 2-(Benzylthio)-1,1-dimethyläthylämin-hydrochlorid [J.L.Corbin et al., Inorg.Chem. 1984, 23(21), 3404] hergestellt. MS : 205 $(M)^+$.

(S)-α-[[[2-[1(Benzyloxy)formamido]-2-methylpropyl]sulfonyl]methyl]-p-fluorhydrozimtsäure:

In analoger Weise wie oben beschrieben, wird, ausgehend von 2-(p-fluorbenzyl)acrylsäureäthylester und tert-Butyl (2-mercapto-1,1-dimethyläthyl)carbamat, nach enzymatischer Hydrolyse die (S)-α-[[[2-[1-(Benzyloxy)formamido]-2-methylpropyl]sulfonyl]methyl]-p-fluorhydrozimtsäure als farbloses Oel erhalten; MS : 502 $(M + Na)^+$.

Der als Ausgangsstoff eingesetzte 2-(p-Fluorbenzyl)acrylsäureäthylester wurde wie folgt hergestellt:

Diese Verbindung wurde in Analogie zu der in Synthesis 1979, 29 beschriebenen Methode, ausgehend von p-Fluorbenzylmalonsäurediäthylester (A.Chraibi, Ann.Pharm.Francaises, 38 , 1980, 343), erhalten, MS : 208 $(M^+)$.

(S)-α-[(tert-Butylsulfonyl)methyl]cyclohexanpropionsäure:

Ein Gemisch von 2 g (7 mMol) (S)-α-[(tert-Butylsulfonyl)methyl]hydrozimtsäure (EPA 0236734) und 2 g Rhodium/Aluminiumoxid-Katalysator (5%ig) in 100 ml Aethanol werden 10 Stunden bei 50° und 10 bar hydriert. Nach dem Abfiltrieren des Katalysators wird die Reaktionslösung unter vermindertem Druck eingedampft, und der Rückstand aus einem 9:1-Gemisch von Hexan und Essigester umkristallisiert. Man erhält 1.6 g (S)-α-[(tert-Butylsulfonyl)methyl]cyclohexanpropionsäure als farblose Nadeln; MS : 308 $(M + NH_4)^+$.

(S)-α-(tert-Butylsulfonyl)methyl]-2-thiophenpropionsäure:

Diese Verbindung wird analog Beispiel 1(d) durch enzymatische Hydrolyse des (S)-α-(tert-Butylsulfonyl)methyl]-2-thiophenpropionsäureäthylesters hergestellt, der seinerseits, analog der in EPA 0236734 beschriebenen Synthese von (RS)-α-[(tert-Butylsulfonyl)methyl]hydrozimtsäureäthylester aus 2-Thionylmethylmalonsäurediäthylester (P.Cagniant et al., Bull.Soc.Chim.Fr. 1954, 1349), hergestellt wird. MS : 318 $(M)^+$.

(RS)-α-(tert-Butylsulfonyl)methyl]-β,β-dimethylhydrozimtsäure:

Diese Verbindung wird analog zu der in EPA 0236734 beschriebenen Synthese von (RS)-α-[(tert-Butylsulfonyl)methyl]hydrozimtsäure, ausgehend von Benzyl-α,α-di methylmalonsäurediäthylester (C.Holmberg et al., Liebigs Ann.Chem. 1981, 748) hergestellt; MS : 330 $(M + NH_4)^+$.

## Beispiel 3

In analoger Weise wie in Beispiel 1 beschrieben, wurden die folgenden Verbindungen hergestellt:

- Aus (S)-α-Amino-N-[(1S,2R,3S)-1-(cyclohexylmethyl)-3-cyclopropyl-2,3-dihydroxypropyl]imidazol-4-propionamid und (S)-α-[(Morpholinosulfonyl)methyl]hydrozimtsäure das (S)-N-[(1S,2R,3S)-1-(Cyclohexylmethyl)-3-cyclopropyl-2,3-dihydroxypropyl]-α-[(S)-α-(morpholinosulfonyl)methyl]hydrocinnamamido]imidazol-4-propionamid als farbloser, amorpher Festkörper, MS : 660 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und (S)-α-[[[(S)-2-(tert-Butyloxycarbonyl)-1-pyrrolidinyl]sulfonyl]methyl]hydrozimtsäure das 1-[[(S)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3-phenylpropyl]sulfonyl]-L-prolin-tert-butylester als farbloser, amorpher Festkörper, MS : 744 $(M+H)^+$.

Die als Ausgangsstoffe eingesetzten Hydrozimtsäuren wurden wie folgt hergestellt:

(S)-α-[(Morpholinosulfonyl)methyl]hydrozimtsäure:

Zu einer Lösung von 6.23 g (21.4 mMol) 2-Benzyl-3-chlorsulfonyl-propionsäureäthylester (EPA 0236734) in 150 ml Methylenchlorid werden bei -10° 6.6 g (75.8 mMol) Morpholin getropft. Die Lösung

wird 1 Stunde bei -10° und 1 Stunde bei 0° gerührt und anschliessend mit 1 N Salzsäure angesäuert. Die organische Phase wird danach mit 50 ml Wasser extra hiert, über Natriumsulfat getrocknet und eingedampft. Der Rückstand (7.64 g) wird zur Reinigung an 300 g Kieselgel unter Verwendung von Methylenchlorid als Eluierungsmittel chromatographiert. Es werden 6.78 g (RS)-α-[(Morpholinosulfonyl)methyl]-hydrozimtsäureäthylester als farbloses Oel erhalten; MS : 341 $(M)^+$. Dieses wird in analoger Weise wie in Beispiel 1(d) beschrieben, durch enzymatische Hydrolyse mittels α-Chymotrypsin in (S)-α-[-(Morpholinosulfonyl)methyl]hydrozimtsäure übergeführt.

(S)-α[[[(S)-2-(tert-Butyloxycarbonyl)-1-pyrrolidinyl]sulfonyl]methyl]hydrozimtsäure:

In analoger Weise wie oben beschrieben, wird durch Umsetzung von 2-Benzyl-3-chlorsulfonylpropionsäureäthylester mit L-Prolin-tert-butylester in Gegenwart von Triäthylamin der 1-[[(RS)-2-(Aethoxycarbonyl)-3-phenylpropyl]sulfonyl]-L-prolin-tert-butylester erhalten, der durch enzymatische Hydrolyse mittels α-Chymotrypsin in (S)-α-[[[(S)-2-(tert-Butyloxycarbonyl)-1-pyrrolidinyl]sulfonyl]methyl]hydrozimtsäure übergeführt wird.

## Beispiel 4

Ein Gemisch von 1.98 g (3.06 mMol) (S)-1-(tert-Butoxycarbonyl)-α-[(S)-α-[(tert-butylsulfonyl)methyl]-hydrocinnamamido]imidazol-4-propionsäure, 0.58 g (2.55 mMol) (1S,2R,3S)-3-Amino-4-cyclohexyl-1-cyclopropyl-1,2-butandiol, 0.5 g (3.06 mMol) 3,4-Dihydro-3-hydroxy-4-oxo-1,2,3-benzotriazin, 0.42 ml (3.07 mMol) Triäthylamin und 1.16 g (3.06 mMol) HBTU in 30 ml Dimethylformamid wird bei Raumtemperatur unter Argon 2 Stunden gerührt. Anschliessend wird das Dimethylformamid im Hochvakuum abgedampft und der Rückstand in 60 ml Essigester aufgenommen. Die Essigesterlösung wird mit 30 ml kalter 2N Natriumbicarbonat-Lösung extrahiert und die Wasserphase mit zweimal 60 ml Essigester nachgewaschen. Die vereinigten organischen Phasen werden der Reihe nach mit 30 ml kalter, gesättigter Ammoniumchlorid-Lösung, 30 ml kalter 2N Natriumbicarbonat-Lösung und 30 ml gesättigter Natriumchlorid-Lösung gewaschen. Die organische Phase wird über Natriumsulfat getrocknet und unter vermindertem Druck eingedampft. Es werden 2.82 g (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]-1-Boc-imidazol-4-propionamid als hellgelber Schaum erhalten.

Die als Ausgangsstoff eingesetzte (S)-1-(tert-Butoxycarbonyl)-α-[(S)-α-[(tert-butylsulfonyl)methyl]-hydrocinnamamido]imidazol-4-propionsäure wurde wie folgt hergestellt:

(a) (S)-α-[(S)-α-[(tert Butylsulfonyl)methyl]hydrocinnamamido]imidazolpropionsäuremethylester:

Ein Gemisch von 5.7 (20 mMol) (S)-α-[(tert-Butylsulfonyl)methyl)hydrozimtsäure (EPA 0236734) 4.85 g (20 mMol) L-Histidinmethylester-dihydrochlorid, 10.3 ml (81.7 mMol) N-Aethylmorpholin und 2.98 g (20 mMol) HOBT in 85 ml Dimethylformamid wird unter Argon bei 0-2° portionsweise mit 4.23 g (22 mMol) EDC versetzt und anschliessend über Nacht bei Raumtemperatur gerührt. Danach wird das Lösungsmittel im Hochvakuum abgedampft, der Rückstand in 100 ml Essigester aufgenommen und der Reihe nach dreimal mit 10 ml 2N Natriumbicarbonat-Lösung, zweimal mit 10 ml gesättigter Natriumchlorid-Lösung, zweimal mit 10 ml gesättigter Ammoniumchlorid-Lösung und zweimal mit 5 ml gesättigter Natriumchlorid-Lösung gewaschen. Die Wasserphasen werden dreimal mit 20 ml Essigester extrahiert, und die gesammelten organischen Phasen über Natriumsulfat getrocknet und unter vermindertem Druck eingedampft. Das erhaltene Rohprodukt (9.4 g) wird zur Reinigung an 300 g Kieselgel unter Verwendung eines 95:5-Gemisches von Methylenchlorid und Methanol als Eluierungsmittel chromatographiert. Es werden 8.6 g (S)-α-(S)-α-[(tert-Butylsulfonyl)methyl]hydrocinnam amido]imidazolpropionsäuremethylester als farbloser Schaum erhalten; MS : 436 $(M+H)^+$.

(b) (S)-1-(tert-Butoxycarbonyl)-α-[(S)-α-[(tert-butylsulfonyl)methyl]hydrocinnamamido]imidazol-4-propionsäure:

Eine Lösung von 1.33 g (3.06 mMol) (S)-α-[(S)-α-[(tert-Butylsulfonyl)methyl]hydrocinnamamido]imidazolpropionsäuremethylester in 4 ml Dioxan wird unter Eiskühlung mit 3.98 ml 1N Natronlauge tropfenweise versetzt und 30 Minuten lang bei dieser Temperatur gerührt, wobei der Methylester quantitativ in die entsprechende Säure übergeführt wird.

Anschliessend wird das Reaktionsgemisch bei 0-5° tropfenweise mit einer Lösung von 0.8 g (3.67 mMol) Di-tert-butyldicarbonat in 4 ml Dioxan versetzt und nach Entfernen des Eisbades während 5 Stunden bei Raumtemperatur gerührt. Danach werden 3.98 ml 1N Salzsäure und 25 ml Wasser zugefügt und mit 55 ml Essigester extrahiert. Die Wasserphase wird mit 50 ml Essigester nachgewaschen, und die vereinigten organischen Phasen werden zweimal mit 25 ml Wasser gewaschen, über Natriumsulfat getrocknet und unter vermindertem Druck eingedampft. Es werden 1.98 g (S)-1-(tert-Butoxycarbonyl)-α-[(S)-α-[(tert-butylsulfonyl)methyl]hydrocinnamamido]imidazol-4-propionsäure als Schaum erhalten; MS : 522 $(M+H)^+$.

## Beispiel 5

In analoger Weise wie in Beispiel 4 beschrieben, wird durch Kondensation von N-[(S)-α-[(tert-Butylsulfonyl)methyl]hydrocinnamoyl]-1-(2,4-dinitrophenyl)-L-histidin mit (1S,2R,3S)-3-Amino-4-cyclohexyl-1-cyclopropy-1,2-butandiol das (S)-α-[(S)-α-[(tert-Butylsulfonyl)methyl]hydrocinnamamido]-N-[(1S,2R,3S)-1-(cyclohexylmethyl)-3-cyclopropyl- 2,3-dihydroxypropyl]-1-(2,4-dinitrophenyl)imidazol-4-propionamid als gelber Festkörper erhalten; MS : 797 $(M+H)^+$.

Das als Ausgangsstoff eingesetzte N-[(S)-α-[(tert-Butylsulfonyl)methyl]hydrocinnamoyl]-1-(2,4-dinitrophenyl)-L-histidin wurde wie folgt hergestellt:

23.3 g (54.6 mMol) (S)-α-[(S)-α-[(tert-Butylsulfonyl)methyl]hydrocinnamamido]-imidazolpropionsäuremethylester in 250 ml Methylenchlorid werden mit 7,43 ml (54,6 mMol) Triäthylamin versetzt. Anschliessend werden unter Eiskühlung 9,94 g (54,6 mMol) 2,4-Dinitro-1-fluorbenzol in 100 ml Methylenchlorid innerhalb von etwa 20 Minuten zugetropft, und das Reaktionsgemisch bis zu vollständigen Umsetzung bei Raumtemperatur gerührt, wobei dies nach 4 Stunden der Fall ist (dünnschichtchromatographisch überprüft). Uebliche Aufarbeitung des Reaktionsgemisches liefert 20,5 g (62%) N-[(S)-α-[(tert-Butylsulfonyl)methyl]hydrocinnamoyl]-1-(2,4-dinitrophenyl)-L-histidinmethylester als braunen Schaum, Rf-Wert 0,4 in einem 30:1-Gemisch von Methylenchlorid und Methanol, MS: 602 $(M+H)^+$.

20,5 g (34,07 mMol) N-[(S)-α-[(tert-Butylsulfonyl)methyl]hydrocinnamoyl]-1-(2,4-dinitrophenyl)-L-histidinmethylester werden in 180 ml Dioxan gelöst, mit 85 ml (170,34 mMol) 2N Salzsäure versetzt und anschliessend 2,5 Stunden auf 80° erhitzt. Uebliche Aufarbeitung und Kristallisation aus Aether/Hexan liefert 15,7 g (78%) N-[(S)-α-[(tert-Butylsulfonyl)methyl]hydrocinnamoyl]-1-(2,4-dinitrophenyl)-L-histidin in Form eines hellgelben amorphen Festkörpers, Rf-Wert 0,2 in einem 5:1-Gemisch von Methylenchlorid und Methanol, MS: 588 $(M+H)^+$.

## Beispiel 6

In analoger Weise wie in Beispiel 4 beschrieben, wurden folgende Verbindungen hergestellt:

- Aus (S)-1-(tert-Butoxycarbonyl)-α-[(S)-α-[(tert-butylsulfonyl)methyl]hydrocinnamamido]imidazol-4-propionsäure und (1S,2R,3S)-3-Amino-1-cyclopropyl-4-(4,4-difluorcyclohexyl)-1,2-butandiol das (S)-α-[(S)-α-(tert-Butylsulfonyl)methyl]hydrocinnamamido]-N-[(1S,2R,3S)3-cyclopropyl-1-[(4,4-difluorcyclohexyl)methyl]-2,3-dihydroxypropyl]-1-Boc-imidazol-4-propionamid;
- aus (S)-1-(tert-Butoxycarbonyl)-α-[(S)-α-[(tert-butylsulfonyl)methyl]hydrocinnamamido]imidazol-4-propionsäure und (1S,2R)-3-Amino-1-cyclopropyl-4-[(RS)-4-hydroxycyclohexyl]-1,2-butandiol das (S)-α-[ (S)-α-[(tert-Butylsulfonyl)methyl]hydrocinnamamido]-N-[(1S,2R,3S)-1-[[(RS)-4-hydroxycyclohexyl]methyl]-3-cyclopropyl-2,3-dihydroxypropyl]-1-Boc-imidazol-4-propionamid;
- aus (S)-1-(tert-Butoxycarbonyl)-α-[(S)-α-[(tert-butylsulfonyl)methyl]hydrocinnamamido]imidazol--4-propionsäure und (1S,2R,3S)-3-Amino-1-cyclopropyl-4-(p-fluorphenyl)-1,2-butandiol das (S)-α-[(S)-α-[(tert-Butylsulfonyl)methyl]hydrocinnamamido]-N-[(1S,2R,3S)-3-cyclopropyl-1-(p-fluorbenzyl)-2,3-dihydroxypropyl]-1-Boc-imidazol-4-propionamid;
- aus (S)-1-(tert-Butoxycarbonyl)-α-[(S)-α-[(tert-butylsulfonyl)methyl]hydrocinnamamido] imidazol-4-propionsäure und (1S,2R,3S)-3-Amino-4-[(S)-3-cyclohexen-1-yl]-1-cyclopropyl-1,2-butandiol das (S)-α-[(S)-α-[(tert-Butylsulfonyl)methyl]hydrocinnamamido]-N-[(1S,2R,3S)-1-[[(S)-3-cyclohexen-1-yl]methyl]-3-cyclopropyl-2,3-dihydroxypropyl]-1-Boc-imidazol-4-propionamid;

- aus (S)-1-(tert-Butoxycarbonyl)-α-[(S)-α-[(tert-butylsulfonyl)methyl]hydrocinnamamido]imidazol-4-propionsäure und (1S,2R,3S)-3-Amino-4-[(R)-3-cyclo hexen-1-yl]-1-cyclopropyl-1,2-butandiol das (S)-α-[(S)-α-[(tert-Butylsulfonyl)methyl]hydrocinnamamido]-N-[(1S,2R,3S)-1-[[(R)-3-cyclohexen-1-yl]methyl]-3-cyclopropyl-2,3-dihydroxypropyl]-1-Boc-imidazol-4-propionamid;
- aus (S)-1-(tert-Butoxycarbonyl)-α-[(S)-α-[(tert-butylsulfonyl)methyl]hydrocinnamamido]imidazol-4-propionsäure und (2RS,3R,4S)-4-Amino-5-cyclohexyl-1,2,3-pentantriol das (S)-α-[(S)-α-[(tert-Butylsulfonyl)methyl]-hydrocinnamamido]-N-[(1S,2R,3RS)-1-(cyclohexylmethyl)-2,3,4-trihydroxybutyl]-1-Boc-imidazol-4-propionamid;
- aus (s)-1-(tert-Butoxycarbonyl)-α-[(S)-α-[(tert-butylsulfonyl)methyl]hydrocinamamido]imidazol-4-propionsäure und (2S,3R,4R oder S,5R oder S)-2-Amino-1-cyclohexyl-3,4,5-heptantriol das (S)-α-[(S)-α-[(tert-Butylsulfonyl)methyl]hydrocinnamamido]-N-[(1S,2R,3R oder S,4R oder S)-1-(cyclohexylmethyl)-2,3,4-trihydroxyhexyl]-1-Boc-imidazol-4-propionamid.

Die als Ausgangsstoffe eingesetzten Aminodiole wurden analog zu dem Verfahren hergestellt, das in WO 87/05302 zur Synthese des tert-Butyl (4S,5R)-4-(cyclohexylmethyl)-5-formyl-2,2-dimethyl-3-oxazolidincarboxylats beschrieben ist, ausgehend vom Boc-Cyclohexylalaninmethylester gefolgt von der Grignard-Reaktion mit Cyclopropylmagnesiumbromid und Abspaltung der Boc- und Isopropyliden-Schutzgruppe analog Beispiel 1(f). Der Ersatz des Boc-Cyclohexylalaninmethylesters ergibt, ausgehend

- von Boc(4,4-Difluorcyclohexyl)alaninmethylester das (1S,2R,3S)-3-Amino-1-cyclopropyl-4-(4,4-difluorcyclohexyl)-1,2-butandiol als farblosen Festkörper, MS : 264 $(M+H)^+$;
- von Boc-[(RS)-4-Hydroxycyclohexyl]alaninmethylester das (1S,2R)-3-Amino-1-cyclopropyl-4-[(RS)-4-hydroxycyclohexyl]-1,2-butandiol als amorphen Feststoff, MS : 244 $(M+H)^+$;
- von Boc-(p-Fluorphenyl)alaninmethylester das (1S,2R,3S)-3-Amino-1-cyclopropyl-4-(p-fluorphenyl)-1,2-butandiol als farblosen Festkörper, MS : 240 $(M+H)^+$;
- von Boc-3-[(S)-2-Cyclohexen-1-yl]-L-alaninmethylester das (1S,2R,3S)-3-Amino-4-[(S)-3-cyclohexen-1-yl]-1-cyclopropyl-1,2-butandiol als gelbliches Oel, MS : 154 $(M-C_4H_7O)^+$;
- von Boc-3-[(R)-2-Cyclohexen-1-yl]-L-alaninmethylester das (1S,2R,3S)-3-Amino-4-[(R)-3-cyclohexen-1-yl]-1-cyclopropyl-1,2-butandiol als gelbliches Oel, MS : 225 $(M)^+$.

Die als Ausgangsstoffe eingesetzten Aminotriole wurden wie folgt hergestellt:

2RS,3R,4S)-4-Amino-5-cyclohexyl-1,2,3-pentantriol

(a) tert-Butyl [(1S,2R,3RS)-1-(cyclohexylmethyl) 2,3,4-trihydroxybutyl]carbamat:

Eine Lösung von 1.5 g (2.24 mMol) tert-Butyl [(1S,2S)-1-(cyclohexylmethyl)-2-hydroxy-3-butenyl]-carbamat, hergestellt nach dem Verfahren von J.J.Plattner et al. in J.Med.Chem., 30 , (10), (1987), 1729, in 10 ml Pyridin wird mit 845 mg (3.36 mMol) Osmiumtetroxid versetzt und während 3 Tagen bei Raumtemperatur im Dunkeln stehen gelassen. Anschliessend wird das Reaktionsgemisch auf 0° abgekühlt, mit 3 ml 38%iger Natriumbisulfit-Lösung versetzt und 2 Stunden gerührt. Danach wird auf Eis und Wasser gegossen und dreimal mit 250 ml Essigester extrahiert. Die vereinigten organischen Extrakte werden nacheinander mit jeweils 60 ml Natriumbicarbonat-, 20%iger Kupfersulfat- und gesättigter Natriumchlorid-Lösung gewaschen, über Magnesiumsulfat getrocknet und unter vermindertem Druck eingedampft. Der Rückstand (1.20 g) wird zur Reinigung an 30 g Kieselgel unter Verwendung eines 100:10:1-Gemisches von Methylenchlorid, Methanol und Ammoniak als Eluierunsmittel chromatographiert, wobei 590 mg tert-Butyl [(1S,2R,3RS)-1-(cyclohexylmethyl)-2,3,4-trihydroxybutyl]carbamat als Schaum erhalten werden, MS : 318 $(M+H)^+$.

(b) (2RS,3R,4S)-4-Amino-5-cyclohexyl-1,2,3-pentantriol:

Eine Lösung 270 mg tert-Butyl [(1S,2R,3RS)-1-(cyclohexylmethyl)-2,3,4-trihydroxybutyl]carbamat, 15 ml Methanol und 10 ml 2N Salzsäure wird über Nacht auf 50° erwärmt. Anschliessend wird die Reaktionslösung unter vermindertem Druck zur Trockene eingedampft, der erhaltene Rückstand zweimal in Toluol aufgenommen und jeweils unter vermindertem Druck zur Trockene eingedampft. Das erhaltene rohe (2RS,3R,4S)-4-Amino-5-cyclohexyl-1,2,3-pentantriol wird ohne weitere Reinigung in der nächsten Stufe eingesetzt; MS : 218 $(M+H)^+$.

(2S,3R,4R oder S,5R oder S)-2-Amino-1-cyclohexyl-3,4,5-heptantriol

(c) tert-Butyl (4S,5R)-5-[(Z)-1-butenyl]-4-(cyclohexylmethyl)-2,2-dimethyl-3-oxazolidincarboxylat:

Ein Gemisch von 1,4 g (4,3 mMol) tert-Butyl (4S,5R)-4-(cyclohexylmethyl)-5-formyl-2,2-dimethyl-3-oxazolidincarboxylat (WO 87/05302) und 1,79 g (4,3 mMol) Instantylid, bestehend aus Propyltriphenylphosphoniumbromid und Natriumamid, in 50 ml Tetrahydrofuran wird 30 Minuten unter Stickstoff bei Raumtemperatur gerührt. Anschliessend wird das Reaktionsgemisch zwischen Wasser und Essigester verteilt. Die organische Phase wird abgetrennt, über Natriumsulfat getrocknet und unter vermindertem Druck eingedampft. Der Rückstand wird zur Reinigung an 80 g Kieselgel unter Verwendung eines 1:1-Gemisches von Methylenchlorid und Petroläther als Eluierungsmittel chromatographiert. Man erhält 900 mg tert-Butyl (4S,5R)-5-[(Z)-1-butenyl]-4-(cyclohexylmethyl)-2,2-dimethyl-3-oxazolidincarboxylat als Harz; MS : 352 $(M+H)^+$.

(d) tert-Butyl (4S,5R)-4-(cyclohexylmethyl)-5-[(1R oder S,2R oder S)-1,2-dihydroxybutyl]-2,2-dimethyl-3-oxazolidincarboxylat:

Zu einer Lösung von 900 mg (2,68 mMol) tert-Butyl (4S,5R)-5-[(Z)-1-butenyl-4-(cyclohexylmethyl)-2,2-dimethyl-3-oxazolidincarboxylat und 832 mg (6,16 mMol) 4-Methylmorpholin-4-oxid-monohydrat in 20 ml Tetrahydrofuran werden unter Argon 22 mg (0,089 mMol) Osmiumtetroxid in Form einer 2-%igen Methylenchloridlösung gegeben. Das Reaktionsgemisch wird 2,5 Stunden bei Raumtemperatur gerührt. dann mit 150 ml gesättigter Natriumchlorid-Lösung versetzt und mit 150 ml Essigester extrahiert. Die organische Phase wird nacheinander mit 10%iger Natriumsulfit-Lösung. 1N Phosphorsäure und Wasser ausgeschüttelt, über Natriumsulfat getrocknet und unter vermindertem Druck eingedampft. Der Rückstand wird zur Reinigung an Kieselgel unter Verwendung eines 2:1-Gemisches von Methylenchlorid und Petroläther als Eluierungsmittel chromatographiert. Es werden 370 mg tert-Butyl (4S,5R)-4-(cyclohexylmethyl)-5-[(1R oder S,2R oder S)-1,2-dihydroxybutyl]-2,2-dimethyl-3-oxazolidincarboxylat als Harz erhalten; MS : 370 $(M-CH_3)^+$.

(e) (2S,3R,4R oder S,5R oder S)-2-Amino-1-cyclohexyl-3,4,5-heptantriol:

Analog zu dem oben zur Herstellung des (2RS,3R,4S)-4- Amino-5-cyclohexyl-1,2,3-pentantriols beschriebenen Verfahren, wird durch Umsetzung von tert-Butyl(4S,5R)-4-(cyclohexylmethyl)-5-[(1R oder S,2R oder S)-1,2-dihydroxybutyl]-2,2-dimethyl-3-oxazolidincarboxylat das (2S,3R,4R oder S,5R oder S)-2-Amino-1-cyclohexyl-3,4,5-heptantriol als amorpher Festkörper erhalten, welcher ohne weitere Reinigung in die nächste Stufe eingesetzt wird.

Die als Ausgangsstoffe eingesetzten Alaninmethylester wurden wie folgt hergestellt:

Boc-(4,4-Difluorcyclohexyl)alaninmethylester und Boc-[(RS)-4-Hydroxycyclohexyl]alaninmethylester

(f) Eine Suspension von 65.8 g (0.22 Mol) Boc-Tyrosin-methylester und 6.6 g Rhodium/Aluminiumoxid-Katalysator (5%ig) in 175 ml Methanol wird 3 Stunden bei 50° und 4 bar Wasserstoff hydriert. Anschliessend wird der Katalysator abfiltriert, das Filtrat unter vermindertem Druck eingedampft, und der erhaltene Rückstand zur Reinigung an 1 kg Kieselgel unter Verwendung eines 4:1-Gemisches von Toluol und Essigester als Eluierungsmittel chromatographiert. Es werden 41.97 g Boc-[(RS)-4-Hydroxycyclohexyl]-alaninmethylester, MS : 302 $(M+H)^+$, und 6.1 g Boc-(4-oxocyclohexyl)alaninmethylester, MS : 300 $(M+H)^+$, jeweils als farbloses Oel erhalten.

(g) Zu einer Lösung von 322 mg (2 mMol) Diäthylamino-Schwefeltrifluorid in 0.5 ml Methylenchlorid wird bei -78° eine Lösung von 300 mg (1 mMol) Boc-(4-oxocyclohexyl)alaninmethylester in 1.5 ml Methylenchlorid mittels einer Spritze gegeben. Man lässt das Reaktionsgemisch auf -10° erwärmen und rührt 1.5 Stunden bei dieser Temperatur nach. Danach wird auf Eis und 30 ml 2N Natriumhydrogencarbonat-Lösung gegossen und zweimal mit je 60 ml Methylenchlorid extrahiert. Die organische Phase wird mit 30 ml Wasser gewaschen, über Natriumsulfat getrocknet und eingedampft. Das Rohprodukt (450 mg) wird an 30 g Kieselgel unter Verwendung eines 9:1-Gemisches von Hexan und Essigester als Eluierungsmittel chromatographiert. Es werden 280 mg Boc-(4,4-Difluorcyclohexyl)-

alaninmethylester als Oel erhalten; MS : 322 $(M+H)^+$.

Boc-3-[(S)-2-cyclohexen-1-yl]-L-alaninmethylester

(h) (S)-3-Cyclohexen-1-methanol:

Zu einer Lösung von 3,57 g (15 mMol) (R)-Tetrahydro-4,4-dimethyl-2-oxo-3-furyl (S)-3-cyclohexen-1-carboxylat (G.Helmchen et al., Tetrahedron Letters 1985, 26 , 3095-3098) in 60 ml absolutem Tetrahydrofuran und 20 ml absolutem Aether werden 0,57 g (15 mMol) Lithiumaluminiumhydrid gegeben. Das Gemisch wird 1 Stunde zum Rückfluss erhitzt, danach auf -20° abgekühlt, mit gesättigter Ammoniumchlorid-Lösung tropfenweise versetzt und mit Aether dreimal extrahiert. Die vereinigten organischen Extrakte werden mit Wasser gewaschen, über Natriumsulfat getrocknet und unter Druck eingeengt. Das zurückbleibende Oel wird im Hochvakuum destilliert. Man erhält bei einer Temperatur von 58° und einem Druck von 0,3 mm Hg (S)-3-cyclohexen-1-methanol als farbloses Oel, MS : 94 $(M-H_2O)^+$.

| Analyse für $C_7H_{12}O$: | | |
|---|---|---|
| Berechnet: | C 74,97; | H 10,78% |
| Gefunden : | C 74,83; | H 11,00%. |

(i) [-(S)-3-Cyclohexen-1-yl]methyl p-toluolsulfonat:

Zu einer auf -10° gekühlten Lösung von 5,6 g (50 mMol) (S)-3-cyclohexen-1-methanol in 20 ml Pyridin wird eine Lösung von 10,48 g (55 mMol) p-Toluolsulfochlorid in 10 ml Pyridin langsam getropft. Die Lösung wird 64 Stunden bei -10° gerührt, danach im Hochvakuum eingedampft, und der Rückstand in Aether aufgenommen. Die Aether lösung wird nacheinander mit 1N Salzsäure, Wasser und gesättigter Natriumbicarbonat-Lösung gewaschen und anschliessend unter vermindertem Druck eingedampft. [(S)-3-Cyclohexen-1-yl]-methyl p-toluolsulfonat wird dabei als farbloses Oel erhalten; MS : 173 $(M)^+$.

(j) (S)-1-(Brommethyl)-3-cyclohexen:

Ein Gemisch von 13,3 g (50 mMol) [(S)-3-Cyclohexen-1-yl]methyl p-toluolsulfonat und 13,0 g (150 mMol) Lithiumbromid in 100 ml absolutem Aceton wird 16 Stunden unter Rückfluss gekocht. Anschliessend wird vom Niederschlag abfiltriert, das Filtrat eingedampft, und der Rückstand in absolutem Aether aufgenommen. Die Aether lösung wird filtriert und eingedampft. Man erhält (S)-1-(Brommethyl)-3-cyclohexen, MS : 174 $(M)^+$, als farbloses Oel, das direkt in der folgenden Stufe eingesetzt wird.

(k) (2S,5R)-2-Cyclohexen-1-ylmethyl]-2,5-dihydro-5-isopropyl-3,6-dimethoxypyrazin:

Eine auf -78° abgekühlte Lösung von 1,78 ml (10 mMol) (2R)-(-)-2-,5-Dihydro-3,6-dimethoxy-2-isopropylpyrazin in 5 ml absolutem Tetrahydrofuran wird tropfenweise mit 6,8 ml (11 mMol) einer Lösung von Butyllithium in Hexan versetzt. Die Reaktionslösung wird 1 Stunde bei -78° und 1 Stunde bei -20° belassen und dann wieder auf -78° abgekühlt. Anschliessend wird eine Lösung von 1,75 g (10 mMol) (S)-1-(Brommethyl)-3-cyclohexen in 5 ml absolutem Tetrahydrofuran zugegeben. Nach 2 Stunden bei Raumtemperatur wird die Lösung wieder auf -10° abgekühlt, mit 5 ml gesättigter Ammoniumchlorid-Lösung versetzt und mit Aether extrahiert. Die organische Phase wird mit Wasser gewaschen, über Natriumsulfat getrocknet und unter vermindertem Druck eingedampft. Der Rückstand wird zur Reinigung zweimal an Kieselgel unter Verwendung eines 9:1-Gemisches von Toluol und Essigester als Eluierungsmittel chromatographiert. (2s,5R)-2-[(S)-3-Cyclohexen-1-ylmethyl]-2,5-dihydro-5-isopropyl-3,6-dimethoxy pyrazin wird als farbloses Oel erhalten; MS : 278 $(M)^+$.

| Analyse für $C_{16}H_{26}N_2O_2$: | | | |
|---|---|---|---|
| Berechnet:<br>Gefunden : | C 69,03;<br>C 69,31; | H 9,41;<br>H 9,52; | N 10,06%<br>N 10,14%. |

(1) Boc-3-[-2-cyclohexen-2-yl]-L-alaninmethylester:

Ein Gemisch von 1,39 g (5 mMol) (2S,5R)-2-[(S)-3-Cyclohexen-1-ylmethyl]-2,5-dihydro-5-isopropyl-3,6-dimethoxypyrazin, 1,90 g (10 mMol) p-Toluolsulfonsäure, 10 ml 0,25N Salzsäure und 5 ml Methanol wird 16 Stunden bei Raumtemperatur und dann 2 Stunden bei 80° gerührt. Anschliessend wird das Reaktionsgemisch unter vermindertem Druck eingedampft, und der Rückstand im Hochvakuum getrocknet. Die so erhaltene feste Masse wird in 30 ml Dimethylformamid suspendiert, mit 1,70 g (7,8 mMol) Di-tert-butyldicarbonat und 1,98 ml (14,3 mMol) Triäthylamin versetzt und 2 Stunden bei Raumtemperatur gerührt. Das Reaktionsgemisch wird danach im Hochvakuum eingedampft, und der Rückstand zwischen Aether und Wasser verteilt. Die Aetherphase wird über Natriumsulfat getrocknet und eingedampft. Der Rückstand wird an Kieselgel unter Verwendung eines 4:1-Gemisches von Toluol und Essigester als Eluierungsmittel chromatographiert. Man erhält Boc-3-[(S)-2-cyclohexen-1-yl]-L-alaninmethylester als farbloses Oel; MS : 227 $(M-C_4H_8)^+$.

| Analyse für $C_{15}H_{25}NO_4$: | | | |
|---|---|---|---|
| Berechnet:<br>Gefunden : | C 62,36;<br>C 62,05; | H 8,93;<br>H 9,02; | N 4,28%<br>N 4,15%. |

Boc-3-[(R)-2-cyclohexen-1-yl]-L-alaninmethylester

In analoger Weise wie oben beschrieben, wird ausgehend von (S)-Tetrahydro-4,4-dimethyl-2-oxo-3-furyl (R)-3-cyclohexen-1-carboxylat, der Boc-3-[(R)-2-cyclohexen-1-yl]-L-alaninmethylester als farbloses Oel erhalten, MS : 227 $(M-C_4H_8)^+$.

Beispiel 7

Eine Lösung von 32 mg (0.043 mMol) tert-Butyl [2-[[(S)-2-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]-3-phenylpropyl]sulfonyl]-2-methylpropyl]carbamat in 2 ml Dioxan wird unter Eiskühlung mit 2 ml (4 mMol) 2N Salzsäure in Dioxan versetzt und 1 Stunde gerührt. Anschliessend wird die Lösung lyophilisiert und man erhält 30 mg (S)-α-[(S)-α-[[[(2-Amino-1,1-dimethyläthyl)sulfonyl]methyl]hydrocinnamamido]-N-[(1S,2R,3S)-1-(cyclohexylmethyl)-3-cyclopropyl-2,3-dihydroxypropyl]imidazol-4-propionamid-dihydrochlorid als farbloses Pulver; MS : 646 $(M+H)^+$.

Beispiel 8

Ein Gemisch von 300 mg (0.38 mMol) Benzyl [2-[[(S)-2-[[(S)-1-[[(1S,2R,3S)-1-(cyclohexylmethyl)-3-cyclopropyl-2,3-dihydroxypropyl]carbamoyl]-2-imidazol-4-yläthyl]carba moyl]-3-phenylpropyl]sulfonyl]-1,1-dimethyläthyl]carbamat und 30 mg Palladium/Kohle (10%ig) in 8 ml Essigsäure wird bei Raumtemperatur während 3 Stunden hydriert. Danach wird der Katalysator abfiltriert, die Reaktionslösung eingedampft, und der erhaltene Rückstand aus Wasser lyophilisiert. Man erhält 240 mg (S)-α-[(S)-α-[[(2-Amino-2-methylpropyl)sulfonyl]methyl]hydrocinnamamido]-N-[(1S,2R,3S)-1-(cyclohexylmethyl)-3-cyclopropyl-2,3-dihydroxypropyl]imidazol-4-propionsäureamiddiacetat als farbloses, amorphes Pulver; MS : 646 $(M+H)^+$.

Beispiel 9

In analoger Weise wie in Beispiel 8 beschrieben, erhält man durch katalytische Hydrierung von Benzyl [2-[[(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]- p-fluorphenäthyl]sulfonyl]-1,1-dimethyläthyl]carbamat das (S)-α-[(S)-α-[[(2-Amino-2-methylpropyl)sulfonyl]methyl]-p-fluorohydrocinnamamido]-N-[(1S,2R,3S)-1-(cyclohexylmethyl)-3-cyclopropyl-2,3-dihydroxypropyl]imidazol-4-propionsäureamid-diacetat als farbloses, amorphes Pulver; MS : 664 $(M+H)^+$.

Beispiel 10

Eine Lösung von 2.82 g (ca. 3.8 mMol) rohem (S)-α-(S)-α-[(tert-Butylsulfonyl)methyl]-hydrocinnamamido]-N-[(1S,2R,3S)-1-(cyclohexylmethyl)-3-cyclopropyl-2,3-dihydroxypropyl]-1-Boc-imidazol-4-propionamid und 0.35g (2.55 mMol) Kaliumcarbonat in 20 ml Methanol wird 1 Stunde bei Raumtemperatur unter Argon gerührt. Danach werden 0.33 g (6.1 mMol) Ammoniumchlorid zugegeben und weitere 15 Minuten bei Raumtemperatur gerührt. Das Reaktionsgemisch wird anschliessend unter vermindertem Druck eingedampft, der Rückstand in 60 ml Essigester aufgenommen, und diese Lösung nacheinander mit 30 ml Wasser und 30 ml gesättigter Natriumchlorid-Lösung gewaschen. Die Wasserphasen werden zweimal mit 60 ml Essigester nachextrahiert. Die vereinigten organischen Phasen werden über Natriumsulfat getrocknet und unter vermindertem Druck eingedampft. Der Rückstand wird in 15 ml Aether verrührt, dann abgesaugt und zur weiteren Reinigung an 150 g Kieselgel unter Verwendung eines 4:1:0.1-Gemisches von Methylenchlorid, Methanol und Ammoniak als Eluierungsmittel chromatographiert. Es werden 1.13 g (S)-α-[(S)-α-[-(tert-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid als farbloser Festkörper erhalten; MS : 631 $(M+H)^+$.

Beispiel 11

In analoger Weise wie in Beispiel 10 beschrieben, wurden folgende Verbindungen hergestellt:

- Aus (S)-α-[(S)-α-[(tert-Butylsulfonyl)methyl]hydrocinnamamido]-N-[(1S;2R,3S)-3-cyclopropyl-1-[(4,4-difluorcyclohexyl)methyl]-2,3-dihydroxypropyl]-1-Boc-imidazol-4-propionamid das (S)-α-[(S)-α-[(tert-Butylsulfonyl)-methyl]hydrocinnamamido]-N-[(1S,2R,3S)-3-cyclopropyl-1-[(4,4-difluorcyclohexyl)methyl]-2,3-dihydroxypropyl]imidazol-4-propionamid als farbloser Festkörper, MS : 667 $(M+H)^+$;
- aus (S)-α-[(S)-α-[(tert-Butylsulfonyl)methyl]hydrocinnamamido]-N-[(1S,2R,3S)-1-[[(RS)-4-hydroxycyclo hexyl]methyl]-3-cyclopropyl-2,3-dihydroxypropyl]-1-Boc-imidazol-4-propionamid das (S)-α-[(S)-α-[(tert-Butylsulfonyl)methyl]hydrocinnamamido]-N-[(1S,2R,3S)-1-[[(RS)-4-hydroxycyclohexyl]methyl]-3-cyclopropyl-2,3-dihydroxypropyl]imidazol-4-propionamid als farbloser Festkörper, MS : 643 $(M+H)^+$;
- aus (S)-α-[(S)-α-[(tert-Butylsulfonyl)methyl]hydrocinnamamido]-N-[(1S,2R,3S)-3-cyclopropyl-1-(p-fluorbenzyl)-2,3-dihydroxypropyl]-1-Boc-imidazol-4-propionamid das (S)-α-[(S)-α-[(tert-Butylsulfonyl)methyl]-hydrocinnamamido]-N-[(1S,2R,3S)-3-cyclopropyl-1-(p-fluorbenzyl)-2,3-dihydroxypropyl]-imidazol-4-propionamid als farbloser Festkörper, MS : 643 $(M+H)^+$;
- aus (S)-α-[(S)-α-[(tert-Butylsulfonyl)methyl]hydrocinnamamido]-N-[(1S,2R,3S)-1-[(S)-3-cyclohexen-1-ylmethyl]-3-cyclopropyl-2,3-dihydroxypropyl]-1-Boc-imidazol-4-propionamid das (S)-α-[(S)-α-[(tert-Butylsulfonyl)methyl]hydrocinnamamido]-N-[(1S,2R,3S)-1-[(S)-3-cyclohexen-1-ylmethyl]-3-cyclopropyl-2,3-dihydroxy propyl]imidazol-4-propionamid als amorpher Festkörper, MS : 737 $(M+H+Matrix)^+$;
- aus (S)-α-[(S)-α-[(tert-Butylsulfonyl)methyl]hydrocinnamamido]-N-[(1S,2R,3S)-1-[(R)-3-cyclohexen-1-ylmethyl]-3-cyclopropyl-2,3-dihydroxypropyl]-1-Boc-imidazol-4-propionamid das (S)-α-[(S)-α-[(tert-Butylsulfonyl)methyl]hydrocinnamamido]-N-[(1S,2R,3S)-1-[(R)-3-cyclohexen-1-ylmethyl]-3-cyclopropyl-2,3-dihydroxypropyl]imidazol-4-propionamid als amorpher Festkörper, MS : 629 $(M)^+$;
- aus (S)-α-[(S)-α-[(tert-Butylsulfonyl)methyl]hydrocinnamamido]-N-[(1S,2R,3RS)-1-(cyclohexylmethyl)-2,3,4-trihydroxybutyl]-1-Boc-imidazol-4-propionamid das (S)-α-[(S)-α-[(tert-Butylsulfonyl)methyl]-hydrocinnamamido]-N-[(1S,2R,3RS)-1-(cyclohexylmethyl)-2,3,4-trihydroxybutyl]-imidazol-4-propionamid als farbloser Festkörper, MS : 621 $(M+H)^+$;

- aus (S)-α-[(S)-α-[(tert-Butylsulfonyl)methyl]hydrocinnamamido]-N-[(1S,2R,3R oder S,4R oder S)-1-(cyclohexylmethyl)-2,3,4-trihydroxyhexyl]-1-Boc-imidazol-4-propionamid das (S)-α-[(S)-α-[(tert-Butylsulfonyl)-methyl]hydrocinnamamido]-N-[(1S,2R,3R oder S,4R oder S)-1(cyclohexylmethyl)-2,3,4-trihydroxyhexyl]-imidazol4-propionamid als kristalliner Festkörper, MS : 649 $(M+H)^+$.

## Beispiel 12

Ein Gemisch von 100 mg (0.16 mMol) (S)-α-[(S)-α-[(tert-Butylsulfonyl)methyl]hydrocinnamamido]-N-[-(1S,2R,3S)-1-(cyclohexylmethyl)-3-cyclopropyl-2,3-dihydroxypropyl]imidazol-4-propionamid, 46 mg (0.24 mMol) 2-(Brommethyl)acrylsäureäthylester und 0.044 ml (0.32 mMol) Triäthylamin in 3 ml Methylenchlorid wird 24 Stunden bei Raumtemperatur unter Argon gerührt. Anschliessend wird die Reaktionsmischung eingedampft, und der Rückstand zur Reinigung an 30 g Kieselgel unter Verwendung eines 14:1:0.1-Gemisches von Methylenchlorid, Methanol und Ammoniak als Eluierungsmittel chromatographiert. Es werden 51 mg Aethyl 4-[(S)-2-[(S)-α-[(tert-butylsulfonyl)methyl]hydrocinnamamido]-2-[[(1S,2R,3S)-1-(cyclohexylmethyl)-3-cyclopropyl-2,3-dihydroxypropyl]carbamoyl]äthyl]-α-methylenimidazol-1-propionat als amorpher Festkörper erhalten; MS : 743 $(M+H)^+$.

## Beispiel 13

In analoger Weise wie in Beispiel 12 beschrieben, wurden folgende Verbindungen hergestellt:

- Aus (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid und 1-(Acetyloxy)äthyl-4-nitrophenylkohlensäureester (J.Med.Chem. 1988, 31,318) das Aethylidenacetat 4-[(S)-2-[(S)-α-[(tert-butylsulfonyl)methyl]-hydrocinnamamido]-2-[[(1S,2R,3S)-1-(cyclohexylmethyl)-3-cyclopropyl-2,3-dihydroxypropyl]carbamoyl]-äthyl]imidazol-1-carboxylat als amorpher Festkörper, MS : 761 $(M+H)^+$;
- aus (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid und (1-Chloräthyl)äthylcarbonat das Aethyl 4-[(S)-2-[(S)-α-[(tert-butylsulfonyl)methyl]hydrocinnamido]-2-[[(1S,2R,3S)-1-(cyclohexylmethyl)-3-cyclopropyl-2,3-dihydroxypropyl]carbamoyl]äthyl] imidazol-1-carboxylat, MS : 703 $(M+H)^+$, und das Aethyl 5-[(S)-2-[(S)-α-[-(tert-butylsulfonyl)methyl]hydrocinnamamido]-2-[[(1S,2R,3S)-1-(cyclohexylmethyl)-3-cyclopropyl-2,3-dihydroxypropyl]carbamoyl]äthyl]imidazol-1-carboxylat, MS : 703 $(M+H)^+$, jeweils als farbloses Oel;
- aus (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid und Chlorameisensäureallylester das Allyl 4-[(S)-2-[(S)-α-[(tert-butylsulfonyl)methyl]hydrocinnamamido]-2-[[(1S,2R, 3S)-1-(cyclohexylmethyl)-3-cyclopropyl-2,3-dihydroxypropyl]carbamoyl]äthyl]imidazol-1-carboxylat als amorpher Festkörper, MS : 715 $(M+H)^+$, - aus (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid und Triphenylchlormethan das (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]-1-tritylimidazol-4-propionamid als amorpher Festkörper, MS : 873 $(M+H)^+$.

## Beispiel 14

Eine Suspension von 250 mg (0,4 mMol) (S)-α-[(S)-α-[(tert-Butylsulfonyl)methyl]hydrocinnamamido]-N-[-(1S,2R,3S)-1-(cyclohexylmethyl)-3-cyclopropyl-2,3-dihydroxypropyl]imidazol-4-propionamid, 60 mg 18-Crown-6, 250 mg Kaliumcarbonat und 2 ml Pivalinsäurechlormethylester wird über Nacht bei Raumtemperatur gerührt. Danach wird das Reaktionsgemisch in Essigester aufgenommen, und die Essigesterlösung mit ge sättigter Ammoniumchlorid-Lösung gewaschen. Nach dem Trocknen über Natriumsulfat wird unter vermindertem Druck eingedampft, und anschliessend das erhaltene Rohprodukt (1,3 g) zur Reinigung an 120 g Kieselgel unter Verwendung eines 200:10:1-Gemisches von Methylenchlorid, Methanol und Ammoniak als

Eluierungsmittel chromatographiert. Man erhält 145 mg [4-[(S)-2-[(S)-α-[(tert-Butylsulfonyl)methyl]-hydrocinnamamido]-2-[[(1S,2R,3S)-1-(cyclohexylmethyl)-3-cyclopropyl-2,3-dihydroxypropyl]carbamoyl]-äthyl]imidazol-1-yl]methyl-pivalat als farblosen Fstkörper; MS : 745 $(M+H)^+$.

Beispiel 15

Zu einem Gemisch von 630 mg (1 mMol) (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid und 20 ml (4 mMol) 0,2N Natronlauge in 20 ml Methanol werden bei 0° unter Rühren gleichzeitig 10,4 ml (2,08 mMol) einer 0,2N methanolischen Jodlösung und 2,4 ml (0,48 mMol) 0,2N Natronlauge getropft. Die farblose Reaktionsmischung wird 5 Minuten bei 0° und danach 2,5 Stunden bei Raumtemperatur nachgerührt. Anschliessend wird mit 25 ml Wasser verdünnt und zweimal mit je 80 ml Essigester extrahiert. Die vereinigten organischen Phasen werden mit gesättigter Natriumchlorid-Lösung gewaschen, über Natriumsulfat getrocknet und unter vermindertem Druck eingedampft. Der Rückstand (750 mg) wird zur Reinigung unter Verwendung eines 15:1-Gemisches von Methylenchlorid und Methanol als Eluierungsmittel chromatographiert. Es werden 504 mg (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]-5-jodimidazol-4-propionamid, MS : 757 $(M+H)^+$, und 38 mg (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]-2,5-jodimidazol-4-propionamid, MS : 883 $(M+H)^+$, jeweils als farbloser Festkörper erhalten.

Beispiel 16

Ein Gemisch von 355 mg (0.58 mMol) (S)-α-Amino-N-[(1S,2R,3S)-1-(cyclohexylmethyl)-3-cyclopropyl-2,3-dihydroxypropyl]-1-tritylimidazol-4-propionamid, 95 mg (0.58 mMol) Benzimidazol-2-carbonsäure [Chem.Ber. 45 , 3489 (1912)], 256 mg (0.58 mMol) BOP und 0.12 ml (0.58 mMol) Huenigbase in 50 ml Acetonitril wird 20 Stunden bei Raumtemperatur gerührt. Danach wird das Reaktionsgemisch unter vermindertem Druck eingedampft, der Rückstand in 60 ml Essigester aufgenommen und zweimal mit 20 ml gesättigter Kaliumhydrogencarbonat-Lösung gewaschen. Nach dem Trocknen der organischen Phase über Natriumsulfat wird das Lösungsmittel unter vermindertem Druck abgedampft. Der Rückstand wird zur Reinigung an 30 g Kieselgel unter Verwendung eines 15:1-Gemisches von Methylenchlorid und Methanol als Eluierungsmittel chromatographiert. Man erhält 379 mg N-[(S)-1-[[(1S,2R,3S)-1-(Cyclohexylmethyl)-3-cyclopropyl-2,3-dihydroxypropyl]carbamoyl]-2-(1-tritylimidazol-4-yl)äthyl]-2-benzimidazolcarboxamid als farblosen Schaum; MS : 751 $(M+H)^+$.

Das als Ausgangsstoff eingesetzte (S)-α-Amino-N-[(1S,2R,3S)-1-(cyclohexylmethyl)-3-cyclopropyl-2,3-dihydroxypropyl]-1-tritylimidazol-4-propionamid wurde wie folgt hergestellt:

(a) tert-Butyl (4S,5R)-4-(cyclohexylmethyl)-5-[(S)-cyclopropylhydroxymethyl]-2,2-dimethyl-3-oxazolidincarboxylat und tert-Butyl (4S,5R)-4-(cyclohexlmethyl)-5-[(R)-cyclopropylhydroxymethyl]-2,2-dimethyl-3-oxazolidincarboxylat:

Zu einer Lösung der aus 3.94 ml (49 mMol) Cyclopropylbromid und 1.2 g (0.049 Grammatom) Magnesiumspänen in 22 ml Tetrahydrofuran hergestellten Grignardverbindung wird bei ca. 15° eine Lösung von 3.21 g (9,8 mMol) tert-Butyl (4S,5R)-4-(cyclohexylmethyl)-5-formyl-2,2-dimethyl-3-oxazolidincarboxylat (WO 87/05302) in 25 ml Tetrahydrofuran getropft, und das Reaktionsgemisch anschliessend während 16 Stunden bei Raumtemperatur unter Argon gerührt. Danach giesst man das Reaktionsgemisch auf 40 ml einer eiskalten, gesättigten Ammoniumchloridlösung und extrahiert zweimal mit je 50 ml Essigester. Die Essigesterextrakte werden mit 40 ml eiskalter, gesättigter Ammoniumchloridlösung gewaschen, vereinigt, über Natriumsulfat getrocknet und unter vermindertem Druck eingedampft. Zur Reinigung wird der Rückstand (4.33 g) über eine mit Toluol und 1% Triäthylamin aufgezogene Säule von 110 g Kieselgel mit einem 95:5-Gemisch von Toluol und Essigester als Eluierungsmittel chromatographiert. Man erhält 1.9 g tert-Butyl (4S,5R)-4-(cyclohexylmethyl)-5-[(S)-cyclopropylhydroxymethyl]-2,2-dimethyl-3-oxazolidincarboxylat, MS: 368 $(M+H)^+$, und 0.5 g tert-Butyl (4S,5R)-4-(cyclohexylmethyl)-5-[(R)-

cyclopropylhydroxymethyl]-2,2-dimethyl-3-oxazolidincarboxylat, MS : 368 $(M+H)^+$, jeweils als farbloses Oel.

(b) (1S,2R,3S)-3-Amino-4-cyclohexyl-1-cyclopropyl-1,2-butandiol:

1.42 g (3.86 mMol) tert-Butyl (4S,5R)-4-(cyclohexylmethyl)-5-[(S)-cyclopropylhydroxymethyl]-2,2-dimethyl-3-oxazolidincarboxylat, gelöst in 15 ml Methanol und 10 ml Wasser, werden mit 4 ml 7.5 N Salzsäure versetzt und 3 Stunden bei 50° gerührt. Die Reaktionslösung wird im Eisbad auf 3° abgekühlt, mit 4 ml 7.5 H Natronlauge tropfenweise versetzt und 1 Stunde nachgerührt. Die erhaltene Suspension wird unter vermindertem Druck eingedampft, zweimal mit 10 ml Toluol azeotrop entwässert, und der Rückstand dreimal mit 10 ml eines 95:5-Gemisches von Methylenchlorid und Methanol ausgerührt. Der unlösliche Rückstand wird abfiltriert, und das Filtrat unter vermindertem Druck eingedampft. Das erhaltene Rohprodukt (1.14 g) wird in 15 ml Aether suspendiert und danach abfiltriert. Man erhält 0.58 g (1S,2R,3S)-3-Amino-4-cyclohexyl-1-cyclopropyl-1,2-butandiol als farblose Kristalle, Smp. 141-142°.

(c) (S)-N [(1S,2R,3S)-1-(cyclohexylmethyl)-3-cyclopropyl-2,3-dihydroxypropyl]-1-trityl-α-(tritylamino)imidazol-4-propionamid:

Ein Gemisch von 0.65 g (2.86 mMol) (1S,2R,3S)-3-Amino-4-cyclohexyl-1-cyclopropyl-1,2-butandiol, 1.27 g (2.86 mMol) BOP, 1.85 g (2.86mMol) Ditrityl-L-Histidin (Tetrahedron Lett. 1987, 6031) und 0.59 ml (2.86 mMol) Hünigbase in 25 ml Acetonitril wird 6 Stunden bei Raumtemperatur gerührt. Anschliessend wird das Reaktionsgemisch unter vermindertem Druck eingedampft, und der erhaltene Rückstand in 150 ml Essigester aufgenommen, zweimal mit 50 ml gesättigter Kaliumhydrogencarbonat-Lösung gewaschen, und die organische Phase über Natriumsulfat getrocknet. Nach dem Abdampfen des Lösungsmittels wird das Rohprodukt zur Reinigung an 120 g Kieselgel unter Verwendung eines 40:1-Gemisches von Methylenchlorid und Methanol als Eluierungsmittel chromatographiert. Es werden 1.52 g (S)-N-[(1S,2R,3S)-1-(cyclohexylmethyl)-3-cyclopropyl-2,3-dihydroxypropyl]-1-trityl-α-(tritylamino)imidazol-4-propionamid als farbloser Schaum erhalten; MS : 849 $(M+H)^+$.

(d) (S)-α-Amino-N-[(1S,2R,3S)-1-(cyclohexylmethyl)-3-cyclopropyl-2,3-dihydroxypropyl]-1-tritylimidazol-4-propionamid:

Eine Lösung von 1.52 g (1.79 mMol) (S)-N-[(1S,2R,3S)-1-(cyclohexylmethyl)-3-cyclopropyl-2,3-dihydroxypropyl]-1-trityl-α-(tritylamino)imidazol-4-propionamid in einem Gemisch von 95.5 ml Methylenchlorid, 3 ml Methanol und 1,5 ml Trifluoressigsäure wird 5 Minuten bei Raumtemperatur gerührt. Anschliessend werden 150 ml 2N Kaliumbicarbonat-Lösung und 150 ml Methylenchlorid zugegeben. Die organische Phase wird abgetrennt, über Natriumsulfat getrocknet und eingedampft. Der Rückstand wird zur Reinigung an 100 g Kieselgel unter Verwendung eines 150:10:0.1-Gemisches von Methylenchlorid, Methanol und Ammoniak als Eluierungsmittel chromatographiert. Es werden 1.03 g (S)-α-Amino-N-[(1S,2R,3S)-1-(cyclohexylmethyl)-3-cyclopropyl-2,3-dihydroxypropyl]-1-tritylimidazol-4-propionamid als farbloser Schaum erhalten; MS : 607 $(M+H)^+$.

## Beispiel 17

In analoger Weise wie in Beispiel 16 beschrieben, wurden die folgenden Verbindungen hergestellt:
- Aus (S)-α-Amino-N-[(1S,2R,3S)-1-(cyclohexylmethyl)-3-cyclopropyl-2,3-dihydroxypropyl]-1-tritylimidazol-4-propionamid und Indol-2-carbonsäure das N-[(S)-1-[[(1S,2R,3S)-1-(Cyclohexylmethyl)-3-cyclopropyl-2,3-dihydroxypropyl]carbamoyl]-2-(1-tritylimidazol)-4-yläthyl]indol-2-carboxamid;
- aus (S)-α-Amino-N-[(1S,2R,3S)-1-(cyclohexylmethyl)-3-cyclopropyl-2,3-dihydroxypropyl]-1-tritylimidazol-4-propionamid und 5-Hydroxyindol-2-carbonsäure das N-[(S)-1-[[(1S,2R,3S)-1-(Cyclohexylmethyl)-3-cyclopropyl-2,3-dihydroxypropyl]carbamoyl]-2-(1-tritylimidazol)-4-yläthyl]-5-hydroxyindol-2-carboxamid;
- aus (S)-α-Amino-N-[(1S,2R,3S)-1-(cyclohexylmethyl)-3-cyclopropyl-2,3-dihydroxypropyl]-1-tritylimidazol-4-propionamid und Benzothiazol-2-carbonsäure (Chem.Ber. 37, 3731) das (S)-α-Benzothiazolcarboxamido-N-[(1S,2R,3S)-1-(cyclohexylmethyl)-3-cyclopropyl-2,3-dihydroxypropyl]-1-tritylimidazol-4-propionamid;

aus (S)-α-Amino-N-[(1S,2R,3S-1-cyclohexylmethyl)-3-cyclopropyl-2,3-dihydroxypropyl]-1-tritylimidazol-4-propionamid und Pyrrol-2-carbonsäure das (S)-N-[(1S,2R,3S)-1-Cyclohexylmethyl)-3-cyclopropyl-2,3-dihydroxypropyl]-α-pyrrol-2-carboxamido-1-tritylimidazol-4-propionamid;

- aus (S)-α-Amino-N-[(1S,2R,3S-1-cyclohexylmethyl)-3-cyclopropyl-2,3-dihydroxypropyl]-1-tritylimidazol-4-propionamid und α-Benzyl-phenylpropionsäure (Ann.Chem. 691, 61) das (S)-N-[(1S,2R,3S)-1-(Cyclohexylmethyl)-3-cyclopropyl-2,3-dihydroxypropyl]-α-(2,2-dibenzylacetamido)-1-tritylimidazol-4-propionamid, $R_f = 0.25$ (Methylenchlorid/Methanol 20:1);

- aus (S)-α-Amino-N-[(1S,2R,3S)-1-(cyclohexylmethyl)-3-cyclopropyl-2,3-dihydroxypropyl]-1-tritylimidazol-4-propionamid und (RS)-3,4-Dihydro-2,3-(1H)-isochinolindicarbonsäure-2-äthylester (EPA 0189203) das (RS)-3-(RS)-1-[[(1S,2R,3S)-1-(Cyclohexylmethyl)-3-cyclopropyl-2,3-dihydroxypropyl]carbamoyl]-2-(1-tritylimidazol)-4-yläthyl]-3,4-dihydro-2(1H)-isochinolincarbonsäureäthylester, $R_f = 0.65$ und 0.6 (-(Methylenchlorid/Methanol/Ammmoniak 150:10:0.1).

## Beispiel 18

In analoger Weise wie in Beispiel 16 beschrieben, wurden die folgenden Verbindungen hergestellt:

- Aus (R oder S)-α-Amino-N-[(1S,2R,3S)-1-(cyclohexylmethyl)-3-cyclopropyl-2,3-dihydroxypropyl]-4-thiazolpropionamid und Indol-2-carbonsäure das N-[(R oder S)-1-[[(1S,2R,3S)-1-(cyclohexylmethyl)-3-cyclopropyl-2,3-dihydroxypropyl]carbamoyl]-2-(4-thiazolyl)äthyl]indol-2-carboxamid als farbloser Festkörper, MS : 525 $(M+H)^+$;
- aus (R oder S)-α-Amino-N-[(1S,2R,3S)-1-(cyclohexylmethyl)-3-cyclopropyl-2,3-dihydroxypropyl]-4-thiazolpropionamid und Benzimidazol-2-carbonsäure das N-[(R oder S)-1-[[(1S,2R,3S)-1-(cyclohexylmethyl)-3-cyclopropyl-2,3-dihydroxypropyl]carbamoyl]-2-(4-thiazolyl)äthyl]-2-benzimidazolcarboxamid als farbloser Festkörper, MS : 526 $(M+H)^+$.

Das als Ausgangsstoff eingesetzte (R)- und (S)-α-Amino-N-[(1S,2R,3S)-1-(cyclohexylmethyl)-3-cyclopropyl-2,3-dihydroxypropyl]-4-thiazolpropionamid wurde wie folgt hergestellt:

(a) tert-Butyl [(RS)-1-[[(1S,2R,3S)-1-(cyclohexylmethyl)-3-cyclopropyl-2,3-dihydroxypropyl]carbamoyl]-2-(4-thiazolyl)äthyl]carbamat (1:1 Epimerengemisch):

In Analogie zu dem in Beispiel 16(c) beschriebenen Verfahren, wird durch Kondensation von (RS)-Boc-3-(4-Thiazolyl)alanin, hergestellt nach der in EPA 0274259 beschriebenen Synthese, und 3-Amino4-cyclohexyl-1-cyclopropyl-1,2-butandiol das tert-Butyl [(RS)-1-[[(1S,2R,3S)-1-(cyclohexylmethyl)-3-cyclopropyl-2,3-dihydroxypropyl]carbamoyl]-2-(4-thiazolyl)äthyl]carbamat (1:1 Epimerengemisch) als farbloser Festkörper erhalten; MS : 482 $(M+H)^+$.

(b) (R)- und (S)-α-Amino-N-[(1S,2R,3S)-1-cyclohexylmethyl)-3-cyclopropyl-2,3-dihydroxypropyl]-4-thiazolpropionamid:

Ein Gemisch vcn 900 mg (1.87 mMol) tert-Butyl [(RS)-1-[[(1S,2R,3S)-1-(cyclohexylmethyl)-3-cyclopropyl-2,3-dihydroxypropyl]carbamoyl]-2-(4-thiazolyl)äthyl]carbamat (1:1 Epimerengemisch) und 9.3 ml 2N Salzsäure in 30 ml Methanol wird 3 Stunden zum Rückfluss erhitzt. Nach üblicher Aufarbeitung wird das erhaltene Produkt zur Trennung der Epimeren an 90 g Kieselgel unter Verwendung eines 150:10: 1 -Gemisches von Methylenchlorid, Methanol und Ammoniak als Eluierungsmittel chromatographiert. Man erhält 410 mg des weniger polaren Epimeren, $R_f = 0,2$ (Methylenchlorid/Methanol/Ammoniak 150:10:1). MS : 382 $(M+H)^+$, und 293 mg des polareren Epimeren, Rf = 0.15 (Methylenchlorid/Methanol/Ammoniak 150:10:1), MS : 382 $(M+H)^+$.

## Beispiel 19

Ein Gemisch von 200 mg (0,55 mMol) (S)-α-Amino-N-[(1S,2R,3S)-1-(cyclohexylmethyl)-3-cyclopropyl-2,3-dihydroxypropyl]imidazol-4-propionamid, 174 mg (0,57 mMol) (RS)-α-(Sulfomethyl)hydrozimtsäure-guanidinsalz, 95 mg (0,7 mMol) HOBT, 62 mg (0,61 mMol) Triäthylamin und 231 mg (0,61 mMol) HBTU in 50

ml Dimethylformamid wird über Nacht bei Raumtemperatur gerührt. Danach wird das Reaktionsgemisch im Hochvakuum zur Trockene eingedampft. und das erhaltene Rohprodukt zur Reinigung an 50 g Kieselgel mit einem 9:1- bis 1:1-Gemisch von Wasser und Methanol als Eluierungsmittel chromatographiert. Zur Trennung der beiden erhaltenen Epimeren wird diese Chromatographie nochmals durchgeführt. Nach dem Lyophilisieren aus Dioxan/Wasser erhält man 81 mg des weniger polaren (R oder S)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3-phenylpropansulfonsäure-guanidinsalzes und 40 mg des polareren (S oder R)-2-[[(S)-1-[[(1S,2R,3S)-1-(Cyclohexylmethyl)-3-cyclopropyl2,3-dihydroxypropyl]carbamoyl]-2-imidazol-4-yläthyl]carbamoyl]-3-phenylpropansulfonsäure-guanidinsalze, jeweils als farbloses Pulver.

Das als Ausgangsstoff eingesetzte (S)-α-Amino-N-[(1S,2R,3S)-1-(cyclohexylmethyl)-3-cyclopropyl-2,3-dihydroxypropyl]imidazol-4-propionamid wurde wie folgt hergestellt:
Ein Gemisch von 343 mg (1.51 mMol) (1S,2R,3S)-3-Amino-4-cyclohexyl-1-cyclopropyl-1,2-butandiol, 995 mg (1,66 mMol) $(Fmoc)_2$His-OH, 0,21 ml (1,61 mMol) 4-Aethylmorpholin, 449 mg (3,22 mMol) HOBT und 347 mg (1,81 mMol) EDC in 20 ml Dimethylformamid wird über Nacht bei Raumtemperatur stehen gelassen. Danach wird das Reaktionsgemisch im Hochvakuum eingedampft, der Rückstand auf ein Gemisch von Eis und 90 ml Natriumbicarbonat-Lösung gegossen und dreimal mit je 150 ml Essigester extrahiert. Die drei Essigesterextrakte werden nacheinander mit 70 ml gesättigter Ammoniumchlorid-Lösung, 70 ml 2N Natriumbicarbonat-Lösung und 70 ml gesättigter Natriumchlorid-Lösung gewaschen, vereinigt, über Magnesiumsulfat getrocknet, filtriert und eingedampft. Das erhaltene Rohprodukt wird in 60 ml Methylenchlorid und 2 ml Piperidin 3 Stunden bei Raumtemperatur gerührt. Dann wird das Reaktionsgemisch eingedampft und der Rückstand mit 50 ml Hexan angerieben und abfiltriert. Das Filtrat wird mit einem 65:10:1-Gemisch von Methylenchlorid, Methanol und Ammoniak als Eluierungsmittel an 70 g Kieselgel chromatographiert, wobei man 390 mg (S)-α-Amino-N-[(1S,2R,3S)-1-(cyclohexylmethyl)-3-cyclopropyl-2,3-dihydroxypropyl]imidazol-4-propionamid als farblosen Schaum erhält; MS : 365 $(M+H)^+$.

Das als Ausgangsstoff eingesetzte (RS)-α-(Sulfomethyl)hydrozimtsäure-guanidinsalz wurde wie folgt hergestellt:

(a) <u>(RS)-α-(Sulfomethyl)hydrozimtsäureäthylester-guanidinsalz</u>

Zu einer Lösung von 4,73 g (16,2 mMol) rohem 2-Benzyl-3-chlorsulfonyl-propionsäureäthylester, hergestellt nach dem in EPA 0236734 beschriebenen Verfahren, in 150 ml Methylenchlorid und 40 ml Dimethylformamid, werden bei -10° 5,06 g (28 mMol) Guanidincarbonat gegeben. Das Reaktionsgemisch wird bei Raumtemperatur über Nacht gerührt und danach eingedampft. Der Rückstand wird zwischen Methylenchlorid und Wasser verteilt, die Wasserphase abgetrennt, unter vermindertem Druck eingedampft, und der erhaltene Rückstand an 50 g Kieselgel unter Verwendung eines 9:1- bis 1:1-Gemisches von Wasser und Methanol als Eluierungsmittel chromatographiert. Das erhaltene Produkt wird aus einem Gemisch von Methanol und Aether umkristallisiert. Es werden 3,29 g (RS)-α-(Sulfomethyl)-hydrozimtsäureäthylester-guanidinsalz als farblose Kristalle erhalten.

| Analyse für $C_{12}H_{16}O_5S \cdot CH_5N_3$: | | | | |
|---|---|---|---|---|
| Berechnet: | C 47,12; | H 6,39; | N 12,68; | S 9,67% |
| Gefunden : | C 47,03; | H 6,39; | N 12,68; | S 9,51%. |

(b) <u>(RS)-α-(Sulfomethyl)hydrozimtsäure-guanidinsalz:</u>

Eine Lösung von 3,11 g (9,4 mMol) (RS)-α-(Sulfomethyl)hydrozimtsäureäthylester-guanidinsalz in 400 ml halbkonzentrierter Salzsäure wird 20 Stunden zum Rückfluss erhitzt. Anschliessend wird die Reaktionslösung unter vermindertem Druck eingedampft, und der erhaltene Rückstand zur Reinigung an 50 g Kieselgel unter Verwendung eines 9:1- bis 1:1-Gemisches von Wasser und Methanol als Eluierungsmittel chromatographiert. Nach dem Lyophilisieren aus Wasser werden 0,6 g (RS)-α-(Sulfomethyl)hydrozimtsäure-guanidinsalz als farbloses Pulver erhalten, MS : 243 $(M-H)^-$.

Beispiel 20

In analoger Weise wie in Beispiel 19 beschrieben, wurden folgende Verbindungen hergestellt:
- Aus (S)-α-Amino-N-[(1S,2R,3S)-1-(cyclohexylmethyl)-3-cyclopropyl-2,3-dihydroxypropyl]imidazol-4-propionamid und N-Boc-(2S,3R)-3-amino-2-hydroxy-4-phenylbuttersäure das tert-Butyl [(1R,2S)-1-benzyl-2-[[(S)-1-[[(1S,2R,3S)-1-(cyclohexylmethyl)-3-cyclopropyl-2,3-dihydroxypropyl]carbamoyl]-2-imidazol-4-yläthyl]-carbamoyl]-2-hydroxyäthyl]carbamat als amorpher Festkörper, MS : 642 $(M+H)^+$;
- aus (S)-α-Amino-N-[(1S,2R,3S-1-cyclohexylmethyl)-3-cyclopropyl-2,3-dihydroxypropyl]imidazol-4-propionamid und 2-(S)-[(4-Morpholinylcarbonyl)oxy]-3-phenylpropionsäure das (S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]phenäthyl 4-morpholincarboxylat als amorpher Festkörper, MS : 626 $(M+H)^+$;
- aus (S)-α-Amino-N-[(1S,2R,3S-1-cyclohexylmethyl)-3-cyclopropyl-2,3-dihydroxypropyl]imidazol-4-propionamid und 2-(S)-[(1-Piperidinylcarbonyl)oxy]-3-phenylpropionsäure das (S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]phenäthyl 1-piperidincarboxylat als amorpher Festkörper, MS : 625 $(M+H)^+$.

Die als Ausgangsstoffe eingesetzten Phenylpropionsäuren wurden wie folgt hergestellt:

2-(S)-[(4-Morpholinylcarbonyl)oxy]-3-phenylpropionsäure wurde, ausgehend von L-Phenylmilchsäuremethylester, nach dem in J.Med.Chem., 31 , (1988), 2277 beschriebenen Verfahren erhalten.

2-(S)-[(1-piperidinylcarbonyl)oxy]-3-phenylpropionsäure wurde ebenfalls ausgehend von L-Phenylmilchsäuremethylester in Analogie zu dem in J.Med.Chem., 31 , (1988) 2277 beschriebenen Verfahren erhalten, indem anstelle von Morpholin Piperidin eingesetzt wurde.

Beispiel 21

In analoger Weise wie in Beispiel 19 beschrieben, wurde durch Kondensation von (S)-α-Amino-N-[(1S,2R,3S)-1-(cyclohexylmethyl)-3-cyclopropyl-2,3-dihydroxypropyl]imidazol-4-propionamid mit Fmoc-Phe-OH das 9H-Fluoren-9-ylmethyl-[(S)-α-[[(S)-1-[[(1S,2R,3S)-1(cyclohexylmethyl)-3-cyclopropyl-2,3-dihydroxypropyl]carbamoyl]-2-imidazol-4-yläthyl]carbamoyl]phenäthyl]carbamat erhalten, das durch Umsetzung mit Piperidin in Methylenchlorid in das (S)-N-[(1S,2R,3S)-1-(Cyclohexylmethyl)-3-cyclopropyl-2,3-dihydroxypropyl]-α-[(3-phenyl-L-alanyl)amino]imidazol-4-propionamid übergeführt wurde. Die weitere Kondensation mit Boc-D-Pro-OH, analog Beispiel 19, lieferte den (R)-2-[[(S)-α-[[(S)-1-[[(1S,2R,3S-1-Cyclohexylmethyl)-3-cyclopropyl-2,3-dihydroxypropyl]carbamoyl]-2-imidazol-4-yläthyl]carbamoyl]phenäthyl]-carbamoyl]-1-pyrrolidincarbonsäure-tert-butylester als amorphen Festkörper, MS : 709 $(M+H)^+$.

Beispiel 22

In analoger Weise wie in Beispiel 21 beschrieben, wurden die folgenden Verbindungen hergestellt:
- Aus (S)-N-[(1S,2R,3S)-1-(Cyclohexylmethyl)-3-cyclopropyl-2,3-dihydroxypropyl]-α-[(3-phenyl-L-alanyl)-amino]imidazol-4-propionamid und 1-tert-Butyl 4-hydrogen N-(tert-butoxycarbonyl)-N-[2-(1-tert-butoxyformamido)äthyl]-L-aspartat das Di-tert-butyl N-[(S)-1-(tert-butoxycarbonyl)-2-[[(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]-phenäthyl]carbamoyl]äthyl]äthylendicarbamat als amorpher Festkörper, MS: 926 $(M+H)^+$;
- aus (S)-N-[(1S,2R,3S)-1-(Cyclohexylmethyl)-3-cyclopropyl-2,3-dihydroxypropyl]-α-[(3-phenyl-L-alanyl)-amino]imidazol-4-propionamid und L-Pyroglutaminsäure das (S)-N-[(1S,2R,3S)-1-(Cyclohexylmethyl)-3-cyclopropyl-2,3-dihydroxypropyl]-α-[(S)-α-[(S)-5-oxo-2-pyrrolidincarboxamido]hydrocinnamamido]imidazol-4-propionamid als amorpher Festkörper, MS : 623 $(M+H)^+$;
- aus (S)-N-[(1S,2R,3S)-1-(Cyclohexylmethyl)-3-cyclopropyl-2,3-dihydroxypropyl]-α-[(3-phenyl-L-alanyl)-amino]imidazol-4-propionamid und Boc-Aminoisobuttersäure das tert-Butyl [1-[[(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]-phenäthyl]carbamoyl]-1-methyläthyl]carbamat als amorpher Festkörper, MS : 697 $(M+H)^+$;
- aus (S)-N[(1S,2R,3S)-1-Cyclohexylmethyl)-3-cyclopropyl-2,3-dihydroxypropyl]-α-[(3-phenyl-L-alanyl)-amino]imidazol-4-propionamid und N-(tert-Butoxycarbonyl)-N-[2-[(tert-butuxycarbonyl)amino]äthyl]glycin (US 4145337) das Di-tert-butyl N-[[[(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]phenäthyl]carbamoyl]methyl]äthylendicarbamat als amorpher Festkörper, MS : 813 $(M+H)^+$.

Das als Ausgangsstoff eingesetzte 1-tert-Butyl 4-hydrogen N-(tert-butoxycarbonyl)-N-[2-(1-tert-butoxyformamido)äthyl]-L-aspartat wurde wie folgt hergestellt:

(a) 4-Benzyl 1-tert-butyl N-[(1-tert-butoxymamido)acetyl]-L-aspartat:

Ein Gemisch von 12 g (54 mMol) L-Asparaginsäure-4-benzylester, 10 ml konzentrierter Schwefelsäure und 100 ml flüssigem Isobutylen in 100 ml trockenem Dioxan wird in einer Druckflasche über Nacht bei Raumtemperalur gerührt. Danach wird die Lösung in 500 ml eiskalte 1N Natriumhydroxid-Lösung getropft. Die wässrige Phase wird dreimal mit Aether extrahiert. Die vereinigten organischen Phasen werden über Natriumsulfat getrocknet und eingedampft. Das erhaltene dickflüssige Oel (13,85 g) wird in 250 ml trockenem Dimethylformamid gelöst und mit 9,55 g (54,5 mMol) Boc-Glycin, 20,7 g (54,5 mMol) HBTU und 6 ml (54,5 mMol) N-Methylmorpholin während 4 Stunden bei Raumtemperatur gerührt. Anschliessend wird das Reaktionsgemisch im Hochvakuum eingedampft, und der erhaltene Rückstand in Aether aufgenommen. Die organische Phase wird mit 5%iger Natriumcarbonat-Lösung und Wasser gewaschen, danach über Natriumsulfat getrocknet und unter vermindertem Druck eingedampft. Der ölige Rückstand wird an Kieselgel unter Verwendung eines 1:1-Gemisches von Hexan und Aether als Eluierungsmittel chromatographiert. Man erhält 4-Benzyl 1-tert-butyl N-[(1-tert-butoxyformamido)acetyl]-L-aspartat als ein dickflüssiges Oel; MS : 437 $(M+H)^+$.

(b) 4-Benzyl 1-tert-butyl N-[(1-tert-butoxyformamido)thioacetyl]-L-aspartat:

Eine Lösung von 14,8 g (33,9 mMol) 4-Benzyl 1-tert-butyl N-[(1-tert-butoxyformamido)acetyl]-L-aspartat und 6,9 g (17 mMol) Lawesson-Reagenz in 140 ml trockenem Toluol wird 2,5 Stunden auf 100° erhitzt. Danach wird das Lösungsmittel unter vermindertem Druck abdestilliert, und der Rückstand dreimal mit 500 ml eines 9:1-Gemisches von Hexan und Aether ausgekocht. Die vereinigten organischen Phasen werden eingedampft, und der Rückstand zur Reinigung an Kieselgel unter Verwendung eines 9:1-Gemisches von Hexan und Aether als Eluierungsmittel chromatographiert, wobei 4-Benzyl 1-tert-butyl N-[(1-tert-butoxyformamido)thioacetyl]-L-aspartat als dickflüssiges Oel erhalten wird; MS : 452 $(M)^+$.

(c) 4-Benzyl 1-tert-butyl (E/Z)-N-[2-(1-tert-butoxyformamido)-1-(methylthio)äthyliden]-L-aspartat:

Ein Gemisch von 1,1 g (2,43 mMol) 4-Benzyl 1-tert-butyl N[(1-tert-butoxyformamido)thioacetyl]-L-aspartat, 1,95 ml Methyljodid und 0,92 g Kaliumcarbonat in 50 ml Aceton wird über Nacht bei Raumtemperatur gerührt. Danach wird das Lösungsmittel unter vermindertem Druck abdestilliert, und der Rückstand in Aether aufgenommen. Die organische Phase wird mit Wasser gewaschen, über Natriumsulfat getrocknet und eingedampft. 4-Benzyl 1-tert-butyl N-[2-(1-tert-butoxyformamido-1-methylthio)äthyliden]-L-aspartat wird als ein Gemisch der E- und Z-Isomeren erhalten; MS : 467 $(M+H)^+$.

(d) 3-[(Benzyloxy)carbonyl]-N-[2-(1-tert-butoxyformamido)äthyl]-L-alanin-tert-butylester:

Ein Gemisch von 0,5 g (1,1 mMol) 4-Benzyl 1-tert-butyl (E/Z)-N-[2-(1-tert-butoxyformamido-1-methylthio)äthyliden]-L-aspartat, 1,0 g Natriumcyanborhydrid und 0,5 ml Essigsäure in 10 ml Methanol wird 2 Stunden bei Raumtemperatur gerührt. Danach wird das Lösungsmittel unter vermindertem Druck abdestilliert, und der Rückstand in Aether aufgenommen. Die Aetherlösung wird mit Wasser gewaschen, über Natriumsulfat getrocknet und anschliessend eingedampft. Das zurückbleibende Oel wird zur Reinigung an Kieselgel unter Verwendung eines 1:1-Gemisches von Cyclohexan und Essigester als Eluierungsmittel chromatographiert, wobei 3-[(Benzyloxy)carbonyl]-N-[2-(1-tert-butoxyformamido)äthyl]-L-alanin-tert-butylester als dickflüssiges Oel erhalten wird; MS : 423 $(M+H)^+$.

(e) 3-(Benzyloxy)carbonyl]-N-[(tert-butoxy)carbonyl]-N-[2-(1-tert-butoxyformamido)äthyl]-L-alanin-tert-butylester:

Ein Gemisch von 350 mg (0,83 mMol) 3-[(Benzyloxy)carbonyl]-N-[2-(1-tert-butoxyformamido)äthyl]-L-alanin-tert- butylester, 10 mg (0,08 mMol) Dimethylaminopyridin, 1 ml (7,2 mMol) Triäthylamin und 200 mg (0,9 mMol) Di-tert-butyldicarbonat in 5 ml Acetonitril wird 2 Stunden bei Raumtemperatur gerührt. Anschliessend wird das Lösungsmittel abdestilliert, und der Rückstand in Aether aufgenommen. Die Aetherlösung wird nacheinander mit gesättigter Natriumbicarbonat-Lösung, Wasser, 1M Weinsäure-Lösung und Wasser gewaschen, über Natriumsulfat getrocknet und eingedampft. Der Rückstand wird zur Reinigung an Kieselgel unter Verwendung eines 7:3-Gemisches von Cyclohexan und Essigester als Eluierungsmittel chromatographiert. 3-[(Benzyloxy)carbonyl]-N-[(tert-butoxy)carbonyl]-N-[2-(1-tert-butoxyformamido)äthyl]-L-alanin-tert-butylester wird als dickflüssiges Oel erhalten, MS : 523 $(M+H)^+$.

(f) 1-tert-Butyl 4-hydrogen N-(tert-butoxycarbonyl)-N-[2-(1-tert-butoxyformamido)äthyl]-L-aspartat:

Eine Suspension von 250 mg (0,48 mMol) 3-[(Benzyloxy)carbonyl]-N-[(tert-butoxy)carbonyl]-N-2-(1-tert-butoxyformamido)äthyl]-L-alanin-tert-butylester und 50 mg Palladium/Kohle (5%ig) in 10 ml Aethanol wird unter Normaldruck bei Raumtemperatur hydriert. Danach wird der Katalysator abfiltriert, und die Lösung unter vermindertem Druck eingedampft. 1-tert-Butyl 4-hydrogen N-(tert-butoxycarbonyl)-N-[2-(1-tert-butoxyformamido)äthyl]-L-aspartat wird als farbloser Schaum erhalten; MS : 433 $(M+H)^+$.

## Beispiel 23

Ein Gemisch von 147 mg (0.64 mMol) (1S oder R,2R,3S)-3-Amino-4-cyclohexyl-1-cyclopropyl-1-fluor-2-butanol, 365 mg (0.7 mMol) (S)-1-(tert-Butoxycarbonyl)-α-[(S)-α-[(tert-butylsulfonyl)methyl]-hydrocinnamamido]imidazol-4-propionsäure, 71 mg (0.7 mMol) Triäthylamin, 114 mg (0.7 mMol) HOOBT und 285 mg (0.7 mMol) HOBTU in 15 ml Dimethylformamid wird über Nacht bei Raumtemperatur unter Argon gerührt. Danach wird das Dimethylformamid am Hochvakuum abgedampft, und der Rückstand in 50 ml Essigester aufgenommen. Die Essigesterphase wird mit 20 ml gesättigter Natriumbicarbonat-Lösung und 20 ml gesättigter Natriumchlorid-Lösung gewaschen, über Natriumsulfat getrocknet und unter vermindertem Druck eingedampft. Man erhält das (S)-α-[(S)-α-[(tert-Butylsulfonyl)methyl]hydrocinnamamido]-N-[-(1S,2R,3S)-1-(cyclohexylmethyl)-3-cyclopropyl-3-fluor-2-hydroxypropyl]-1-Boc-imidazol-4-propionamid als gelblichen Schaum.

Das als Ausgangsstoff eingesetzte (1S oder R,2R,3S)-3-Amino-4-cyclohexyl-1-cyclopropyl-1-fluor-2-butanol wurde wie folgt hergestellt:

(a) tert-Butyl (4S,5R)-4-(cyclohexylmethyl)-5-[(S oder R)-cyclopropylfluormethyl]-2,2-dimethyl-3-oxazolidincarboxylat und tert-Butyl (4S,5R) 4-(cyclohexmethyl)-5-[(R oder S)-cyclopropylfluormethyl]-2,2-dimethyl-3-oxazolidincarboxylat:

Eine Lösung von 4.0 g (10.8 mMol) tert-Butyl (4S,5R)-4-(cyclohexylmethyl)-5-[(R)-cyclopropylhydroxymethyl]-2,2-dimethyl-3-oxazolidincarboxylat in 50 ml Methylenchlorid wird bei Raumtemperatur tropfenweise mit 2.6 g (16.3 mMol) Diäthylamino-Schwefeltrioxid versetzt. Die rötliche Reaktionslösung wird über Nacht bei Raumtemperatur gerührt und anschliessend mit 50 ml Wasser versetzt. Die organische Phase wird abgetrennt, über Natriumsulfat getrocknet und eingedampft. Das erhaltene Rohprodukt wird zur Reinigung und Auftrennung der resultierenden Epimeren unter Verwendung von Methylenchlorid als Eluierungsmittel an 300 g Kieselgel chromatographiert. Man erhält 1.90 g des weniger polaren Epimeren tert-Butyl (4S,5R)-4-(cyclohexylmethyl)-5-[(S oder R)-cyclopropylfluormethyl]-2,2-dimethyl3-oxazolidincarboxylat, MS : 354 $(M - CH_3)^+$, und 1.08 g des polareren Epimeren tert-Butyl (4S,5R)-4-(cyclohexylmethyl)-5-[(R oder S)-cyclopropylfluormethyl]-2,2-dimethyl-3-oxazolidincarboxylat, MS : 354 $(M - CH_3)^+$, jeweils als farbloses Oel.

(b) (1S oder R,2R,3S)-3-Amino-4-cyclohexyl-1-cyclopropyl-1-fluor-2-butanol:

Eine Lösung von 0.7 g (1.9 mMol) tert-Butyl (4S,5R)-4-(cyclohexylmethyl)-5-[(S oder R)-cyclopropylfluormethyl]-2,2-dimethyl-3-oxazolidincarboxylat in 3 ml Methylenchlorid wird bei Raumtemperatur mit 3 ml eines frisch hergestellten 1:1-Gemisches einer 1M Phenollösung und einer 1M Trimethylchlorsilanlösung, beide jeweils in Methylenchlorid, versetzt. Die Reaktionslösung wird 36 Stunden bei Raumtemperatur gerührt, anschliessend auf 30 ml eiskalte, gesättigte Natriumcarbonat-Lösung gegossen und zweimal mit jeweils 100 ml Essigester extrahiert. Die organische Phase wird danach über Natriumsulfat getrocknet und unter vermindertem Druck eingedampft. Das Rohprodukt (300 mg) wird zur Reinigung an 100 g Kieselgel unter Verwendung eines 9:1:0.1-Gemisches von Methylenchlorid, Isopropanol und Ammoniak als Eluierungsmittel chromatographiert. Es werden 230 mg (1S oder R,2R,3S)-3-Amino-4-cyclohexyl-1-cyclopropyl-1-fluor-2-butanol als farbloser Schaum erhalten; MS : 230 $(M+H)^+$.

Die als Ausgangsstoff eingesetzte (S)-1-(tert-Butoxycarbonyl)-α-[(S)-α-[(tert-butylsulfonyl)methyl]-hydrocinnamamido]imidazol-4-propionsäure wurde wie folgt hergestellt:

(c) (S)-α-[(S)-α-[(tert-Butylsulfonyl)methyl]hydrocinnamamido]imidazol-4-propionsäuremethylester:

Ein Gemisch von 5.7 (20 mMol) (S)-α-[(tert-Butylsulfonyl)methyl]hydrozimtsäure (EPA 0236734) 4.85 g (20 mMol) L-Histidinmethylester-dihydrochlorid, 10.3 ml (81.7 mMol) N-Aethylmorpholin und 2.98 g (20 mMol) HOBT in 85 ml Dimethylformamid wird unter Argon bei 0-2° portionsweise mit 4.23 g (22 mMol) EDC versetzt und anschliessend über Nacht bei Raumtemperatur gerührt. Danach wird das Lösungsmittel im Hochvakuum abgedampft, der Rückstand in 100 ml Essigester aufgenommen und der Reihe nach dreimal mit 10 ml 2N Natriumbicarbonat-Lösung, zweimal mit 10 ml gesättigter Natriumchlorid-Lösung, zweimal mit 10 ml gesättigter Ammoniumchlorid-Lösung und zweimal mit 5 ml gesättigter Natriumchlorid-Lösung gewaschen. Die Wasserphasen werden dreimal mit 20 ml Essigester extrahiert, und die gesammelten organischen Phasen über Natriumsulfat getrocknet und unter vermindertem Druck eingedampft. Das erhaltene Rohprodukt (9.4 g) wird zur Reinigung an 300 g Kieselgel unter Verwendung eines 95:5-Gemisches von Methylenchlorid und Methanol als Eluierungsmittel chromatographiert. Es werden 8.6 g (S)-α-[(S)-α-[(tert-Butylsulfonyl)methyl]hydrocinnamamido]imidazol-4-propionsäuremethylester als farbloser Schaum erhalten; MS : 436 $(M+H)^+$.

(d) (S)-1-(tert-Butoxycarbonyl)-α-[(S) α-[(tert-butylsulfonyl]methyl]hydrocinnamamido]imidazol-4-propionsäure:

Eine Lösung von 1.33 g (3.06 mMol) (S)-α-[(S)-α-[(tert-Butylsulfonyl)methyl]hydrocinnamamido]imidazol-4-propionsäuremethylester in 4 ml Dioxan wird unter Eiskühlung mit 3.98 ml 1N Natronlauge tropfenweise versetzt und 30 Minuten bei dieser Temperatur gerührt, wobei der Methylester quantitativ in die entsprechende Säure übergeführt wird.

Anschliessend wird das Reaktionsgemisch bei 0-5° tropfenweise mit einer Lösung von 0.8 g (3.67 mMol) Di-tert-butyl-dicarbonat in 4 ml Dioxan versetzt und nach Entfernen des Eisbades während 5 Stunden bei Raumtemperatur gerührt. Danach werden 3.98 ml 1N Salzsäure und 25 ml Wasser zugefügt und mit 55 ml Essigester extrahiert. Die Wasserphase wird mit 50 ml Essigester nachgewaschen, und die vereinigten organischen Phasen werden zweimal mit 25 ml Wasser gewa schen, über Natriumsulfat getrocknet und unter vermindertem Druck eingedampft. Es werden 1.98 g (S)-1-(tert-Butoxycarbonyl)-α-[(S)-α-[(tert-butylsulfonyl)methyl]hydrocinnamamido]imidazol-4-propionsäure als Schaum erhalten; MS : 522 $(M+H)^+$.

## Beispiel 24

In analoger Weise wie in Beispiel 23 beschrieben, wurden die folgenden Verbindungen hergestellt:

- Aus (S)-1-(tert-Butoxycarbonyl)-α-[(S)-α-[(tert-butylsulfonyl)methyl]hydrocinnamamido]imidazol-4-propionsäure und (1R oder S,2R,3S)-3-Amino-4-cyclohexyl-1-cyclopropyl-1-fluor-2-butanol das (S)-α-[(S)-α-[(tert-Butylsulfonyl)methyl]hydrocinnamamido]-N-[(1S,2R, 3R)-1-(cyclohexylmethyl)-3-cyclopropyl-3-fluor-2-hydroxypropyl]-1-Boc-imidazol-4-propionamid;
- aus (S)-1-(tert-Butoxycarbonyl)-α-[(S)-α-[(tert-butylsulfonyl)methyl]hydrocinnamamido]imidazol-4-propionsäure und (1S,2R,3s)-3-Amino-4-cyclohexyl-1-cyclopropyl-1,2-butandiol das (S)-α-[(S)-α-[(tert-Butylsulfonyl)-methyl]hydrocinnamamido]-N-[(1S,2R,3S)-1-(cyclohexylmethyl)-3-cyclopropyl-2,3-dihydroxypropyl]-1-Boc-imidazol-4-propionamid;

- aus (S)-1-(tert-Butoxycarbonyl)-α-[(S)-α-](tert-butylsulfonyl)methyl]hydrocinnamamido]imidazol-4-propionsäure und (1S,2R,3S)-3-Amino-1-azido-4-cyclohexyl-lcyclopropyl-2-butanol das (S)-N-[(1S,2R,3S)-3-Azido-1-(cyclohexylmethyl)-3-cyclopropyl-2-hydroxypropyl]-α-[(S)-α-[(tert-butylsulfonyl)methyl]hydrocinnamamido]-1-Boc-imidazol-4-propionamid;
- aus (s)-1-(tert-Butoxycarbonyl)-α-[(S)-α-[(tert-butylsulfonyl)methyl]hydrocinnamamido]imidazol-4-propionsäure und (2R/S,3S,4S)-4-Amino-5-cyclohexyl-2-cyclopropyl-2,3-pentandiol das (S)-α-[(S)-α-[(tert-Butylsulfonyl)methyl]hydrocinnamamido]-N-[(1S,2R,3R/S)-1-(cyclohexylmethyl)-3-cyclopropyl-2,3-dihydroxybutyl]-1-Boc-imidazol-4-propionamid.

Die als Ausgangsstoffe eingesetzten Amine wurden wie folgt hergestellt:

(IR oder S,2R,3S)-3-Amino-4-cyclohexyl-1-cyclopropyl-1-fluor-2-butanol

In analoger Weise wie in Beispiel 23(b) beschrieben, wurde aus tert-Butyl (4S,5R)-4-(cyclohexylmethyl)-5-[(R oder S)-cyclopropylfluormethyl]-2,2-dimethyl-3-oxazolidincarboxylat nach Abspaltung der Boc- und Isopropyliden-Schutzgruppe das (1R oder S,2R, 3S)-3-Amino-4-cyclohexyl-1-cyclopropyl-1-fluor-2-butanol als farbloses Oel erhalten, MS : 230 $(M+H)^+$.

(1S,2R,3S)-3-Amino-1-azido-4-cyclohexyl-1-cyclopropyl-2-butanol

(a) tert-Butyl (4S,5R)-4-(cyclohexylmethyl)-5-[(S)-cyclopropylazidomethyl]-2,2-dimethyl-3-oxzolidincarboxylat:

Ein Gemisch von 1,63 g (25 mMol) Natriumazid, 1,5 ml Wasser und 10 ml Benzol wird unter Eiskühlung (<10°) und Rühren tropfenweise mit 0,67 ml (12,5 mMol) konzentrierter Schwefelsäure versetzt. Danach wird auf 0° abgekühlt, die Benzolphase abdekantiert und über Magnesiumsulfat getrocknet. Die so erhaltene Stickstoffwasserstoffsäure-Lösung wird zu einem Gemisch von 368 mg (1 mMol) tert-Butyl (4S,5R)-4-(cyclohexylmethyl)-5-[(R)-cyclopropylhydroxymethyl]-2,2-dimethyl-3-oxazolidincarboxylat und 289 mg (1,1 mMol) Triphenylphosphin in 5 ml Benzol gegeben. Anschliessend werden 0,15 ml (1,1 mMol) Azodicarbonsäurediäthylester zugegeben, und das Reaktionsgemisch unter Argon bei Raumtemperatur über Nacht weitergerührt. Danach wird das Reaktionsgemisch eingedampft, und der Rückstand zur Reinigung an 50 g Kieselgel unter Verwendung eines 3:1-Gemisches von Toluol und Essigester als Eluierungsmittel chromatographiert. Es werden 210 mg tert-Butyl (4S,5R)-4-(cyclohexylmethyl)-5-[(S)-cyclopropyl-azidomethyl]-2,2-dimethyl-3-oxazolidincarboxylat, MS : 393 $(M+H)^+$, als allmählich kristallisierendes Oel sowie 130 mg Edukt erhalten.

(b) (1S,2R,3s)-3-Amino-1-azido-4-cyclohexyl-1-cyclopropyl-2-butanol:

Analog zu dem oben zur Herstellung des (2RS,3R,4S)-4-Amino-5-cyclohexyl-1,2,3-pentantriols beschriebenen Verfahren, wird durch Umsetzung von tert-Butyl (4S,5R)-4-(cyclohexylmethyl)-5-[(S)-cyclopropyl-azidomethyl]-2,2-dimethyl-3-oxazolidincarboxylat das (1S,2R,3S)-3-Amino-1-azido-4-cyclohexyl-1-cyclopropyl-2-butanol als Oel erhalten, MS : 253 $(M+H)^+$.

(2R/S,3S,4S)-4-Amino-5-cyclohexyl-2-cyclopropyl-2,3-Pentandiol

(c) tert-Butyl (4S,5R)-4-(cyclohexylmethyl)-5-(cyclopropylcarbonyl-2,2-dimethyl-3-oxazolidincarboxylat:

Ein Gemisch von 500 mg (1.36 mMol) tert-Butyl (4S,5R)-4-(cyclohexylmethyl)-5-[(R)-cyclopropylhydroxymethyl)-2,2-dimethyl-3-oxazolidincarboxylat und 750 mg (1.77 mMol) 1,1-Dihydro-1,1,1-triacetoxy-1,2-benziodoxol-3(1H)-on in 10 ml Methylenchlorid wird 3 Stunden bei Raumtemperatur gerührt. Danach werden 50 ml Aether und 20 ml 2N Natronlauge zugegeben und 1 Stunde bei Raumtemperatur gerührt. Die organische Phase wird abgetrennt, mit 20 ml 2N Natronlauge ge waschen, über Natriumsulfat getrocknet und eingedampft. Man erhält 400 mg tert-Butyl (4S,5R)-4-(cyclohexylmethyl)-5-(cyclopropylcarbonyl)-2,2-dimethyl-3-oxazolidincarboxylat als gelbes Oel;, MS : 366 $(M+H)^+$.

(d) tert-Butyl (4S,5R)-4-(cyclohexylmethyl)-5-[(R/S)-1-cyclopropyl-1-hydroxyäthyl-2,2-dimethyl-3-oxazolidincarboxylat:

Eine Lösung von 400 mg (1.09 mMol) tert-Butyl (4S,5R)-4-(cyclohexylmethyl)-5 (cyclopropylcarbonyl)-2,2-dimethyl-3-oxazolidincarboxylat in 10 ml Diäthyläther wird bei 0° mit 3.9 ml einer 1.36 M Lösung von Methylmagnesiumjodid in 5 ml Diäthyläther innerhalb von 5 Minuten versetzt. Anschliessend wird das Reaktionsgemisch 2.5 Stunden bei Raumtemperatur gerührt, dann auf 0° abgekühlt und mit 30 ml halbgesättigter Ammoniumchlorid-Lösung versetzt. Die wässrige Phase wird zweimal mit 20 ml Diäthyläther extrahiert, danach werden die vereinigten organischen Extrakte über Natriumsulfat getrocknet und eingedampft. Zur Reinigung wird der Rückstand an 30 g Kieselgel unter Verwendung eines 3:1-Gemisches von Petroläther und Aether als Eluierungsmittel chromatographiert. Man erhält 338 mg tert-Butyl (4S,5R)-4-(cyclohexylmethyl)-5-[(R/S)-1-cyclopropyl-1-hydroxyäthyl-2,2-dimethyl-3-oxazolidincarboxylat als kristallinen Festkörper; MS : 366 $(M-CH_3)^+$.

(e) (2R/S,3S,4S)-4-Amino-5-cyclohexyl-2-cyclopropyl-2,3-pentandiol:

Analog zu dem oben zur Herstellung des (2RS,3R,4S)-4-Amino-5-cyclohexyl-1,2,3-pentantriols beschriebenen Verfahren, wird durch Umsetzung von tert-Butyl (4S,5R)-4-(cyclohexylmethyl)-5-[(R/S)-1-cyclopropyl-1-hydroxyäthyl-2,2-dimethyl-3-oxazolidincarboxylat das (2R/S,3S,4S)-4-Amino-5-cyclohexyl-2-cyclopropyl-2,3-pentandiol als amorpher Festkörper erhalten, welcher ohne weitere Reinigung in die nächste Stufe eingesetzt wird.

## Beispiel 25

In analoger Weise wie in Beispiel 23 beschrieben, wird durch Kondensation von N-[(S)-α-[(tert-Butylsulfonyl)-methyl]hydrocinnamoyl]-1-(2,4dinitrophenyl)-L-histidin und (2R,3S)-3-Amino-4-cyclohexyl-1-cyclopropyl-2-hydroxy-1-butanon das (S)-α-[(S)α-[(tert-Butylsulfonyl)methyl]-hydrocinnamamido]-N-[(1S,2R)-1-(cyclohexylmethyl)-3-cyclopropyl-2-hydroxy-3-oxopropyl]-1-(2,4-dinitrophenyl)imidazol-4-propionamid als gelber Festkörper erhalten, MS : 795 $(M+H)^+$.

Das als Ausgangsstoff eingesetzte N-[(S)-α-[(tert-Butylsulfonyl)methyl]hydrocinnamoyl]-1-(2,4-dinitrophenyl)-L-histidin wurde wie folgt hergestellt:

23.3 g (54.6 mMol) (S)-α-[(S)-α-[(tert-Butylsulfonyl)methyl]hydrocinnamamido]-imidazolpropionsäuremethylester in 250 ml Methylenchlorid werden mit 7,43 ml (54,6 mMol) Triäthylamin versetzt. Anschliessend werden unter Eiskühlung 9,94 g (54,6 mMol) 2,4-Dinitro-1-fluorbenzol in 100 ml Methylenchlorid innerhalb von etwa 20 Minuten zugetropft, und das Reaktionsgemisch bis zu vollständigen Umsetzung bei Raumtemperatur gerührt, wobei dies nach 4 Stunden der Fall ist (dünnschichtchromatographisch überprüft). Uebliche Aufarbeitung des Reaktionsgemisches liefert 20,5 g (62%) N-[(S)-α-[(tert-Butylsulfonyl)methyl]hydrocinnamoyl]-1-(2,4-dinitrophenyl)-L-histidinmethylester als braunen Schaum, Rf-Wert 0,4 in einem 30:1-Gemisch von Methylenchlorid und Methanol, MS: 602 $(M+H)^+$.

20,5 g (34,07 mMol) N-[(S)-α-[(tert-Butylsulfonyl)-methyl]hydrocinnamoyl]-1-(2,4-dinitrophenyl)-L-histidinmethylester werden in 180 ml Dioxan gelöst, mit 85 ml (170,34 mMol) 2N Salzsäure versetzt und anschliessend 2,5 Stunden auf 80° erhitzt. Uebliche Aufarbeitung und Kristallisation aus Aether/Hexan liefert 15,7 g (78%) N-[(S)-α-[(tert-Butylsulfonyl)methyl]hydrocinnamoyl]-1-(2,4-dinitrophenyl)-L-histidin in Form eines hellgelben amorphen Festkörpers, Rf-Wert 0,2 in einem 5:1-Gemisch von Methylenchlorid und Methanol, MS: 588 $(M+H)^+$.

Das als Ausgangsstoff eingesetzte (2R,3S)-3-Amino-4-cyclohexyl-1-cyclopropyl-2-hydroxy-1-butanon wurde wie folgt hergestellt:

(2R,3S)-3-Amino-4-cyclohexyl-1-cyclopropyl-2-hydroxy-1-butanon:

Analog zu dem in Beispiel 24 zur Herstellung des (2RS,3R,4S)-4-Amino-5-cyclohexyl-1,2,3-pentantriols

beschriebenen Verfahren, wird ausgehend von tert-Butyl (4S,5R)-4-(cyclohexylmethyl)-5-(cyclopropylcarbonyl)-2,2-dimethyl-3-oxazolidincarboxylat das (2R,3S)-3-Amino-4-cyclohexyl-1-cyclopropyl-2-hydroxy-1-butanon erhalten, MS : 226 $(M+H)^+$.

Beispiel 26

Ein Gemisch von 62.8 mg (0.1 mMol) (S)-α-[(S)-α-[(tert-Butylsulfonyl)methyl]hydrocinnamamido]-N-[(1S,2R)-1-(cyclohexylmethyl)-3-cyclopropyl-2 hydroxy-3-oxopropyl]imidazol-4-propionamid, 12 mg (0.2 mMol) Hydroxylamin-hydrochlorid und 0.027 ml (0.2 mMol) Triäthylamin in 2.5 ml Methanol wird 20 Stunden lang zum Rückfluss erhitzt. Nach üblicher Aufarbeitung wird das erhaltene Rolprodukt zur Reinigung an 10 g Kieselgel unter Verwendung eines 8:1-Gemisches von Methylenchlorid und Methanol als Eluierungsmittel chromatographiert. Man erhält 28 mg (S)-α-[(S)-α-[(tert-Butylsulfonyl)methyl]-hydrocinnamamido]-N-[(1S,2R,E/Z = 2:1)-1-(cyclohexylmethyl)-3-cyclopropyl-2-hydroxy-3-(hydroxyimino)-propyl]imidazol-4-propionamid als amorphen Festkörper; MS : 644 $(M+H)^+$.

Beispiel 27

Eine Lösung von 85 mg (0,12 mMol) (R)-2-[[(S)-α-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]phenäthyl]carbamoyl]-1-pyrrolidincarbonsäure-tert-butylester in 1 ml Methanol und 1 ml 2N Salzsäure wird 2 Stunden bei 50° gerührt. Anschliessend wird das Reaktionsgemisch unter vermindertem Druck eingedampft und zweimal mit Toluol azeotrop entwässert. Zur Reinigung wird der Rückstand an 20 g Kieselgel unter Verwendung eines 65:10:1-Gemisches von Methylenchlorid, Methanol und Ammoniak als Eluierungsmittel chromatographiert. Die erhaltenen 50 mg Rohprodukt werden danach in einem Methylenchlorid-Methanol-Aether-Gemisch umgefällt. Man erhält 35 mg (S)-N-[(1S,2R,3S)-1-(Cyclohexylmethyl)-3-cyclopropyl-2,3-dihydroxypropyl]-α-[(S)-α-(D-propylamino)hydrocinnamamido]imidazol-4-propionamid als farbloses Pulver; MS : 609 $(M+H)^+$.

Beispiel 28

In analoger Weise wie in Beispiel 27 beschrieben, wurden folgende Verbindungen erhalten:

- Aus Di-tert-butyl N-[(S)-1-(tert-butoxycarbonyl)-2-[[(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]phenäthyl]carbamoyl]äthyl]-äthylendicarbamat das N2-(2-Aminoäthyl)-N4-[(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]phenäthyl]-L-asparagin als amorpher Festkörper, MS : 670 $(M+H)^+$;
- aus Di-tert-butyl N-[[[(S)-α-[[(S)-1-[[(1S,2R,3S)-1-(cyclohexylmethyl)-3-cyclopropyl-2,3-dihydroxypropyl]-carbamoyl]-2-imidazol-4-yläthyl]carbamoyl]phenäthyl]carbamoyl]methyl]äthylendicarbamat das (S)-α-[(S)-α-[2-[(2-Aminoäthyl)amino]acetamido]hydrocinnamamido]-N-[(1S,2R,3S)-1-cyclohexylmethyl)-3-cyclopropyl-2,3-dihydroxypropyl]imidazol-4-propionamid als amorpher Festkörper, MS : 612 $(M+H)^+$.

Beispiel 29

Ein Gemisch von 610 mg (0.8 mMol) rohem (S)-α-[(S)-α-[(tert-Butylsulfonyl)methyl]hydrocinnamamido]-N-[(1S,2R,3S)-1-(cyclohexylmethyl)-3-cyclopropyl-3-fluor-2-hydroxypropyl]-1-Boc-imidazol-4-propionamid und 110 mg (0.8 mMol) Kaliumcarbonat in 10 ml Methanol wird 1 Stunde bei Raumtemperatur unter Argon gerührt. Anschliessend werden 100 mg (1.9 mMol) Ammoniumchlorid zugegeben. Das Gemisch wird 15 Minuten bei Raumtemperatur weitergerührt und danach unter vermindertem Druck eingedampft. Der Rückstand wird in 20 ml Essigester gelöst und nacheinander mit 10 ml Wasser und 10 ml gesättigter Natriumchlorid-Lösung gewaschen. Die Wasserphasen werden zweimal mit 20 ml Essigester nachextrahiert. Die gesammelten organischen Phasen werden über Natriumsulfat getrocknet und unter vermindertem Druck

eingedampft. Der Rückstand (300 mg Schaum) wird zur Reinigung an 50 g Kieselgel unter Verwendung eines 9:1:0.1-Gemisches von Methylenchlorid, Isopropanol und Ammoniak als Eluierunsmittel chromatographiert. Nach dem Lyophilisieren aus Dioxan werden 95 mg (S)-α-[(S)-α-[(tert-Butylsulfonyl)methyl]-hydrocinnamamido]-N-[(1S,2R,3S)-1-(cyclohexylmethyl)-3-cyclopropyl-3-fluor-2-hydroxypropyl]imidazol-4-propionamid als farbloses Pulver erhalten; MS : 633 $(M+H)^+$.

## Beispiel 30

In analoger Weise wie in Beispiel 29 beschrieben, wurden folgende Verbindungen hergestellt:

- Aus (S)-α-[(S)-α-[(tert-Butylsulfonyl)methyl]hydrocinnamamido]-N-[(1S,2R,3R)-1-(cyclohexylmethyl)-3-cyclopropyl-3-fluor-2-hydroxypropyl]-1-Boc-imidazol4-propionamid das (S)-α-[(S)-α-[(tert-Butylsulfonyl)-methyl]hydrocinnamamido]-N-[(1S,2R,3R)-1-(cyclohexylmethyl)-3-cyclopropyl-3-fluor-2-hydroxypropyl]-imidazol-4-propionamid als farbloser, amorpher Festkörper, MS : 633 $(M+H)^+$;
- aus (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]-1-Boc-imidazol-4-propionamid das (S)-α-[(S)-α-[(tert-Butylsulfonyl)methyl]-hydrocinnamamido]-N-[(1S,2R,3S)-1-(cyclohexylmethyl)-3-cyclopropyl-2,3-dihydroxypropyl]-imidazol-4-propionamid, MS : 631 $(M+H)^+$, als amorpher Festkörper;
- aus (S)-N-[(1S,2R,3S)-3-Azido-1-(cyclohexylmethyl)-3-cyclopropyl-Z-hydroxypropyl]-α-[(S)-α-(tert-butylsulfonyl)methyl]hydrocinnamamido]-1-Boc-imidazol-4-propionamid das (S)-N-[(1S,2R,3S)-3-Azido-1-(cyclohexylmethyl)-3-cyclopropyl-2-hydroxypropyl]-α-[(S)-α-(tert-butylsulfonyl)methyl]hydrocinnamamido]-imidazol-4-propionamid, MS : 656 $(M+H)^+$;
- aus (S)-α-[(S)-α [(tert-Butylsulfonyl)methyl]hydrocinnamamido]-N-[(1S,2R,3R/S)-1-(cyclohexylmethyl)-3-cyclopropyl-2,3-dihydroxybutyl]-1-Boc-imidazol-4-propionamid das (S)-α-[(S)-α-[(tert-Butylsulfonyl)methyl]-hydrocinnamamido]-N-[(1S,2R,3R/S)-1-(cyclohexylmethyl)-3-cyclopropyl-2,3-dihydroxybutyl]imidazol-4-propionamid, MS 645 $(M+H)^+$, als amorpher Festkörper.

## Beispiel 31

Ein Gemisch von 375 mg (0.39 mMol) (S)-α-2-Benzimidazolcarboxamido-N-[(1S,2R,3S)-1-(cyclohexylmethyl)-3-cyclopropyl-2,3-dihydroxypropyl]-1-tritylimidazol-4-propionamid und 1.8 g (15.6 mMol) Pyridinium-hydrochlorid in 40 ml Methanol wird 3.5 Stunden zum Rückfluss erhitzt. Danach wird das Reaktionsgemisch eingedampft, und der Rückstand zwischen 100 ml Essigester und 50 ml Wasser verteilt. Die organische Phase wird mit 100 ml halbgesättigter Ammoniumchlorid-Lösung und 100 ml 2N Kaliumbicarbonat-Lösung gewaschen, danach über Natriumsulfat getrocknet und unter vermindertem Druck eingedampft. Der Rückstand wird zur reinigung an 40 g Kisselgel unter Verwendung eines 6:1-gemisches von Methylenchlorid und Methanol als Eluierungsmittel chromatographiert. Es werden 110 mg N-[(S)-1-[[(1S,2R,3S)-1-(Cyclohexylmethyl)-3-cyclopropyl-2,3-dihydroxypropyl]carbamoyl]-2-imidazol-4-yläthyl]-2-benzimidazolcarboxamid als amorpher Festkörper erhalten; MS : 509 $(M+H)^+$.

## Beispiel 32

In analoger Weise wie in Beispiel 31 beschrieben, wurden die folgenden Verbindungen hergestellt:

- Aus N-[(S)-1-[[(1S,2R,3S)-1-(Cyclohexylmethyl)-3-cyclopropyl-2,3-dihydroxypropyl]carbamoyl]-2 (1-tritylimidazol)-4-yläthyl]indol-2-carboxamid das N-[(S)-1-[[(1S,2R,3S)-1 (Cyclohexylmethyl)-3-cyclopropyl-2,3-dihydroxypropyl]carbamoyl]-2-imidazol-4-yläthyl]indol-2-carboxamid als amorpher Festkörper, MS : 508 $(M+H)^+$;
- aus N-[(S)-1-[[(1S,2R,3S)-1-(Cyclohexylmethyl)-3-cyclopropyl-2,3-dihydroxypropyl]carbamoyl]-2-(1-tritylimidazol)-4-yläthyl]-5-hydroxyindol-2-carboxamid das N-[(S)-1-[[(1S,2R,3S)-1-(Cyclohexylmethyl)-3-cyclopropyl-2,3-dihydroxypropyl]carbamoyl]-2-imidazol-4-yläthyl]-5-hydroxy-indol-2-carboxamid als amorpher Festkörper, MS : 524 $(M+H)^+$;
- aus (S)-α-Benzothiazolcarboxamido-N-[(1S,2R,3S)-1-(cyclohexylmethyl)-3-cyclopropyl-2,3-dihydroxypropyl]-1-tritylimidazol-4-propionamid das (S)-α-Benzothiazolcarboxamido-N-[(1S,2R,3S)-1-(cyclohexylmethyl)-3-cyclopropyl-2,3-dihydroxypropyl]imidazol-4-propionamid als amorpher Festkörper, MS

: 526 $(M+H)^+$;

- aus (S)-N-[(1S,2R,3S)-1-(Cyclohexylmethyl)-3-cyclopropyl-2,3-dihydroxypropyl]-α-pyrrol-2-carboxamido-1-tritylimidazol-4-propionamid das (S)-N-[(1S,2R,3S)-1-(Cyclohexylmethyl)-3-cyclopropyl-2,3-dihydroxypropyl]-α-pyrrol-2-carboxamidoimidazol-4-propionamid als amorpher Festkörper, MS : 458 $(M+H)^+$;
- aus (S)-N-[(1S,2R,3S)-1-(Cyclohexylmethyl)-3-cyclopropyl-2,3-dihydroxypropyl]-α-(2,2-dibenzylacetamido)-1-tritylimidazol-4-propionamid das (S)-N-[(1S,2R,3S)-1-(Cyclohexylmethyl)-3-cyclopropyl-2,3-dihydroxypropyl]-α-(2,2-dibenzylacetamido)imidazol-4-propionamid als amorpher Festkörper, MS : 587 $(M+H)^+$;
- aus Aethyl (RS)-3-[(S)-1-[[(1S,2R,3S)-1-(cyclohexylmethyl)-3-cyclopropyl-2,3-dihydroxypropyl]carbamoyl]-2-(1-tritylimidazol)-4-yläthyl]-3,4-dihydro-2(1H)-isochinolincarboxylat (1:1 Epimerengemisch) das Aethyl (RS)-3 [(S)-1-[[(1S,2R,3S)-1-(cyclohexylmethyl)-3-cyclopropyl-2,3-dihydroxypropyl]carbamoyl]-2-imidazol-4-yläthyl]-3,4-dihydro-2(1H)-isochinolincarboxylat (1:1 Epimerengemisch) als amorpher Festkörper, MS : 596 $(M+H)^+$.

## Beispiel 33

In analoger Weise wie in Beispiel 29 beschrieben, wurde, ausgehend von (S)-α-[(S)-α-[(tert-Butylsulfonyl)methyl]hydrocinnamamido]-N-[(1S,2R)-1-(cyclohexylmethyl)-3-cyclopropyl-2-hydroxy-3-oxopropyl]-1-(2,4-dinitrophenyl)imidazol-4-propionamid durch Behandlung mit Kaliumcarbonat in Methanol das (S)-α-[(S)-α-[(tert-Butylsulfonyl)methyl]hydrocinnamamido]-N-[(1S,2R)-1-(cyclohexylmethyl)-3-cyclopropyl-2-hydroxy-3-oxopropyl]imidazol-4-propionamid als amorpher Festkörper erhalten; MS : 629 $(M+H)^+$.

## Beispiel 34

Eine Suspension von 450 mg (0.57 mMol) Benzyl [[(2S oder R,3R,4S)-4-[(S)-α-[(S)-α-[(tert-butylsulfonyl)methyl]hydrocinnamamido]imidazol-4-propionamido]-5-cyclohexyl-2,3-dihydroxypentyl]-cyclopropylcarbamat und 90 mg Palladium/Kohle (10%ig) in 20 ml Eisessig wird während 5.5 Stunden bei Raumtemperatur hydriert. Anschliessend wird der Katalysator abfiltriert, das Filtrat eingedampft, und der erhaltene Rückstand (440 mg) zur Reinigung unter Verwendung eines 140:10:1-Gemisches von Methylenchlorid, Methanol und Ammoniak als Eluierungsmittel chromatographiert. Es werden 103 mg (S)-α-[(S)-α-[(tert-Butylsulfonyl)methyl]hydrocinnamamido]-N-[(1S,2R)-1-(cyclohexylmethyl)-2-[(S oder R)-3-cyclopropyl-2-oxo-5-oxazolindinyl]-2-hydroxyäthyl]imi dazol-4-propionamid als amorpher Festkörper erhalten; MS : 660 $(M+H)^+$.

Das als Ausgangsstoff eingesetzte Benzyl [[(2S oder R,3R,4S)-4-[(S)-α-[(S)-α-[(tert-butylsulfonyl)-methyl]hydrocinnamamido]imidazol-4-propionamido]-5-cyclohexyl-2,3-dihydroxypentyl]cyclopropylcarbamat wurde wie folgt hergestellt:

(a) tert-Butyl (4S,5R)-4-(cyclohexylmethyl)-5-[(S)-2-(cyclopropylamino)-1-hydroxyäthyl]-2,2-dimethyl-3-oxazolidincarboxylat (und tert-Butyl (4S,5R)-4-(cyclohexylmethyl)-5-[(R)-2-(cyclopropylamino)-1-hydroxyäthyl]-2,2-dimethyl-3-oxazolidincarboxylat):

Ein Gemisch von 733 mg (2.16 mMol) tert-Butyl (4S,5R)-4-(cyclohexylmethyl)-2,2-dimethyl-5-[(RS)-2-oxiranyl]-3-oxazolidincarboxylat (WO 88/4664) und 2 ml Cyclopropylamin in 15 ml Methanol wird über Nacht bei 50° unter Argon gerührt. Danach wird das Reaktionsgemisch eingedampft, und der Rückstand zur Reinigung und Trennung der Epimeren an 100 g Kieselgel unter Verwendung eines 98:2-Gemisches von Chloroform und Methanol als Eluierungsmittel chromatographiert. Man erhält 185 mg des weniger polaren tert-Butyl (4S,5R)-4-(cyclohexylmethyl)-5-[(S)-2-(cyclopropylamino)-1-hydroxyäthyl]-2,2-dimethyl-3-oxazolidincarboxylat, MS : 397 $(M+H)^+$, und 341 mg des polareren tert-Butyl (4S,5R)-4-(cyclohexylmethyl)-5-[(R)-2-(cyclopropylamino)-1-hydroxyäthyl]-2,2-dimethyl-3-oxazolidincarboxylat, MS : 397 $(M+H)^+$, jeweils als farbloses Oel.

(b) tert-Butyl (4S,5R)-5-[(S)-2-[1-(benzyloxy)-N-cyclopropylformamido]-1-hydroxyäthyl]-4-(cyclohexylmethyl)-2,2-dimethyl-3-oxazolidincarboxylat:

Ein Gemisch von 180 mg (0.45 mMol) tert-Butyl (4S,5R)-4-(cyclohexylmethyl)-5 [(S)-2-(cyclopropylamino)-1- hydroxyäthyl]-2,2-dimethyl-3-oxazolidincarboxylat, 0.126 ml (0.98 mMol) Triäthylamin und 136 mg (0.545 mMol) N-Benzyloxycarbonyloxy)succinimid in 5 ml Methylenchlorid wird 2 Stunden bei Raumtemperatur gerührt. Anschliessend wird der Rückstand auf Eis und 2N Natriumbicarbonat-Lösung gegossen und dann dreimal mit je 150 ml Aether extrahiert. Die organische Phase wird mit 70 ml Wasser gewaschen, dann über Magnesiumsulfat getrocknet und eingedampft. Es werden 296 mg tert-Butyl (4S,5R)-5-[(S)-2-[1-(benzyloxy)-N-cyclopropylformamido]-1-hydroxyäthyl]-4-(cyclohexylmethyl)-2,2-dimethyl-3-oxazolidincarboxylat als farbloses Oel erhalten; MS : 531 $(M+H)^+$.

(c) Benzyl [(2S oder R,3R,4S)-4-amino-5-cyclohexyl-2,3-dihydroxypentyl]cyclopropylcarbamat:

In analoger Weise wie in Beispiel 24 beschrieben, wurde nach Abspaltung der Boc- und Isopropyliden-Schutzgruppe mittels Salzsäure in Methanol aus tert-Butyl (4S,5R)-5-[(S)-2-[1-(benzyloxy)-N-cyclopropylformamido]-1-hydroxyäthyl]-4-(cyclohexylmethyl)-2,2-dimethyl-3-oxazolidincarboxylat das Benzyl [(2S oder R,3R,4S)-4-amino-5-cyclohexyl-2,3-dihydroxypentyl]cyclopropylcarbamat als amorpher Festkörper erhalten.

(d) Benzyl [[(2S oder R,3R,4S)-4-[(S)-α-[(S)-α-[(tert-butylsulfonyl)methyl]hydrocinnamamido]imidazol-4-propionamido]-5-cyclohexyl-2,3-dihydroxypentyl]cyclopropylcarbamat:

In analoger Weise wie in Beispiel 23 beschrieben, wurde durch Kondensation von (S)-1-(tert-Butoxycarbonyl)-α-[(S)-α-[(tert-butylsulfonyl)methyl]hydrocinnamamido]imidazol-4-propionsäure und Benzyl [(2S oder R,3R,4S)-4-amino-5-cyclohexyl-2,3-dihydroxypentyl]cyclopropylcarbamat und anschliessender Abspaltung der Boc-Schutzgruppe mittels Kaliumcarbonat in Methanol, analog Beispiel 29, das Benzyl [[(2S oder R,3R,4S)-4-[(S)-α-[(S)-α-[(tert-butylsul fonyl)methyl]hydrocinnamamido]imidazol-4-porpionamido]-5-cyclohexyl-2,3-dihydroxypentyl]cyclopropylcarbamat hergestellt.

## Beispiel 35

In analoger Weise wie in Beispiel 34 beschrieben, wurde durch katalytische Hydrierung von Benzyl [[-(2R oder S,3R,4S)-4-[(S)-α-[(S)-α-[(tert-butylsulfonyl)methyl]hydrocinnamamido]imidazol-4-propionamido]-5-cyclohexyl-2,3-dihydroxypentyl]cyclopropylcarbamat das (S)-α-[(S)-α-[(tert-Butylsulfonyl)methyl]-hydrocinnamamido]-N-[(1S,2R)-1-(cyclohexylmethyl)-2-[(R oder S)-3-cyclopropyl-2-oxo-5-oxazolidinyl]-2-hydroxyäthyl]imidazol-4-propionamid als amorpher Festkörper erhalten; MS : 660 $(M+H)^+$.

Das als Ausgangsstoff eingesetzte benzyl [[(2R oder S,3R,4S)-4-[(S)-α-[(S)-α-[(tert-butylsulfonyl)methyl]-hydrocinnamamido]imidazol-4-propionamido]-5-cyclohexyl-2,3-dihydroxypentyl]cyclopropylcarbamat wurde wie folgt hergestellt:

Ausgehend von tert-Butyl (4S,5R)-4-(cyclohexylmethyl)-5-[(R)-2-(cyclopropylamino)-1 hydroxyäthyl]-2,2-dimethyl-3-oxazolidincarboxylat, wurde in einer zu Beispiel 34(b)-(d) analogen Weise durch Einführung der Benzyloxycarbonyl-Schutzgruppe mittels N-Benzyloxycarbonyloxy)succinimid, gefolgt von der Abspaltung der Boc- und Isopropyliden-Schutzgruppe mittels Salzsäure in Methanol, das Benzyl [[(2R oder S,3R,4S)-4-[(S)-α-[(S)-α-[(tert-butylsulfonyl)methyl]hydrocinnamamido]imidazol-4-propionamido]-5-cyclohexyl-2,3-dihydroxypentyl]cyclopropylcarbamat erhalten.

## Beispiel 36

Eine Suspension von 60 mg (0,09 mMol) (S)-N-[(1S,2R,3S)-3-Azido-1-(cyclohexylmethyl)-3-cyclopropyl-2-hydroxypropyl]- α-[(S)-α-[(tert-butylsulfonyl)methyl]hydrocinnamamido]imidazol-4-propionamid und 20 mg Palladium/Kohle (5%ig) in 15 ml Methanol wird 4 Stunden bei Raumtemperatur hydriert. Nach dem Abfiltrieren des Katalysators wird das Lösungsmittel unter vermindertem Druck abgedampft. Das Rohprodukt (40 mg) wird zur Reinigung an 10 g Kieselgel unter Verwendung eines 80:10:1-Gemisches von Methylenchlorid, Methanol und Ammoniak als Eluierungsmittel chromatographiert. Es werden 35 mg (S)-N-[-(1S,2R,3S)-3-Amino-1-(cyclohexylmethyl)-3-cyclopropyl-2-hydroxypropyl]-α-[(S)-α-[(tert-butylsulfonyl)-methyl]hydrocinnamamido]imidazol-4-propionamid als farbloser Festkörper erhalten; MS : 630 $(M+H)^+$.

## Beispiel 37

Eine Lösung von 90 mg (0.3 mMol) (S)-α-[(S)-α-[(tert-Butylsulfonyl)methyl]hydrocinnamamido]-N-[(S)-1-[(4R,5S)-5-cyclopropyl-2,2-dimethyl-1,3-dioxolan-4-yl]-2-(4-oxocyclohexyl)äthyl]imidazol-4-propionamid in 1 ml Methanol und 1 ml 2N Salzsäure wird 3 Stunden bei Raumtemperatur gerührt. Anschliessend wird die Reaktionslösung eingedampft, und der Rückstand zur Reinigung an 20 g Kieselgel unter Verwendung eines 14:1:0.1-Gemisches von Methylenchlorid, Methanol und Ammoniak als Eluierungsmittel chromatographiert. Es werden 13 mg (S)-α-[(S)-α-[(tert-Butylsulfonyl)methyl]hydrocinnamamido]-N-[(1S,2R,3S)-3-cyclopropyl-2,3-dihydroxy-1-[(4-oxocyclohexyl)methyl]propyl]imidazol-4-propionamid als farbloser Festkörper erhalten; MS : 684 $(M+H)^+$.

Das als Ausgangsstoff eingesetzte (S)-α-[(S)-α-[(tert-Butylsulfonyl)methyl]hydrocinnamamido]-N-[(S)-1-[-(4R,5S)-5-cyclopropyl-2,2-dimethyl-1,3-dioxolan-4-yl]-2-(4-oxocyclohexyl)äthyl]imidazol-4-propionamid wurde wie folgt hergestellt:

(a) Boc-[(RS)-4-Hydroxycyclohexyl]alaninmethylester:

Eine Suspension von 65,8 g (0,22 Mol) Boc-Tyrosinmethylester und 6,6 g Rhodium/Aluminiumoxid-Katalysator (5%ig) in 175 ml Methanol wird 3 Stunden bei 50° und 4 bar Wasserstoffdruck hydriert. Anschliessend wird der Katalysatorabfiltriert, das Filtrat unter vermindertem Druck eingedampft, und der erhaltene Rückstand zur Reinigung an 1 kg Kieselgel unter Verwendung eines 4:1-Gemisches von Toluol und Essigester als Eluierungsmittel chromatographiert. Es werden 41,97 g Boc-[(RS)-4-Hydroxycyclohexyl]-alaninmethylester, MS : 302 $(M+H)^+$, und 6,1 g Boc-(4-Oxocyclohexyl)alaninmethylester, MS : 300 $(M+H)^+$, jeweils als farbloses Oel, erhalten.

(b) (1S,2R,3S)-Amino-1-cyclopropyl-4-[(RS)-4-hydroxycyclohexyl]-1,2-butandiol:

Diese Verbindung wurde analog zu dem Verfahren, das in WO 87/05302 zur Synthese des tert-Butyl (4S,5R)-4-(cyclohexylmethyl)-5-formyl-2,2-dimethyl-3-oxazolidincarboxylats aus Boc-Cyclohexylalaninmethylester beschrieben ist, durch Ersatz des Boc-Cyclohexylalaninmethylesters durch Boc-[(RS)-4-Hydroxycyclohexyl]alaninmethylester, gefolgt von der Grignard-Reaktion mit Cyclopropylmagnesiumbromid und Abspaltung der Boc- und Isopropyliden-Schutzgruppe analog Beispiel 16(a) und (b) als amorpher Festkörper erhalten, MS : 244 $(M+H)^+$.

(c) (S)-α-[(S)-α-[(tert-Butylsulfonyl)methyl]hydrocinnamamido]-N-[(1S,2R,3S)-1-[[(RS)-4-hydroxycyclohexyl]-methyl]-3-cyclopropyl-2,3-dihydroxypropyl]imidazol-4-propionamid:

Analog zu den in Beispiel 23 und 29 beschriebenen Verfahren, wird durch Kondensation von (S)-1-(tert-Butoxycarbonyl)-α-[(S)-α-[(tert-Butylsulfonyl)methyl]hydrocinnam amido]imidazol-4-propionsäure mit (1S,2R)-3-Amino-1-cyclopropyl-4-[(RS)-4-hydroxycyclohexyl]-1,2-butandiol und nachfolgender Abspaltung der Boc-Schutzgruppe das (S)-α-[(S)-α-[(tert-Butylsulfonyl)methyl]hydrocinnamamido]-N-[(1S,2R, 3S)-1-[[-(RS)-4-hydroxycyclohexyl]methyl]-3-cyclopropyl-2,3-dihydroxypropyl]imidazol-4-propionamid als farbloser Festkörper erhalten; MS : 647 $(M+H)^+$.

(d) (S)-α-[(S)-α-[(tert-Butylsulfonyl)methyl]hydrocinnamamido]-N-[(S)-1-[(4R,5S)-5-cyclopropyl-2,2-dimethyl-1,3-dioxolan-4-yl]-2-[(RS)-4-hydroxycyclohexyl]äthyl]imidazol-4-propionamid:

Ein Gemisch von 300 mg (0.46 mMol) (S)-α-[(S)-α-[(tert-Butylsulfonyl)methyl]hydrocinnamamido]-N-[-

(1S,2R,3S)-1-[[(RS)-4-hydroxycyclohexyl]methy]-3-cyclopropyl-2,3-dihydroxypropyl]imidazol-4-propionamid, 300 mg (1.5 mMol) p-Toluolsulfonsäure und 15 ml (122 mMol) 2,2-Dimethoxypropan in 5 ml Aceton wird 2 Stunden bei Raumtemperatur gerührt. Anschliessend wird das Reaktionsgemisch auf Eis und 70 ml 2N Natriumbicarbonat-Lösung gegossen und zweimal mit je 150 ml Essigester extrahiert. Die vereinigten organischen Extrakte werden mit 70 ml Wasser gewaschen, danach über Natriumsulfat getrocknet und unter vermindertem Druck eingedampft. Der Rückstand wird an 30 g Kieselgel unter Verwendung eines 14:1:0.1-Gemisches von Methylenchlorid, Methanol und Ammoniak als Eluierungsmittel chromatographiert. Man erhält 270 mg (S)-α-[(S)-α-[(tert-Butylsulfonyl)methyl]hydrocinnamamido]-N-[(S)-1-[(4R,5S)-5-cyclopropyl-2,2-dimethyl-1,3-dioxolan-4-yl]-2-[(RS)-4-hydroxycyclohexyl]äthyl]imidazol-4-propionamid als farblosen Schaum; MS : 687 $(M+H)^+$.

(e) (S)-α-[(S)-α-[(tert-Butylsulfonyl)methyl]hydrocinnamamido]-N-[(S)-1-[(4R,5S)-5-cyclopropyl-2,2-dimethyl-1,3-dioxolan-4-yl]-2-(4-oxocyclohexyl)äthyl]imidazol-4-propionamid:

Ein Gemisch von 260 mg (0.38 mMol)(S)-α-[(S)-α-[(tert-Butylsulfonyl)methyl]hydrocinnamamido]-N-[(S)-1-[(4R,5S)-5-cyclopropyl-2,2-dimethyl-1,3-dioxolan-4-yl]-2-[(RS)-4-hydroxycyclohexyl]äthyl]imidazol-4-propionamid und 300 mg (1.9 mMol) Pyridin-Schwefeltrioxid-Komplex in 5 ml Dimethylsulfoxid wird 1.5 Stunden bei Raumtemperatur gerührt. Das Reaktionsgemisch wird danach auf gesättigte Ammoniumchlorid-Lösung gegossen und dreimal mit je 150 ml Essigester extrahiert. Die vereinigten organischen Extrakte werden mit 70 ml gesättigter Ammoniumchlorid-Lösung und 70 ml Wasser gewaschen, über Natriumsulfat getrocknet und eingedampft. Der Rückstand (190 mg) wird an 20 g Kieselgel unter Verwendung eines 14:1:0.1-Gemisches von Methylenchlorid, Methanol und Ammoniak als Eluierungsmittel chromatographiert. Man erhält 90 mg (S)-α-[(S)-α-[(tert-Butylsulfonyl)methyl]hydrocinnamamido]-N-[(S)-1-[(4R,5S)-5-cyclopropyl-2,2-dimethyl-1,3-dioxolan-4-yl]-2-(4-oxocyclohexyl)äthyl]imidazol-4-propionamid als farbloses Oel; MS : 685 $(M+H)^+$.

## Beispiel 38

Eine Lösung von 335 mg (0,44 mMol) (S)-α-[(S)-α-[(tert-Butylsulfonyl)methyl]hydrocinnamamido]-N-[-(1S,2R,3S)-1-(cyclohexylmethyl)-3-cyclopropyl-2,3-dihydroxypropyl]-5-jodimidazol-4-propionamid und 79 mg (0,88 mMol) Kupfer-(I)-cyanid in 25 ml Dimethylformamid wird über Nacht bei 120° unter Argon gerührt. Anschliessend wird das Reaktionsgemisch unter vermindertem Druck eingedampft, der Rückstand in 30 ml eines 95:5-Gemisches von Methylenchlorid und Methanol aufgeschlämmt und über Dicalite filtriert. Das Filtrat wird eingedampft, und der Rückstand zur Reinigung an 50 g Kieselgel unter Verwendung eines 160:10:1-Gemisches von Methylenchlorid, Methanol and Ammoniak als Eluierungsmittel chromatographiert. Nach dem Ausfällen aus einem Methylenchlorid-Aether-Gemisch werden 40 mg (S)-α-[(S)-α-[(tert-Butylsulfonyl)methyl]hydrocinnamamido]-N-[(1S,2R,3S)-1-(cyclohexylmethyl)-3 cyclopropyl-2,3 dihydroxypropyl]-5-cyan-imidazol-4-propionamid als farbloses Pulver erhalten; MS : 656 $(M+H)^+$.

Das als Ausgangsstoff eingesetzte (S)-α-[(S)-α-[(tert-Butylsulfonyl)methyl]hydrocinnamamido]-N-(1S,2R,3S)-1-(cyclohexylmethyl)-3-cyclopropyl-2,3-dihydroxypropyl]-5-jodimidazol-4-propionamid wurde wie folgt hergestellt:

Zu einem Gemisch von 630 mg (1 mMol) (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid und 20 ml (4 mMol) 0,2N Natronlauge in 20 ml Methanol werden bei 0° unter Rühren gleichzeitig 10,4 ml (2,08 mMol) einer 0,2N methanolischen Jodlösung und 2,4 ml (0,48 mMol) 0,2N Natronlauge getropft. Die farblose Reaktionsmischung wird 5 Minuten bei 0° und danach 2,5 Stunden bei Raumtemperatur gerührt. Anschliessend wird mit 25 ml Wasser verdünnt und zweimal mit je 80 ml Essigester extrahiert. Die vereinigten organischen Extrakte werden mit gesättigter Natriumchlorid-Lösung gewaschen, über Natriumsulfat getrocknet und unter vermindertem Druck eingedampft. Der Rückstand (750 mg) wird zur Reinigung unter Verwendung eines 15:1-Gemisches von Methylenchlorid und Methanol als Eluierungsmittel chromatographiert. Es werden 504 mg (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]-5-jodimidazol-4-propionamid, MS : 757 $(M+H)^+$, und 38 mg (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]-2,5-dijodimidazol-4-propionamid, MS : 883 $(M+H)^+$, jeweils als farbloser Fest körper, erhalten.

Beispiel 39

Zu einer Lösung von 150 mg ( 0.2 mMol) (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid in 15 ml Methylenchlorid werden bei Raumtemperatur 1.5 ml (2.8 mMol) Phosgen in Toluol (20%ig) gegeben, und die Reaktionslösung 3 Stunden gerührt. Danach wird eingedampft und der Rückstand zur Reinigung an 10 g Kieselgel unter Verwendung eines 96:4-Gemisches von Methylenchlorid und Methanol als Eluierungsmittel chromatographiert. Nach dem Lyophilisieren aus Dioxan/Wasser erhält man 57 mg (1R,2S)-1-[(S)-1-[(S)-α-[(S)-α-[(tertButylsulfonyl)hydrocinnamamido]-imidazol-4-propionamido]-2 cyclohexyläthyl]-2-cyclopropyläthylen-cyclischer-kohlensäureester-hydrochlorid als farbloses Pulver; MS : 657 $(M+H)^+$.

Beispiel 40

Ein Gemisch von 200 mg (0.3 mMol) (S)-α-[(S)-α-[(tert-Butylsulfonyl)methyl]hydrocinnamamido]-N-[-(1S,2R,3S)-1-(cyclohexylmethyl)-3-cyclopropyl-2,3-dihydroxypropyl]imidazol-4-propionamid und 543 mg (3 mMol) Schwefeltrioxid-Triäthylaminkomplex in 3 ml Dimethylformamid wird 48 Stunden bei Raumtemperatur gerührt. Danach werden 480 mg (5.8 mMol) Natriumacetat, gelöst in möglichst wenig Wasser, zugegeben, und das Reaktionsgemisch im Hochvakuum eingedampft. Der Rückstand wird in 5 ml Acetonitril verrührt, danach wird vom Ungelösten abfiltriert, und das Filtrat unter vermindertem Druck eingedampft. Der erhaltene Rückstand wird zur Reinigung an 50 g Kieselgel mit einem 25:5:3-Gemisch von Essigester, Methanol und Wasser als Eluierungsmittel chromatographiert. Nach dem Lyophilisieren aus Dioxan/Wasser werden 159 mg (1R,2S)-1- [(S)-1-[(S)-α-[(S)-α-[(tert-Butylsulfonyl]hydrocinnamamido]imidazol-4-propionamido]-2-cyclohexyläthyl]-2-cyclopropyläthylen-disulfat-natriumsalz (1:2) als farbloses Pulver erhalten; MS : 808 $(M + NH_4)^+$.

Beispiel 41

Eine Suspension von 304 mg (1.6 mMol) (S)-2-Acetoxybernsteinsäuremonomethylester (Hua Hsüh Hsüh Pao 38 , 502, 1980, CA 94, 174940), 330 mg (1.6 mMol) Dicyclohexylcarbodiimid, 14 mg (0.12 mMol) 4-Dimethylaminopyridin und 252 mg (0.4 mMol) (S)-α-[(S)-α-[(tert-Butylsulfonyl)methyl]-hydrocinnamamido]-N-[(1S,2R,3S)-1-(cyclohexylmethyl)-3-cyclopropyl-2,3-dihydroxypropyl]imidazol-4-propionamid in 20 ml Methylenchlorid wird 4 Stunden bei Raumtemperatur gerührt. Anschliessend wird das Reaktionsgemisch eingedampft, der Rückstand mit 50 ml Aether verrrührt und danach von ungelöstem Dicyclohexylharnstoff abfiltriert. Nach dem Eindampfen der Aetherlösung wird der erhaltene Rückstand (500 mg) an 50 g Kieselgel unter Verwendung eines 20:1-Gemisches von Methylenchlorid und Methanol als Eluierungsmittel chromatographiert. Es werden 76 mg (1R,2S)-1-[(S)-1-[(S)-α-[(S)-α-[(tert-Butylsulfonyl)methyl]-hydrocinnamamido]imidazol-4-propionamido]-2-cyclohexyläthyl]-2-cyclopropyläthylen bis[(S)-2-acetoxy-3-(methoxycarbonyl)propionat als amorpher Festkörper erhalten; MS : 976 $(M+H)^+$.

Beispiel 42

In analoger Weise wie in Beispiel 41 beschrieben, wurden die folgenden Verbindungen hergestellt:

- Aus (S)-α-[(S)-α-[(tert-Butylsulfonyl)methyl]-hydrocinnamamido]-N-[(1S,2R,3S)-1-(cyclohexylmethyl)-3-cyclopropyl-2,3-dihydroxypropyl]imidazol-4-propionamid und 2-Acetoxybenzoesäure das (1R,2S)-1-[(S)-1-[-(S)-α-[(S)-α-[(tert-Butylsulfonyl)methyl]hydrocinnamamido]- imidazol-4-propionamido]-2-cyclohexyläthyl]-2 cyclopropyläthylen bis(o-acetoxybenzoat) als amorpher Festkörper, MS : 956 $(M+H)^+$;
- aus (S)-α [(S)-α-[(tert-Butylsulfonyl)methyl]hydrocinnamamido]-N-[(1S,2R,3S)-1-(cyclohexylmethyl)-3 cyclopropyl-2,3-dihydroxypropyl]imidazol-4-propionamid und 2-Aminobenzoesäure das (1S,2R,3S)-3-[(S)-α-[(S)-α-[(tert-Butylsulfonyl)methyl]hydrocinnamamido]imidazol-4-propionamido]-4-cyclohexyl-1-cyclopropyl-2-hydroxybutyl o-aminobenzoat als amorpher Festkörper, MS : 750 $(M+H)^+$.

Beispiel 43

Ein Gemisch von 315 mg (0.5 mMol) (S)-α-[(S)-α-[(tert-Butylsulfonyl)methyl]hydrocinnamamido]-N-[-(1S,2R,3S)-1-(cyclohexylmethyl)-3-cyclopropyl-2,3-dihydroxypropyl]imidazol-4-propionamid, 0.345 ml (2.5 mMol) Triäthylamin und 426 mg (2.5 mMol) Chloressigsäureanhydrid in 25 ml Methylenchlorid wird 2 Stunden bei Raumtemperatur gerührt. Anschliessend wird zwischen 50 ml Methylenchlorid und 50 ml Wasser verteilt, die organische Phase abgetrennt und über Natriumsulfat getrocknet. Nach dem Eindampfen wird der erhaltene Rückstand (350 mg) zur Reinigung an 25 g Kieselgel unter Verwendung eines 20:1-Gemisches von Methylenchlorid und Methanol als Eluierungsmittel chromatographiert. Es werden 371 mg (1R,2S)-1-[(S)-1-[(S)-α-[(S)-α-[(tert-Butylsulfonyl)methyl]hydrocinnamamido]imidazol-4-propionamido]-2-cyclohexyläthyl]-2-cyclopropyläthylen bis(chloracetat) als Schaum erhalten; MS : 783 $(M+H)^+$.

Eine Lösung von 371 mg (0.47 mMol) (1R,2S)-1-[(S)-1-[(S)-α-[(S)-α-[(tert-Butylsulfonyl)methyl]-hydrocinnamamido]imidazol-4-propionamido]-2-cyclohexyläthyl]-1-cyclopropyläthylen bis(chloracetat) in 15 ml (172 mMol) Morpholin wird 16 Stunden bei 90° gerührt. Danach wird das Morpholin unter vermindertem Druck abgedampft, der Rückstand in 100 ml Essigester aufgenommen und mit 100 ml Wasser gewaschen. Die organische Phase wird über Natriumsulfat getrocknet und danach eingedampft. Der erhaltene Rückstand wird zur Reinigung an 30 g Kieselgel unter Verwendung eines 10:1-Gemisches von Methylenchlorid und Methanol also Eluierungsmittel chromatographiert. Man erhält 101 mg (1R,2S)-1-[(S)-1-[(S)-α-[(S)-α-[-(tert-Butylsulfonyl)methyl]hydrocinnamamido]imidazol-3-propionamido]-2-cyclohexyläthyl]-2-cyclopropyläthylen di-4-morpholinoacetat als amorphen Festkörper; MS : 763 $[M+H-(CH_3)_3CSO_2H]^+$.

Beispiel 44

Ein Gemisch von 100 mg (0.16 mMol) (S)-α-[(S)-α-[(tert-Butylsulfonyl)methyl]hydrocinnamamido]-N-[-(1S,2R,3S)-1-(cyclohexylmethyl)-3-cyclopropyl-2,3-dihydroxypropyl]imidazol-4 propionamid, 126 mg (0.63 mMol) 2-(3-Pyridyl)benzoesäure [Z.Chem. 7 , 22 (1967)], 195 mg (10.6 mMol) 4-Dimethylaminopyridin und 212 mg (0.56 mMol) HBTU in 10 ml Methylenchlorid wird bei Raumtemperatur unter Argon 24 Stunden gerührt. Anschliessend wird das Reaktionsgemisch eingedampft, und der Rückstand in 160 ml Essigester aufgenommen. Die Essigesterlösung wird mit 80 ml 2N Natriumcarbonat-Lösung und 80 ml gesättigter Ammoniumchlorid-Lösung gewaschen. Die vereinigten wässrigen Phasen werden nochmals zweimal mit je 160 ml Essigester extrahiert. Die vereinigten Essigesterlösungen werden über Magnesiumsulfat getrocknet und unter vermindertem Druck eingedampft. Das erhaltene Rohprodukt (350 mg) wird zur Reinigung an 50 g Kieselgel unter Verwendung eines 250:10:1-Gemisches von Methylenchlorid, Methanol und Ammoniak als Eluierungsmittel chromatographiert. Man erhält 140 mg (1R,2S)-1-[(S)-1-[(S)-α-[(S)-α-[(tert-Butylsulfonyl)-methyl]hydrocinnamamido]imidazol-4-propionamido]-2-cyclohexyläthyl]-2-cyclopropyläthylen bis[o-(2-pyridyl)benzoat] als amorphen Festkörper; MS : 993 $(M+H^+)$.

Beispiel 45

In analoger Weise wie in Beispiel 44 beschrieben, wurden die folgenden Verbindungen hergestellt:

- Aus (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid und 3-(3-Pyridyl)propionsäure (J.Chem.Soc.Japan 62 , 183, 1941) das (1R,2S)-1-[(S)-1-[(S)-α-[(S)-α-[(tert-Butylsulfonyl)methyl]hydrocinnamamido]imidazol-4-propionamido]-2-cyclohexyläthyl]-2-cyclopropyläthylen di-3-pyridinpropionat als amorpher Festkörper, MS : 897 $(M+H)^+$;
- aus (S)-α-[(S)-α-[(tert-Butylsulfonyl)methyl]hydrocinnamamido]-N-[(1S,2R,3S)-1 (cyclohexylmethyl)-3-cyclopropyl-2,3-dihydroxypropyl]imidazol-4-propionamid und Cyclopropancarbonsäure der (1S,2R,3S)-3-[-(S)-α-[(S)-α-[(tert-Butylsulfonyl)methyl]hydrocinnamamido]imidazol-4-propionamido]-4-cyclohexyl-1-cyclopropyl-2-hydroxybutyl cyclopropancarboncarboxylat, MS : 699 $(M+H)^+$, und der (1R,2S)-1-[(S)-1-[(S)-α-[(S)-α-[(tert-Butylsulfonyl)methyl]hydrocinnamamido]imidazol-4-propionamido]-2-cyclohexyläthyl]-2-cyclopropyläthylen dicyclopropancarboxylat, MS : 767 $(M+H)^+$, jeweils als amorpher Festkörper;
- aus (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid und 3-(4-Imidazol)propionsäure das (1R,2S)-1-[(S)-1-[(S)-α-[(S)-α-[(tert-Butylsulfonyl)methyl]hydrocinnamamido]imidazol-4-propionamido]-2-cyclohexyläthyl]-2-cyclopropyläthylen diimidazol-4-carboxylat, MS : 875 $(M+H)^+$, und das (1S,2R,3S)-3-[(S)-α-[(S)-α-[(tert-Butylsulfonyl)methyl]hydrocinnamamido]imidazol-4-propionamido]-4-cyclohexyl-1-cyclopropyl-2-hydroxybutyl imidazol-4-propionat, MS : 753 $(M+H)^+$, jeweils als amorpher Festkörper.

Beispiel 46

Ein Gemisch von 150 mg (0.24 mMol) (S)-α-[(S)-α-[(tert-Butylsulfonyl)methyl]hydrocinnamamido]-N-[-(1S,2R,3S)-1-(cyclohexylmethyl)-3-cyclopropyl-2,3-dihydroxypropyl]imidazol-4-propionamid, 340 mg (1.2 mMol) 3-Phenylpropionsäureanhydrid und 40 mg (0.33 mMol) 4-Dimethylaminopyridin in 1.5 ml Pyridin wird 24 Stunden bei Raumtemperatur gerührt. Anschliessend wird das Reaktionsgemisch unter vermindertem Druck eingedampft, und der Rückstand zur Reinigung an 35 g Kieselgel unter Verwendung eines 200:10:1-Gemisches von Methylenchlorid, Methanol und Ammoniak als Eluierungsmittel chromatographiert. Man erhält 240 mg (1R,2S)-1-[(S)-1-[(S)-α-[(S)-α•[(tert-Butylsulfonyl)methyl]hydrocinnamamido]imidazol-4-propionamido]-2-cyclohexyläthyl)-2-cyclopropyläthylen dihydrocinnamat als farbloses Oel; MS : 895 $(M+H)^+$.

Beispiel 47

Eine Lösung von 200 mg (0,32 mMol) (S)-α-[(S)-α-[(tert-Butylsulfonyl)methyl]hydrocinnamamido]-N-[-(1S,2R,3S)-1-(cyclohexylmethyl)-3-cyclopropyl-2,3-dihydroxypropyl]imidazol-4-propionamid, 0,23 ml (1,6 mMol) Diäthylchlorophosphat und 50 mg Dimethylaminopyridin in 5 ml Pyridin wird 2 Tage bei Raumtemperatur stehengelassen. Das Reaktionsgemisch wird anschliessend unter vermindertem Druck zur Trockene eingedampft, und der Rückstand in Essigester aufgenommen. Die organische Phase wird zweimal mit gesättigter Natriumbicarbonat-Lösung gewaschen, über Natrium sulfat getrocknet und anschliessend eingedampft. Das Rohprodukt (340 mg) wird zur Reinigung an 50 g Kieselgel unter Verwendung eines 150:10:1-Gemisches von Methylenchlorid, Methanol und Ammoniak als Eluierungsmittel chromatographiert, wobei,neben Ausgangsmaterial, 35 mg (1S,2R,3S)-3-[(S)-α-[(S)-α-[(tert-Butylsulfonyl)methyl]-hydrocinnamamido]imidazol-4-propionamido]-4-cyclohexyl-1-cyclopropyl-2-hydroxybutyl diäthylphosphat als farbloser Schaum erhalten werden; MS : 767 $(M+H)^+$.

Beispiel 48

In analoger Weise wie in Beispiel 47 beschrieben, wurde durch Umsetzung von (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid mit Essigsäureanhydrid das (1R,2S)-1-[(S)-1-Acetyl-α-(S)-α-[(tert-butylsulfonyl)methyl]imidazol-4-propionamido]-2-cyclohexyläthyl]-2-cyclopropyläthylen diacetat als farbloser Festkörper erhalten; MS : 757 $(M+H)^+$.

Beispiel 49

In analoger Weise wie in Beispiel 16 beschrieben, wurden die folgenden Verbindungen hergestellt:
- Aus (S)-α-Amino-N-[(1S,2R,3S)-1-(cyclohexylmethyl)-3-cyclopropyl-2,3-dihydroxypropyl]-1-tritylimidazol-4-propionamid und 5-Chlorindol-2-carbonsäure das 5-Chlor-N-[(S)-1-[[(1S,2R,3S)-1-(cyclohexylmethyl)-3-cyclopropyl-2,3-dihydroxypropyl]carbamoyl]-2-(1-tritylimidazol)-4-yläthyl]indol-2-carboxamid;
- aus (S)-α-Amino-N-[(1S,2R,3S)-1-(cyclohexylmethyl)-3-cyclopropyl-2,3-dihydroxypropyl]-1-tritylimidazol-4-propionamid und 5-Fluorindol-2-carbonsäure das N-[(S)-1-[[(1S,2R,3S)-1-(Cyclohexylmethyl)-3-cyclopropyl-2,3-dihydroxypropyl]carbamoyl]-2-(1-tritylimidazol)-4-yläthyl]-5-fluorindol-2-carboxamid;
- aus (S)-α-Amino-N-[(1S,2R,3S)-1-(cyclohexylmethyl)-3-cyclopropyl-2,3-dihydroxypropyl]-1-tritylimidazol-4-propionamid und 5-Methylindol-2-carbonsäure das N-[(S)-1-[[(1S,2R,3S)-1-(Cyclohexylmethyl)-3-cyclopropyl-2,3-dihydroxypropyl]carbamoyl]-2-(1-tritylimidazol)-4-yläthyl]-5-methylindol-2-carboxamid;

- aus (S)-α-Amino-N-[(1S,2R,3S)-1-(cyclohexylmethyl)-3-cyclopropyl-2,3-dihydroxypropyl]-1-tritylimidazol-4-propionamid und 5-Methoxyindol-2-carbonsäure das N-[(S)-1-[[(1S,2R,3S)-1-(Cyclohexylmethyl)-3-cyclopropyl-2,3-dihydroxypropyl]carbamoyl]-2-(1-tritylimidazol)-4-yläthyl]-5-methoxyindol-2-carboxamid;
- aus (S)-α-Amino-N-[(1S,2R,3S)-1-(cyclohexylmethyl)-3-cyclopropyl-2,3-dihydroxypropyl]-1-tritylimidazol-4-propionamid und 3-Chlorindol-2-carbonsäure (J.Med.Chem. 1972, 659) das 3-Chlor-N-[(S)-1-[[(1S,2R,3S)-1-(cyclohexylmethyl)-3-cyclopropyl-2,3-dihydroxypropyl]carbamoyl]-2-(1-tritylimidazol)-4-yläthyl]indol-2-carboxamid;
- aus (S)-α-Amino-N-[(1S,2R,3S)-1-(cyclohexylmethyl)-3-cyclopropyl-2,3-dihydroxypropyl]-1-tritylimidazol-4-propionamid und 3,6-Dichlorindol-2-carbonsäure das 3,6-Dichlor-N-[(S)-1-[[(1S,2R,3S)-1-(cyclohexylmethyl)-3-cyclopropyl-2,3-dihydroxypropyl]carbamoyl]-2-(1-tritylimidazol)-4-yläthyl]indol-2-carboxamid;
- aus (S)-α-Amino-N-[(1S,2R,3S)-1-(cyclohexylmethyl)-3-cyclopropyl-2,3-dihydroxypropyl]-1-tritylimidazol-4-propionamid und Chinolin-2-carbonsäure das N-[(S)-1-[[(1S,2R,3S)-1-(Cyclohexylmethyl)-3-cyclopropyl-2,3-dihydroxypropyl]carbamoyl]-2-(1-tritylimidazol)-4-yläthyl]-2-chinolincarboxamid;
- aus (S)-α-Amino-N-[(1S,2R,3S)-1-(cyclohexylmethyl)-3-cyclopropyl-2,3-dihydroxypropyl]-1-tritylimidazol-4-propionamid und 1,4-Benzodioxan-2-carbonsäure (Chim.Ther.1984, 411) das (S)-α-[(RS)-1,4-Benzodioxan-2-carboxamido]-N-[(1S,2R,3S)-1-(cyclohexylmethyl)-3-cyclopropyl-2,3-dihydroxypropyl]-1-tritylimidazol-4-propionamid;
- aus (S)-α-Amino-N-[(1S,2R,3S)-1-(cyclohexylmethyl)-3-cyclopropyl-2,3-dihydroxypropyl]-1-tritylimidazol-4-propionamid und (RS)-Boc-(3-pyridyl)alanin (Pharmacology 1969, 271) das tert-Butyl [(RS)-1-[[(S)-1-[[(1S,2R,3S)-1-(cyclohexylmethyl)-3-cyclopropyl-2,3-dihydroxypropyl]-carbamoyl]-2-(1-tritylimidazol)-4-yläthyl]carbamoyl]-2-(3-pyridyl)äthyl]carbamat.

Die als Ausgangsstoff eingesetzte 3,6-Dichlorindol-2-carbonsäure wurde analog zu dem in J.Med.Chem. 1972, 659 zur Herstellung der 3-Chlorindol-2-carbonsäure beschriebenen Verfahren hergestellt.

## Beispiel 50

In analoger Weise wie in Beispiel 16 beschrieben, wurden die folgenden Verbindungen hergestellt:

- Aus (R)-2-Amino-N-[(1S,2R,3S)-1-(cyclohexylmethyl)-3-cyclopropyl-2,3-dihydroxypropyl]-3-(methylthio)-propionamid und N-[3-[1-(tert-Butoxy)formamido]-3-methylbutyryl]-3-(p-methoxyphenyl)-L-alanin das tert-Butyl [2-[[(S)-α- [[(R)-1-[[(1S,2R,3S)-1-(cyclohexylmethyl)-3-cyclopropyl-2,3-dihydroxypropyl]carbamoyl-2-(methylthio)äthyl]carbamoyl]-p-methoxyphenäthyl]carbamoyl]-1,1-dimethyl]carbamat als amorpher Festkörper, MS : 621 $(M+H\text{-}Boc)^+$;
- aus (S)-α-Amino-N-[(1S,2R,3S)-1-(cyclohexylmethyl)-3-cyclopropyl-2,3-dihydroxypropyl]-4-thiazolpropionamid (Herstellung siehe Beispiel 18) und N-[3-[1-(tert-Butoxy)formamido]-3-methylbutyryl]-3-(p-methoxyphenyl)-L-alanin das tert-Butyl [2-[[(S)-α-[[(S)-1-[[(1S,2R,3S)-1-(cyclohexylmethyl)-3-cyclopropyl-2,3-dihydroxypropyl]carbamoyl]-2-(5-thiazolyl)äthyl]carbamoyl]-p-methoxyphenäthyl]carbamoyl]-1,1-dimethyläthyl]carbamat als farbloser Schaum, MS : 758 $(M+H)^+$;
- aus (S)-2-Amino-$N^1$-[(1S,2R,3S)-1-(cyclohexylmethyl)-3-cyclopropyl-2,3-dihydroxypropyl]-bernsteinsäureamid und (S)-α-[(tert-Butylsulfonyl)methyl]hydroximtsäure das (S)-2-[(S)-α-[(tert-Butylsulfonyl)methyl]hydrocinnamamido]-$N^1$-[(1S,2R,3S)-1-(cyclohexylmethyl)-3-cyclopropyl-2,3-dihydroxypropyl]-bernsteinsäureamid als farbloser Schaum, MS: 608 $(M+H)^+$.

Das als Ausgangsstoff eingesetzte (R)-2-Amino-N-[(1S,2R,3S)-1-(cyclohexylmethyl)-3-cyclopropyl-2,3-dihydroxypropyl]-3-(methylthio)propionamid wurde wie folgt hergestellt:

In analoger Weise wie in Beispiel 18a) und b) beschrieben, wurde durch Kondensation von (R)-Boc-(S-methyl)cystein (J.Chem.Soc. C 1967, 2632) und (1S,2R,3S)-3-Amino-4-cyclohexyl-1-cyclopropyl-1,2-butandiol das tert-Butyl [(R)-1-[[(1S,2R,3S)-1-(cyclohexylmethyl)-3-cyclopropyl-2,3-dihydroxypropyl]carbamoyl]-2-(methylthio)äthyl]carbamat als amorpher Festkörper erhalten, MS : 445 $(M+H)^+$. Die nachfolgende Abspaltung der Boc-Schutzgruppe mittels Salzsäure in Methanol lieferte das (R)-2-Amino-N-[(1S,2R,3S)-1-(cyclohexylmethyl)-3-cyclopropyl-2,3-dihydroxypropyl]-3-(methylthio)propionamid.

Das als Ausgangsstoff eingesetzte N-[3-[1-(tert-Butoxy)formamido]-3-methylbutyryl]-3-(p-methoxyphenyl)-L-alanin wurde wie folgt hergestellt:

In analoger Weise zu dem in Beispiel 20 zur Herstellung von N-[3-[1-(Benzyloxy)formamido]-3-methylbutyryl]-3-(p-methoxyphenyl)-L-alanin beschriebenen Verfahren, wurde der N-[3-[1-(tert-Butoxy)-formamido]-3-methylbutyryl]-3-(p-methoxyphenyl)-L-alaninmethylester erhalten, indem bei der Kondensationsreaktion anstelle von N-[(Benzyloxy)carbonyl]-2,3-dimethyl-β-alanin (EPA 0230266) das N-[(tert-Butoxy)carbonyl]-2,3-dimethyl-β-alanin eingesetzt wurde, das seinerseits in analoger Weise, wie in EPA 0230266 für das N-[(Benzyloxy)carbonyl]-2,3-dimethyl-β-alanin beschrieben, durch Ersatz des Benzylalkohols durch tert-Butanol erhalten wurde. Die anschliessende basische Hydrolyse ergab das N-[3-[1-(tert-Butoxy)formamido]-3-methylbutyryl]-3-(p-methoxyphenyl)-L-alanin als amorpher Festkörper.

Das als Ausgangsstoff eingesetzte (S)-2-Amino-N[1]-[(1S,2R,3S)-1-(cyclohexylmethyl)-3-cyclopropyl-2,3-dihydroxypropyl]bernsteinsäureamid wurde wie folgt hergestellt:

In analoger Weise wie in Beispiel 18a) beschrieben, wurde durch Kondensation von N-α-Fmoc-asparagin und (1S,2R,3S)-3-Amino-4-cyclohexyl-1-cyclopropyl-1,2-butandiol und nachfolgende Abspaltung der Fmoc-Schutzgruppe mittels Piperidin in Methylenchlorid das (S)-2-Amino-N[1]-[(1S,2R,3S)-1-(cyclohexylmethyl)-3-cyclopropyl-2,3-dihydroxypropyl]bernsteinsäureamid als farbloser Festkörper erhalten, MS : 342 $(M+H)^+$.

## Beispiel 51

In analoger Weise wie in Beispiel 19 beschrieben, wurden die folgenden Verbindungen hergestellt:

- Aus (S)-α-Amino-N-[(1S,2R,3S)-1-(cyclohexylmethyl)-3-cyclopropyl-2,3-dihydroxypropyl]imidazol-4-propionamid und N-(4-Morpholinylcarbonyl)-L-phenylalanin (J.Med.Chem. 1988,31,2277) das (S)-N-[(1S,2R,3S)-1-(Cyclohexylmethyl)-3-cyclopropyl-2,3-dihydroxypropyl]-α-[(S)-α-(4-morpholincarboxamido)-hydrocinnamamido]imidazol-4-propionamid als amorpher Festkörper, MS : 625 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und N-[3-[1-(Benzyloxy)formamido]-3-methylbutyryl]-3-(p-methoxyphenyl)-L-alanin das Benzyl [2-[[-(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]-p-methoxyphenäthyl]carbamoyl]-1,1-dimethyläthyl]carbamat als farbloser Schaum, MS : 775 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und N-[3-[1-(Benzyloxy)formamido]propionyl]-3-(p-methoxyphenyl)-L-alanin das Benzyl [2-[[(S)-α-[[-(S)-1-[[(1S,2R,3S)-1-(cyclohexylmethyl)-3-cyclopropyl-2,3-dihydroxypropyl]carbamoyl]-2-imidazol-4-yläthyl]-carbamoyl]-p-methoxyphenäthyl]carbamoyl]äthyl]carbamat als farbloser Festkörper, MS : 747 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und N-[6-[-1-(Benzyloxy)formamido]hexanoyl]-3-(p-methoxyphenyl)-L-alanin das Benzyl [5-[[(S)-α-[[-(S)-1-[[(1S,2R, 3S)-1-(cyclohexylmethyl)-3-cyclopropyl-2,3-dihydroxypropyl]carbamoyl]-2-imidazol-4-yläthyl]-carbamoyl]-p-methoxyphenäthyl]carbamoyl]pentyl]carbamat als farbloser Festkörper, MS: 789 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und N-[[p-[1-(Benzyloxy)formamido]phenyl]acetyl]-3-(p-methoxyphenyl)-L-alanin der p-[[[(S)-α-[[(S)-1-[[(1S,2R,3S)-1-(Cyclohexylmethyl)-3-cyclopropyl-2,3-dihydroxypropyl]carbamoyl]-2-imidazol-4-yläthyl]-carbamoyl]-p-methoxyphenäthyl]carbamoyl]methyl]carbanilsäurebenzylester als farbloser Festkörper, MS : 809 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und N-[[cis/trans-4-[1-(Benzyloxy)formamido]cyclohexyl]acetyl]-3-(p-methoxyphenyl)-L-alanin das Benzyl cis/trans-4-[[[(S)-α-1-[[(1S,2R,3S)-1-(cyclohexylmethyl)-3-cyclopropyl-2,3-dihydroxypropyl]-carbamoyl-2-imidazol-4-yläthyl]carbamoyl]-p-methoxyphenäthyl]carbamoyl]methyl]cyclohexancarbamat als amorpher Festkörper, MS : 815 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und N-[(S)-4-[1-(Benzyloxy)formamido]-2-hydroxybutyryl]-3-(p-methoxyphenyl)-L-alanin das Benzyl [-(S)-3-[[(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]-p-methoxyphenäthyl]carbamoyl]-2-hydroxyäthyl]carbamat als farbloser Schaum, MS : 777 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und N-(3-Hydroxy-3-methylbutyryl)-3-(p-methoxyphenyl]-L-alanin das (S)-N-[(1S,2R,3S)-1-(Cyclohexylmethyl)-3-cyclopropyl-2,3-dihydroxypropyl]-α-[(S)-α-(3-hydroxy-3- methylbutyramido)-p-methoxyhydrocinnamamido]imidazol-4-propionamid als farbloser Schaum, MS : 642 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und 3-(p-Methoxyphenyl)-N-[3-methyl-3-(4-morpholincarboxamido)-3-methylbutyryl]-L-alanin das N-[1-[[(S)-α-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-p-methoxyphenäthyl]carbamoyl]-1-methyläthyl]-4-morpholincarboxamid als farbloser Schaum, MS : 754 $(M+H)^+$;

- aus (S)-α-Amino-N-[(1S,2R,3S)-1-(cyclohexylmethyl)-3-cyclopropyl-2,3-dihydroxypropyl]imidazol-4-propionamid und N-[[1-[1-(Benzyloxy)formamido]cyclopropyl]acetyl]-3-(p-methoxyphenyl)-L-alanin das Benzyl [1-[-[[(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]-p-methoxyphenäthyl]carbamoyl[methyl]cyclopropyl]carbamat als farbloser Festkörper, MS : 773 $(M+H)^+$;

- aus (S)-α-Amino-N-[(1S,2R,3S)-1-(cyclohexylmethyl)-3-cyclopropyl-2,3-dihydroxypropyl]imidazol-4-propionamid und N-[(RS)-3-Chinuclidinyl]-3-phenyl-L-alanin (Peptides, Proc.11th Am.Pept.Sym. 1989, Herausgeb. J.E.Rivier und G.R.Marshall, S.411) das (S)-N-[(1S,2R,3S)-1-(Cyclohexylmethyl)-3-cyclopropyl-2,3-dihydroxypropyl]-α-[(S)-[[(RS)-3-chinuclidinyl]amino]hydrocinnamamido]imidazol-4-propionamid als farbloser Festkörper, MS : 621 $(M+H)^+$;

- aus (S)-α-Amino-N-[(1S,2R,3S)-1-(cyclohexylmethyl)-3-cyclopropyl-2,3-dihydroxypropyl]imidazol-4-propionamid und (RS)-α-[4-[1-(Benzyloxy)formamido]-4-methyl-2-oxopentyl]hydrozimtsäure das Benzyl [(R oder S)-5-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]-1,1-dimethyl-3-oxo-6-phenylhexyl]carbamat, MS : 744 $(M+H)^+$ und sein polareres Epimer Benzyl [(S oder R)-5-[[(S)-1-[[(1S,2R,3S)-1-(cyclohexylmethyl)-3-cyclopropyl-2,3-dihydroxypropyl]-carbamoyl]-2-imidazol-4-yläthyl]carbamoyl]-1,1-dimethyl-3-oxo-6-phenylhexyl]carbamat, MS : 744 $(M+H)^+$, jeweils als farbloser Festkörper.

Die als Ausgangsstoff eingesetzte (RS)-α-[4-[1-(Benzyloxy)formamido]-4-methyl-2-oxopentyl]-hydrozimtsäure wurde wie folgt hergestellt:

Analog zu der in J.Med.Chem. 32,1378 (1989) beschriebenen, allgemeinen Methode zur Herstellung N-geschützter Dipeptid-Ketoisosterer wurde aus N-[(Benzyloxy)carbonyl]-3,3-dimethyl-β-alanin (EPA 0230266) und Benzylmalonsäurediäthylester die (RS)-α-[4-[1-(Benzyloxy)formamido]-4-methyl-2-oxopentyl]-hydrozimtsäure als farbloser Schaum erhalten, MS : 398 $(M+H)^+$.

Die als Ausgangsstoffe eingesetzten 3-(p-Methoxyphenyl)-L-alanin-Derivate wurden wie folgt hergestellt:

N-[3-[1-(Benzyloxy)formamido]-3-methylbutyryl]-3-(p-methoxyphenyl)-L-alanin

(a) N-[3-[1-(Benzyloxy)formamido]-3-methylbutyryl]-3-(p-methoxyphenyl)-L-alaninmethylester

Eine Suspension von 1.12 g (4.47 mMol) N-[(Benzyloxy)carbonyl]-2,3-dimethyl-β-alanin (EPA 0230266), 1.1 g (4.47 mMol) 3-(p-Methoxyphenyl)-L-alaninmethylester-hydrochlorid, 1.7 g (4.47 mMol) HBTU und 1.25 ml (8.94 mMol) Triäthylamin in 40 ml Acetonitril wird 4 Stunden lang bei Raumtemperatur gerührt. Nach Zugabe von 50 ml gesättigter Natriumchloridlösung wird dreimal mit je 50 ml Essigester extrahiert. Die vereinigten organischen Phasen werden in der Folge mit 2N Salzsäure, Wasser, 50%iger Natriumhydrogencarbonatlösung und Wasser gewaschen, über Natriumsulfat getrocknet und unter vermindertem Druck eingedampft. Der Rückstand wird zur Reinigung an Kieselgel unter Verwendung eines 95:5-Gemisches von Methylenchlorid und Methanol als Eluierungsmittel chromatographiert. N-[3-[1-(Benzyloxy)formamido]-3-methylbutyryl]-3-(p-methoxyphenyl)-L-alaninmethylester wird als farbloses Öl erhalten; MS : 443 $(M+H)^+$.

(b) N-[3-[1-(Benzyloxy)formamido]-3-methylbutyryl]-3-(p-methoxyphenyl)-L-alanin

Zu einer Lösung von 1.7 g (3.84 mMol) N-[3-[1-(Benzyloxy)formamido]-3-methylbutyryl]-3-(p-methoxyphenyl)-L-alaninmethylester in 16 ml Dioxan werden 0.184 g (4.39 mMol) Lithiumhydroxid-hydrat, gelöst in 8 ml Wasser, getropft. Die Lösung wird 1 Stunde lang bei 0° gerührt, dann mit 20 ml Wasser versetzt und zweimal mit Äther extrahiert. Die wässerige Phase wird mit 6N Salzsäure sauer gestellt und zweimal mit Äther extrahiert. Die vereinigten Ätherextrakte werden mit gesättigter Natriumchloridlösung gewaschen, über Natriumsulfat getrocknet und dann unter vermindertem Druck eingedampft. N-[3-[1-(Benzyloxy)-formamido]-3-methylbutyryl]-3-(p-methoxyphenyl)-L-alanin wird als farbloses Öl erhalten, MS : 429 $(M+H)^+$.

In analoger Weise wie für die Herstellung von N-[3-[1-(Benzyloxy)formamido]-3-methylbutyryl]-3-(p-methoxyphenyl)-L-alanin beschrieben, wurden die folgenden Verbindungen hergestellt:

N-[3-[1-(Benzyloxy)formamido]propionyl]-3-(p-methoxyphenyl)-L-alanin als farbloser Festkörper, MS : 401 $(M+H)^+$, durch Kondensation von N-Benzyloxycarbonyl-β-alanin und 3-(p-Methoxyphenyl)-L-alaninmethylester-hydrochlorid und anschliessende basische Hydrolyse;

N-[6-[-1-(Benzyloxy)formamido]hexanoyl]-3-(p-methoxyphenyl)-L-alanin als farbloser Festkörper, MS : 443 $(M+H)^+$, durch Kondensation von 6-[1-(Benzyloxy)formamido)hexansäure (EPA 0229667) und 3-(p-Methoxyphenyl)-L-alaninmethylesterhydrochlorid und anschliessende basische Hydrolyse;

N-[[p-[1-(Benzyloxy)formamido]phenyl]acetyl]-3-(p-methoxyphenyl)-L-alanin als farbloser Festkörper, MS : 463 $(M+H)^+$, durch Kondensation von [p-[1-(Benzyloxy)formamido]phenyl]essigsäure [Chem.Ber. 101, 1223 (1968)] und 3-(p-Methoxyphenyl)-L-alaninmethylester-hydrochlorid und anschliessende basische Hydrolyse;

N-[[cis/trans-4-[1-(Benzyloxy)formamido]cyclohexyl]acetyl]-3-p-methoxyphenyl)-L-alanin als farbloser Festkörper, MS : 469 $(M+H)^+$, durch Kondensation von cis/trans-4-[1-(Benzyloxy)formamido]-cyclohexanessigsäure (Izv. Akad. Nauk. SSSR, Ser. Khim. 1980, 1426) und 3-(p-Methoxyphenyl)-L-alaninmethylester-hydrochlorid und anschliessende basische Hydrolyse;

N-[(S)-4-[1-(Benzyloxy)formamido]-2-hydroxybutyryl]-3-(p-methoxyphenyl)-L-alanin als farbloser Festkörper, MS : 431 $(M+H)^+$, durch Kondensation von (S)-4-[1-(Benzyloxy)formamido]-2-hydroxybuttersäure [Carbohydrate Res. 28, 263 (1973)] und 3-(p-Methoxyphenyl)-L-alaninmethylester-hydrochlorid und anschliessende basische Hydrolyse;

N-(3-Hydroxy-3-methylbutyryl)-3-(p-methoxyphenyl]-L-alanin als farbloses Öl, MS : 296 $(M+H)^+$, durch Kondensation von β-Hydroxyvaleriansäure und 3-(p-Methoxyphenyl)-L-alaninmethylester-hydrochlorid und anschliessende basische Hydrolyse;

3-(p-Methoxyphenyl)-N-[3-methyl-3-(4-morpholincarboxamido)-3-methylbutyryl]-L-alanin als farbloser Schaum, MS : 408 $(M+H)^+$, durch Kondensation von 3-(4-Morpholincarboxa mido)-3-methylbuttersäure und 3-(p-Methoxyphenyl)-L-alaninmethylester-hydrochlorid und anschliessende basische Hydrolyse.

N-[[1-[1-(Benzyloxy)formamido]cyclopropyl]acetyl]-3-(p-methoxyphenyl)-L-alanin als farbloser Festkörper, MS : 427 $(M+H)^+$, durch Kondensation von 1-[1-(Benzyloxy)formamido]cyclopropanessigsäure und 3-(p-Methoxyphenyl)-L-alaninmethylester-hydrochlorid und anschliessende basische Hydrolyse.

Die als Ausgangsstoff eingesetzte 3-(4-Morpholincarboxamido)-3-methylbuttersäure wurde analog zu dem in EPA 0230266 beschriebenen Verfahren zur Herstellung von N-[(Benzyloxy)carbonyl]-3,3-dimethyl-β-alanin synthetisiert, indem anstelle von Benzylalkohol Morpholin eingesetzt wurde.

Die als Ausgangsstoff eingesetzte 1-[1-(Benzyloxy)formamido]cyclopropanessigsäure wurde analog zu dem in EPA 0230266 beschriebenen Verfahren zur Herstellung von N-[(Benzyloxy)carbonyl]-3,3-dimethyl-β-alanin synthetisiert, indem anstelle der 2,2-Dimethylbernsteinsäure die 1-Carboxycyclopropanessigsäure (Bull.Soc. Chim.Fr. 1971, 2290) eingesetzt wurde.

## Beispiel 52

In analoger Weise wie in Beispiel 21 beschrieben, wurde durch Ersatz von Boc-D-prolin durch N-Benzyloxycarbonyl-D-alanin in der Kondensationsreaktion mit (S)-N-[(1S,2R,3S)-1-(Cyclohexylmethyl)-3-cyclopropyl-2,3-dihydroxypropyl]-α-[(3-phenyl-L-alanyl)amino]imidazol-4-propionamid das Benzyl [(R)-1-[[(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]phenäthyl]carbamoyl]äthyl]carbamat als amorpher Festkörper erhalten, MS : 717 $(M+H)^+$.

## Beispiel 53

In analoger Weise wie in Beispiel 19 beschrieben, wurde durch Kondensation von (S)-α-Amino-N-[(1S,2R,3S)-1-(cyclohexylmethyl)-3-cyclopropyl-2,3-dihydroxypropyl]imidazol-4-propionamid und N-Boc-p-methoxy-L-phenylalanin das tert-Butyl [(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]-p-methoxyphenäthyl]carbamat als farbloser Festkörper, MS : 642 $(M+H)^+$, erhalten. Die nachfolgende Abspaltung der Boc-Schutzgruppe mittels Salzsäure in Methanol, analog Beispiel 18b), lieferte (S)-α-[(S)-α-Amino-p-methoxyhydrocinnamamido]-N-[(1S,2R,3S)-1-(cyclohexylmethyl)-3-cyclopropyl-2,3-dihydroxypropyl]imidazol-4-propionamid-hydrochlorid als farblosen Festkörper, MS : 542 $(M+H)^+$. Die weitere Kondensation mit N-Benzyloxycarbonyl-L-adipinsäure-α-methylester [J.Org.Chem. 52, 5342 (1987)] analog Beispiel 19, ergab das Benzyl [(S)-4-[[(S)-α-[[(S)-1-[[(1S,2R,3S)-1-(cyclohexylmethyl-3-cyclopropyl-2,3-dihydroxypropyl]carbamoyl]-2-imidazol-4-yläthyl]-carbamoyl]-p-methoxyphenäthyl]carbamoyl]-1-(methoxycarbonyl)butyl]carbamat als farblosen Festkörper, MS : 833 $(M)^+$.

Beispiel 54

In analoger Weise wie in Beispiel 1 beschrieben, wurden die folgenden Verbindungen hergestellt:
- Aus (S)-α-Amino-N-[(1S,2R,3S)-1-(cyclohexylmethyl)-3-cyclopropyl-2,3-dihydroxypropyl]imidazol-4-propionamid und (S)-α-[(Phenylsulfonyl)methyl]hydrozimtsäure (EPA 0236734) das (S)-N-[(1S,2R,3S)-1-(Cyclohexylmethyl)-3-cyclopropyl-2,3-dihydroxypropyl]-α-[(S)-α-[(phenylsulfonyl)methyl]hydrocinnamamido]-imidazol-4-propionamid als amorpher Festkörper, MS : 651 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und (S)-α-[tert-Butylsulfonyl)methyl]-3-thiophenpropionsäure das (S)-α-[(S)-α-[(tert-Butylsulfonyl)-methyl]-3-thiophenpropionamido]-N-[(1S,2R,3S)-1-(cyclohexylmethyl)-3-cyclopropyl-2,3-dihydroxypropyl]-imidazol-4-propionamid als farbloser Schaum, MS : 637 $(M+H)^+$;
- aus (S)-α-Amino-N-[(1S,2R,3S)-1-(cyclohexylmethyl)-3-cyclopropyl-2,3-dihydroxypropyl]imidazol-4-propionamid und (S)-α-[[(Cyclopropylmethyl)sulfonyl]methyl]hydrozimtsäure das (S)-N-[(1S,2R,3S)-1-(Cyclohexylmethyl)-3-cyclopropyl-2,3-dihydroxypropyl]-α-[(S)-α[[cyclopropylmethyl)-sulfonyl]methyl]-hydrocinnamamido]imidazol-4-propionamid als farbloser Schaum, MS : 629 $(M+H)^+$;
- aus (R)-2-Amino-N-[(1S,2R,3S)-1-(cyclohexylmethyl)-3-cyclopropyl-2,3-dihydroxypropyl]-3-(methylthio)-propionamid und (S)-α-[(tert-Butylsulfonyl)methyl]hydrozimtsäure (EPA 0236734) das (S)-α-[(tert-Butylsulfonyl)methyl]-N-[(R)-1-[[(1S,2R,3S)-1-(cyclohexylmethyl)-3-cyclopropyl-2,3-dihydroxypropyl]carbamoyl]-2-(methylthio)äthyl]hydrozimtsäureamid als amorpher Festkörper, MS : 611 $(M+H)^+$.

Das als Ausgangsstoff eingesetzte (R)-2-Amino-N-[(1S,2R,3S)-1-(cyclohexylmethyl)-3-cyclopropyl-2,3-dihydroxypropyl]-3-(methylthio)propionamid wurde wie folgt hergestellt:

In analoger Weise wie in Beispiel 1g) beschrieben, wurde durch Kondensation von (R)-Boc-(S-methyl)-cystein (J.Chem.Soc. C 1967, 2632) und (1S,2R,3S)-3-Amino-4-cyclohexyl-1-cyclopropyl-1,2-butandiol das tert-Butyl [(R)-1-[[(1S,2R,3S)-1-(cyclohexylmethyl)-3-cyclopropyl-2,3-dihydroxypropyl]carbamoyl]-2-(methylthio)äthyl]carbamat als amorpher Festkörper erhalten, MS : 445 $(M+H)^+$. Die nachfolgende Abspaltung der Boc-Schutzgruppe mittels Salzsäure in Methanol lieferte das (R)-2-Amino-N-[(1S,2R,3S)-1-(cyclohexylmethyl)-3-cyclopropyl-2,3-dihydroxypropyl]-3-(methylthio)propionamid.

Die als Ausgangsstoffe eingesetzten Säurederivate wurden wie folgt hergestellt:

(S)-α-[(tert-Butylsulfonyl)methyl]-3-thiophenpropionsäure

Diese Verbindung wurde analog Beispiel 1 durch enzymatische Hydrolyse des (RS)-α-[(tert-Butylsulfonyl)methyl]-3-thiophenpropionsäureäthylesters hergestellt, der seinerseits, ausgehend von 3-Thienylmethylmalonsäurediäthylester [J.Am.Chem.Soc. 76 , 4466 (1954)], analog der in EPA 0236734 beschriebenen Synthese von (RS)-α-[(tert-Butylsulfonyl)methyl]hydrozimtsäureäthylester hergestellt wurde, MS : 318 $(M)^+$.

S-α-[[(Cyclopropylmethyl)sulfonyl]methyl]hydrozimtsäure

Diese Verbindung wurde analog Beispiel 1d) durch enzymatische Hydrolyse des (RS)-α-[[(Cyclopropylmethyl)sulfonyl]methyl]hydrozimtsäureäthylesters hergestellt, der seinerseits, analog der in EPA 0236734 beschriebenen Synthese von (RS)-α-[(tert-Butylsulfonyl)methyl]hydrozimtsäureäthylester, hergestellt wurde, indem das tert-Butylthiol durch das Cyclopropylmethylthiol ersetzt wurde, MS : 282 $(M)^+$.

Beispiel 55

In analoger Weise wie in Beispiel 4 beschrieben, wurden die folgenden Verbindungen hergestellt:
- Aus (S)-1-(tert-Butoxycarbonyl)-α-[(S)-α-[(tert-butylsulfonyl)methyl]hydrocinnamamido]imidazol-4-propionsäure und rac-ribo-3-Amino-1-cyclopropyl-4-[(cyclopropylmethyl)thio]- 1,2-butandiol das (S)-α-[(S)-α-(tert-Butylsulfonyl)methyl]hydrocinnamamido]-N-[(1R,2R,3S)-3-cyclopropyl-1-[[(cyclopropylmethyl)thio]methyl]-2,3-dihydroxypropyl]-1-Boc-imidazol-4-propionamid und das (S)-α-[(S)-α-[(tert-Butylsulfonyl)methyl]-hydrocinnamamido]-N-[(1S,2S,3R)-3-cyclopropyl-1-[[(cyclopropylmethyl)thio]methyl]-2,3-dihydroxypropyl]-1-Boc-imidazol-4-propionamid.

Das als Ausgangsstoff eingesetzte rac-ribo-3-Amino-1-cyclopropyl-4-[(cyclopropylmethyl)thio]-1,2-butandiol wurde analog zu dem Verfahren hergestellt, das in WO 87/05302 zur Synthese des tert-Butyl (4S,5R)-4-(cyclohexylmethyl)-5-formyl-2,2-dimethyl-3-oxazolidincarboxylats beschrieben ist, ausgehend vom Boc-cyclohexylalaninmethylester, gefolgt von der Grignard-Reaktion mit Cyclopropylmagnesiumbromid und Abspaltung der Boc- und Isopropyliden-Schutzgruppe analog Beispiel 1f). Der Ersatz des Boc-cyclohexylalaninmethylesters ergab, ausgehend von tert-Butyl rac[2-[(cyclopropylmethyl)thio]-1-(äthoxycarbonyl)äthyl]-carbamat das rac-ribo-3-Amino-1-cyclopropyl-4-[(cyclopropylmethyl)thio]-1,2-butandiol als farblosen Festkörper, MS : 232 $(M+H)^+$.

Das als Ausgangsstoff eingesetzte tert-Butyl rac-[2-[(cyclopropylmethyl)thio]-1-(äthoxycarbonyl)äthyl]-carbamat wurde wie folgt hergestellt:

Zu einer Suspension von 2.49 g (64,8 mMol) Natriumhydrid (60%ig in Öl) in 200 ml Dimethylformamid werden bei 0° 14.7 g (59 mMol) N-Boc-L-cysteinäthylester, gelöst in 300 ml Dimethylformamid, getropft und 30 Minuten bei 0° nachgerührt. Anschliessend werden 8.37 g (62 mMol) Cyclopropylmethylbromid in 30 ml Dimethylformamid zugetropft. Die Lösung wird über Nacht bei Raumtemperatur gerührt. Das Dimethylformamid wird unter vermindertem Druck abgedampft, der Rückstand zwischen Äther und Wasser verteilt, und die wässerige Phase viermal mit Äther extrahiert. Die vereinigten organischen Phasen werden je zweimal mit Wasser und gesättigter Natriumchloridlösung gewaschen, über Natriumsulfat getrocknet und unter vermindertem Druck eingedampft. Der Rückstand wird zur Reinigung an Kieselgel unter Verwendung eines 1:19-Gemisches von Essigester und Hexan als Eluierungsmittel chromatographiert. Es werden 8.4 g tert-Butyl rac-[2-[(cyclopropylmethyl)thio]-1-(äthoxycarbonyl)äthyl]carbamat als farbloses Öl erhalten, MS : 304 $(M+H)^+$.

## Beispiel 56

In analoger Weise wie in Beispiel 4 beschrieben, wurden folgende Verbindungen hergestellt:

- Aus (S)-1-(tert-Butoxycarbonyl)-α-[(S)-α-[(tert-butylsulfonyl)(methyl]hydrocinnamamido]imidazol-4-propionsäure und (1R oder S,2S oder R,3R,4S)-4-Amino-4-cyclohexyl-1-cyclopropyl-1,2,3-pentantriol das (S)-α-[(S)-α-[(tert-Butylsulfonyl)methyl]hydrocinnamamido]-N-[(1S,2R,3S oder R,4R oder S)-1-(cyclohexylmethyl)-4-cyclopropyl-2,3,4-trihydroxybutyl]-1-Boc-imidazol-4-propionamid;
- aus (S)-1-(tert-Butoxycarbonyl)-α-[(S)-α-[(tert-butylsulfonyl)methyl]hydrocinnamamido]imidazol-4-propionsäure und (1S oder R,2S oder R,3R,4S)-4-Amino-5-cyclohexyl-1-cyclopropyl-1,2,3-pentantriol das (S)-α-[(S)-α-[(tert-Butylsulfonyl)methyl]hydrocinnamamido]-N-[(1S,2R,3S oder R,4S oder R)-1-(cyclohexylmethyl)-4-cyclopropyl-2,3,4-trihydroxybutyl]-1-Boc-imidazol-4-propionamid;
- aus (S)-1-(tert-Butoxycarbonyl)-α-[(S)-α-[(tert-butylsulfonyl)methyl]hydrocinnamamido]imidazol-4-propionsäure und (1R oder S,2R oder S,3R,4S)-4-Amino-5-cyclohexyl-1-cyclopropyl-1,2,3-pentantriol das (S)-α-[(tert-Butylsulfonyl)methyl]hydrocinnamamido]-N-[(1S,2R,3R oder S,4R oder S)-1-(cyclohexylmethyl)-4-cyclopropyl-2,3,4- trihydroxybutyl]-1-Boc-imidazol-4-propionamid;
- aus (S)-1-(tert-Butoxycarbonyl)-α-[(tert-butylsulfonyl)methyl]hydrocinnamamido]imidazol-4-propionsäure und (1S oder R,2R oder S,3R,4S)-4-Amino-5-cyclohexyl-1-cyclopropyl-1,2,3-pentantriol das (S)-α-[(S)-α-[-(tert-Butylsulfonyl)methyl]hydrocinnamamido]-N-[(1S,2R,3R oder S,4S oder R)-1-(cyclohexylmethyl)-4-cyclopropyl-2,3,4-trihydroxybutyl]-1-Boc-imidazol-4-propionamid.

Die als Ausgangsstoffe eingesetzten 4-Amino-5-cyclohexyl-1-cyclopropyl-1,2,3-pentantriole wurden wie folgt hergestellt:

### (a) (4S,5R)-4-(Cyclohexylmethyl)-5-[(S oder R)-cyanohydroxymethyl]-2,2-dimethyl-3-oxazolidincarbonsäure-tert-butylester und (4S,5R)-4-(Cyclohexylmethyl)-5-[(R oder S)-cyanohydroxymethyl]-2,2-dimethyl-3-oxazolidincarbonsäure-tert-butylester

Zu einer Lösung von 325.4 mg (1 mMol) (4S,5R)-4-(Cyclohexylmethyl)-5-formyl-2,2-dimethyl-3-oxazolidincarbonsäure-tert-butylester (WO 87/05302) in 10 ml Tetrahydrofuran wird eine Lösung von 1.14 g (5 mMol) Tetraäthyl-orthotitanat in 5 ml Tetrahydrofuran gegeben. Nach 30-minütigem Rühren werden 20 mg Kaliumcyanid/18-Crown-6-Komplex, gefolgt von einer Lösung von 0.25 ml (2 mMol) Trimethylsilylcyanid in 5 ml Tetrahydrofuran zugefügt. Nach 60 Minuten ist die Reaktion beendet. Das Reaktionsgemisch wird auf gesättigte Ammoniumchloridlösung gegossen, dreimal mit Esssigester extrahiert, und die vereinigten organischen Phasen werden mit gesättigter Natriumchloridlösung gewaschen, über Magnesiumsulfat getrocknet und unter vermindertem Druck eingedampft. Das Rohprodukt wird zur Reinigung an 50 g Kieselgel

unter Verwendung eines 3:2-Gemisches von Hexan und Äther als Eluierungsmittel chromatographiert. Das erhaltene Material wird zweimal aus Äther/Hexan umkristallisiert. Dabei wird (4S,5R)-4-(Cyclohexylmethyl)-5-[(S oder R)-cyanohydroxymethyl]-2,2-dimethyl-3-oxazolidincarbonsäure-tert-butylester in Form farbloser Kristalle erhalten, MS : 353 $(M+H)^+$, während (4S,5R)-4-(Cyclohexylmethyl)-5-[(R oder S)-cyanohydroxymethyl]-2,2-dimethyl-3-oxazolidincarbonsäure tert-butylester in der Mutterlauge angereichert vorliegt.

(b) (4S,5R)-4-(Cyclohexylmethyl)-5-[(R oder S)-(cyclopropylcarbonyl)hydroxymethyl]-2,2-dimethyl-3-oxazolidincarbonsäure-tert-butylester und (4S,5R)-4-(Cylohexylmethyl)-5-[(S oder R)-(cyclopropylcarbonyl)-hydroxymethyl]-2,2-dimethyl-3-oxazolidincarbonsäure-tert-butylester

Zu einem auf üblichem Wege aus 3.3 ml (41.2 mMol) Cyclopropylbromid und 1.0 g Magnesium (41.2 Grammatom) in 35 ml Äther hergestellten Grignardreagens wird eine Lösung von 2.64 g (7.5 mMol) (4S,5R)-4-(Cyclohexylmethyl)-5-[(S oder R)-cyanohydroxymethyl]-2,2-dimethyl-3-oxazolidincarbonsäure-tert-butylester und (4S,5R)-4-(Cyclohexylmethyl)-5-[(R oder S)-cyanohydroxymethyl]-2,2-dimethyl-3-oxazolidincarbonsäure-tert-butylester in 30 ml Äther bei 40° innerhalb von 20 Minuten getropft. Nach Entfernen des Ölbades wird das Reaktionsgemisch 2 Stunden bei Raumtemperatur gerührt. Anschliessend wird bei 0° mit 35 ml wässeriger Zitronensäure angesäuert und 1 Stunde unter Eiskühlung gerührt. Danach wird das Reaktionsgemisch mit Äther extrahiert, die organische Phase mit 2N Natriumhydrogencarbonatlösung gewaschen, über Magnesiumsulfat getrocknet und unter vermindertem Druck eingedampft. Zur Reinigung wird der Rückstand an 450 g Kieselgel unter Verwendung eines 4:1- und 7:3-Gemisches von Hexan und Äther als Eluierungsmittel chromatographiert. Es werden 500 mg (4S,5R)-4-(Cyclohexylmethyl)-5-[(R oder S)-(cyclopropylcarbonyl)hydroxymethyl]-2,2-dimethyl-3-oxazolidincarbonsäure-tert-butylester als farbloser Festkörper, MS : 396 $(M+H)^+$, und 850 mg (4S,5R)-4-(Cyclohexylmethyl)-5-[(S oder R)-(cyclopropyl carbonyl)hydroxymethyl]-2,2-dimethyl-3-oxazolidincarbonsäure-tert-butylester als farbloser Schaum, MS : 396 $(M+H)^+$, erhalten.

(c) (4S,5R)-4-(Cyclohexylmethyl)-5-[(1S oder R, 2S oder R)-2-cyclopropyl-1,2-dihydroxyäthyl]-2,2-dimethyl-3-oxazolidincarbonsäure-tert-butylester und (4S,5R)-4-(Cylohexylmethyl)-5-[(1S oder R, 2R oder S)-2-cyclopropyl-1,2-dihydroxyäthyl]-2,2-dimethyl-3-oxazolidincarbonsäure-tert-butylester

Zu einer Lösung von 340 mg (0.86 mMol) (4S,5R)-4-(Cyclohexylmethyl)-5-[(R oder S)-(cyclopropylcarbonyl)hydroxymethyl]-2,2-dimethyl-3-oxazolidincarbonsäure-tert-butylester in 15 ml Methylenchlorid werden 0.3 ml (5.16 mMol) Essigsäure gegeben, das Gemisch auf 0° abgekühlt und mit 65 mg (1.72 mMol) Natriumborhydrid versetzt. Das Reaktionsgemisch wird 5 Stunden bei 0° gerührt. Danach werden tropfenweise 6.5 ml einer 2N Natriumhydrogencarbonatlösung zugegeben, und das Reaktionsgemisch in Methylenchlorid aufgenommen. Die organische Phase wird mit Wasser gewaschen, über Magnesiumsulfat getrocknet und unter vermindertem Druck eingedampft. Der Rückstand wird zur Reinigung an 60 g Kieselgel unter Verwendung eines 4:1-Gemisches von Toluol und Esssigester als Eluierunsmittel chromatographiert. Es werden 90 mg (4S,5R)-4-(Cyclohexylmethyl)-5-[(1S oder R, 2S oder R)-2-cyclopropyl-1,2-dihydroxyäthyl]-2,2-dimethyl-3-oxazolidincarbonsäure-tert-butylester als farbloser Festkörper, MS : 398 $(M+H)^+$, und 247 mg (4S,5R)-4-(Cyclohexylmethyl)-5-[(1S oder R, 2R oder S)-2-cyclopropyl-1,2-dihydroxyäthyl]-2,2-dimethyl-3-oxazolidincarbonsäure-tert-butylester als farbloser Festkörper, MS : 398 $(M+H)^+$, erhalten.

In analoger Weise wie oben beschrieben, wurden aus (4S,5R)-4-(Cyclohexylmethyl)-5-[(S oder R)-(cyclopropylcarbonyl)hydroxymethyl]-2,2-dimethyl-3-oxazolidincar bonsäure-tert-butylester (4S,5R)-4-(Cyclohexylmethyl)-5-[(1R oder S, 2S oder R)-2-cyclopropyl-1,2-dihydroxyäthyl]-2,2-dimethyl-3-oxazolidincarbonsäure-tert-butylester als farbloser Festkörper, MS : 398 $(M+H)^+$, und (4S,5R)-4-(Cyclohexylmethyl)-5-[(1R oder S, 2R oder S)-2-cyclopropyl-1,2-dihydroxyäthyl]-2,2-dimethyl-3-oxazolidincarbonsäure-tert-butylester als farbloser Festkörper, MS : 398 $(M+H)^+$, erhalten.

(d) (1R oder S,2S oder R,3R,4S)-4-Amino-5-cyclohexyl-1-cyclopropyl-1,2,3-pentantriol

90 mg (0.2 mMol) (4S,5R)-4-(Cyclohexylmethyl)-5-[(1S oder R,2R oder S)-2-cyclopropyl-1,2-dihydroxyäthyl]-2,2-dimethyl-3-oxazolidincarbonsäure-tert-butylester werden in 5 ml 0.3N methanolischer

Salzsäure 3 Tage bei Raumtemperatur stehen gelassen. Danach wird das Gemisch unter vermindertem Druck zur Trockene eingedampft. Der Rückstand wird zur Reinigung an 25 g Kieselgel unter Verwendung eines 65:10:1-Gemisches von Methylenchlorid, Methanol und Ammoniak als Eluierungsmittel chromatographiert. Es werden 46 mg (1R oder S,2S oder R,3R,4S)-4-Amino-5-cyclohexyl-1-cyclopropyl-1,2,3-pentantriol als farbloser Festkörper erhalten, MS : 258 $(M+H)^+$.

In analoger Weise wurden die folgenden Amine hergestellt:

- Aus (4S,5R)-4-(Cyclohexylmethyl)-5-[(1S oder R,2S oder R)-2-cyclopropyl-1,2-dihydroxyäthyl]-2,2-dimethyl-3-oxazolidincarbonsäure-tert-butylester das (1S oder R,2S oder R,3R,4S)-4-Amino-5-cyclohexyl-1-cyclopropyl-1,2,3-pentantriol als farbloser Festkörper, MS : 258 $(M+H)^+$;
- aus (4S,5R)-4-(Cyclohexylmethyl)-5-[(1R oder S,2S oder R)-2-cyclopropyl-1,2-dihydroxyäthyl]-2,2-dimethyl-3-oxazolidincarbonsäure-tert-butylester das (1S oder R,2R oder S,3R,4S)-4-Amino-5-cyclohexyl-1-cyclopropyl-1,2,3-pentantriol als farbloser Festkörper, MS : 258 $(M+H)^+$;
- aus (4S,5R)-4-(Cyclohexylmethyl)-5-[(1R oder S,2R oder S)-2-cyclopropyl-1,2-dihydroxyäthyl]-2,2-dimethyl-3-oxazolidincarbonsäure-tert-butylester das (1R oder S,2R oder S,3R,4S)-4-Amino-5-cyclohexyl-1-cyclopropyl-1,2,3-pentantriol als farbloser Festkörper, MS : 258 $(M+H)^+$.

## Beispiel 57

In analoger Weise wie in Beispiel 10 beschrieben, wurden die folgenden Verbindungen hergestellt:

- Aus (S)-α-[(S)-α-[(tert-Butylsulfonyl)methyl]hydrocinnamamido]-N-[(1R,2R,3S)-3-cyclopropyl-1-[[(cyclopropylmethyl)thio]methyl]-2,3-dihydroxypropyl]-1-Boc-imidazol-4-propionamid das (S)-α-[(S)-α-[(tert-Butylsulfonyl)methyl]hydrocinnamamido]-N-[(1R,2R,3S)-3-cyclopropyl-1-[[(cyclopropylmethyl)thio]methyl]-2,3-dihydroxypropyl]imidazol-4-propionamid als farbloser Festkörper, MS : 635 $(M+H)^+$;
- aus (S)-α-[(S)-α-[(tert-Butylsulfonyl)methyl]hydrocinnamamido]-N-[(1S,2S,3R)-3-cyclopropyl-1-[[(cyclopropylmethyl)thio]methyl]-2,3-dihydroxypropyl]-1-Boc-imidazol-4-propionamid das (S)-α-[(S)-α-[(tert-Butylsulfonyl)methyl]hydrocinnamamido]-N-[(1S,2S,3R)-3-cyclopropyl-1-[[(cyclopropylmethyl)thio]methyl]-2,3-dihydroxypropyl]imidazol-4-propionamid als farbloser Festkörper, MS : 635 $(M+H)^+$.

## Beispiel 58

In analoger Weise wie in Beispiel 10 beschrieben, wurden folgende Verbindungen hergestellt:

- Aus (S)-α-[(S)-α-[(tert-Butylsulfonyl)methyl]hydrocinnamamido]-N-[(1S,2R,3S oder R,4R oder S)-1-(cyclohexylmethyl)-4-cyclopropyl-2,3,4-trihydroxybutyl]-1-Boc-imidazol-4-propionamid das (S)-α-[(S)-α-[(tert-Butylsulfonyl)methyl]hydrocinnamamido] -N-[(1S,2R,3S oder R,4R oder S)-1-(cyclohexylmethyl)-4-cyclopropyl-2,3,4-trihydroxybutyl]imidazol-4-propionamid als farbloser Festkörper, MS : 661 $(M+H)^+$;
- aus (S)-α-[(S)-α-[(tert-Butylsulfonyl)methyl]hydrocinnamamido]-N-[(1S,2R,3S oder R,4S oder R)-1-(cyclohexylmethyl)-4-cyclopropyl-2,3,4-trihydroxybutyl]-1-Boc-imidazol-4-propionamid das (S)-α-[(S)-α-[(tert-Butylsulfonyl)methyl]hydrocinnamamido]-N-[(1S,2R,3S oder R,4S oder R)-1-(cyclohexylmethyl)-4-cyclopropyl-2,3,4-trihydroxybutyl]imidazol-4-propionamid als farbloser Festkörper, MS : 661 $(M+H)^+$;
- aus (S)-α-[(S)-α-[(tert-Butylsulfonyl)methyl]hydrocinnamamido]-N-[(1S,2R,3R oder S,4R oder S)-1-(cyclohexylmethyl)-4-cyclopropyl-2,3,4-trihydroxybutyl]-1-Boc-imidazol-4-propionamid das (S)-α-[(S)-α-[(tert-Butylsulfonyl)methyl]hydrocinnamamido]-N-[(1S,2R,3R oder S,4R oder S)-1-(cyclohexylmethyl)-4-cyclopropyl-2,3,4-trihydroxybutyl]imidazol-4-propionamid als farbloser Festkörper, MS : 661 $(M+H)^+$;
- aus (S)-α-[(S)-α-[(tert-Butylsulfonyl)methyl]hydrocinnamamido]-N-[(1S,2R,3R oder S,4S oder R)-1-(cyclohexylmethyl)-4-cyclopropyl-2,3,4-trihydroxybutyl]-1-Boc-imidazol-4-propionamid das (S)-α-[(S)-α-[(tert-Butylsulfonyl)methyl]hydrocinnamamido]-N-[(1S,2R,3R oder S,4S oder R)-1-(cyclohexylmethyl)-4-cyclopropyl-2,3,4-trihydroxybutyl]imidazol-4-propionamid als farbloser Festkörper, MS : 661 $(M+H)^+$.

## Beispiel 59

In analoger Weise wie in Beispiel 31 beschrieben, wurden folgende Verbindungen hergestellt:

- Aus N-[(S)-1-[[(1S,2R,3S)-1-(Cyclohexylmethyl)-3-cyclopropyl-2,3-dihydroxypropyl]carbamoyl]-2-(1-tritylimidazol)-4-yläthyl]-5-fluorindol-2-carboxamid das N-[(S)-1-[[(1S,2R,3S)-1-(Cyclohexylmethyl)-3-cyclopropyl-2,3-dihydroxypropyl]carbamoyl]-2-imidazol-4-yläthyl]-5-fluorindol-2-carboxamid als amorpher Festkörper, MS : 526 $(M+H)^+$;

- aus N-[(S)-1-[[(1S,2R,3S)-1-(Cyclohexylmethyl)-3-cyclopropyl-2,3-dihydroxypropyl]carbamoyl]-2-(1-tritylimidazol)-4-yläthyl]-5-methylindol-2-carboxamid das N-[(S)-1-[[(1S,2R,3S)-1-(Cyclohexylmethyl)-3-cyclopropyl-2,3-dihydroxypropyl]carbamoyl]-2-imidazol-4-yläthyl]-5-methylindol-2-carboxamid als amorpher Festkörper, MS : 522 $(M+H)^+$;
- aus N-[(S)-1-[[(1S,2R,3S)-1-(Cyclohexylmethyl)-3-cyclopropyl-2,3-dihydroxypropyl]carbamoyl]-2-(1-tritylimidazol)-4-yläthyl]-5-methoxyindol-2-carboxamid das N-[(S)-1-[[(1S,2R,3S)-1-(Cyclohexylmethyl)-3-cyclopropyl-2,3-dihydroxypropyl]carbamoyl]-2-imidazol-4-yläthyl]-5-methoxyindol-2-carboxamid als amorpher Festkörper, MS : 538 $(M+H)^+$;
- aus 3-Chlor-N[(S)-1-[[(1S,2R,3S)-1-(cyclohexylmethyl)-3-cyclopropyl-2,3-dihydroxypropyl]carbamoyl]-2-(1-tritylimidazol)-4-yläthyl]indol-2-carboxamid das 3-Chlor-N-[(S)-1-[[(1S,2R,3S)-1-(cyclohexylmethyl)-3-cyclopropyl-2,3-dihydroxypropyl]carbamoyl]-2-imidazol-4-yläthyl]indol-2-carboxamid als amorpher Festkörper, MS : 542 $(M+H)^+$;
- aus 3,6-Dichlor-N-[(S)-1-[[(1S,2R,3S)-1-(cyclohexylmethyl)-3-cyclopropyl-2,3-dihydroxypropyl]carbamoyl]-2-(1-tritylimidazol)-4-yläthyl]indol-2-carboxamid das 3,6-Dichlor-N-[(S)-1-[[(1S,2R,3S)-1-(cyclohexylmethyl)-3-cyclopropyl-2,3-dihydroxypropyl]carbamoyl]-2-imidazol-4-yläthyl]indol-2-carboxamid als amorpher Festkörper, MS : 576 $(M+H)^+$;
- aus (S)-α-Benzothiazolcarboxamido-N-[(1S,2R,3S)-1-(cyclohexylmethyl)-3-cyclopropyl-2,3-dihydroxypropyl]-1-tritylimidazol-4-propionamid das (S)-α-Benzothiazolcarboxamido-N-[(1S,2R,3S)-1-(cyclohexylmethyl)-3-cyclopropyl-2,3-dihydroxypropyl]imidazol-4-propionamid als amorpher Festkörper, MS : 526 $(M+H)^+$;
- aus N-[(S)-1-[[(1S,2R,3S)-1-(cyclohexylmethyl)-3-cyclopropyl-2,3-dihydroxypropyl]carbamoyl]-2-(1-tritylimidazol)-4-yläthyl]-2-chinolincarboxamid das N-[(S)-1-[[(1S,2R,3S)-1-(cyclohexylmethyl)-3-cyclopropyl-2,3-dihydroxypropyl]carbamoyl]-2-imidazol-4-yläthyl]-2-chinolincarboxamid als amorpher Festkörper, MS : 611 $(M+H)^+$;
- aus (S)-α-[(RS)-1,4-Benzodioxan-2-carboxamido]-N-[(1S,2R,3S)-1-(cyclohexylmethyl)-3-cyclopropyl-2,3-dihydroxypropyl]-1-tritylimidazol-4-propionamid das (S)-α-[(RS)-1,4-Benzodioxan-2-carboxamido]-N-[(1S,2R,3S)-1-(cyclohexylmethyl)-3-cyclopropyl-2,3-dihydroxypropyl]imidazol-4-propionamid als amorpher Festkörper, MS : 527 $(M+H)^+$;
- aus tert-Butyl [(RS)-1-[[(S)-1-[[(1S,2R,3S)-1-(cyclohexylmethyl)-3-cyclopropyl-2,3-dihydroxypropyl]-carbamoyl]-2-(1-tritylimidazol)-4-yläthyl]carbamoyl]-2-(3-pyridyl)äthyl]carbamat das tert-Butyl [(RS)-1-[[(S)-1-[[(1S,2R,3S)-1-(cyclohexylmethyl)-3-cyclopropyl-2,3-dihdyroxypropyl]carbamoyl]-2-imidazol-4-yläthyl]-carbamoyl]-2-(3-pyridyl)äthyl]carbamat als amorpher Festkörper, MS : 613 $(M+H)^+$.

## Beispiel 60

In analoger Weise wie Beispiel 34 beschrieben, wurden durch katalytische Hydrierung folgende Verbindungen hergestellt:

- Aus Benzyl [(R oder S)-5-[[(S)-1-[[(1S,2R,3S)-1-(cyclohexylmethyl)-3-cyclopropyl-2,3-dihydroxypropyl]-carbamoyl]-2-imidazol-4-yläthyl]carbamoyl]-1,1-dimethyl-3-oxo-6-phenylhexyl]carbamat das (S)-α-[(R oder S)-α-(4-Amino-4-methyl-2-oxopentyl)hydrocinnamamido]-N-[(1S,2R,3S)-1-(cyclohexylmethyl)-3-cyclopropyl-2,3-dihydroxypropyl]imidazol-4-propionamid-diacetat als farbloser Festkörper, MS : 610 $(M+H)^+$;
- aus Benzyl [(S oder R)-5-[[(S)-1-[[(1S,2R,3S)-1-(cyclohexylmethyl)-3-cyclopropyl-2,3-dihydroxypropyl]-carbamoyl]-2-imidazol-4-yläthyl]carbamoyl]-1,1-dimethyl-3-oxo-6-phenylhexyl]carbamat das (S)-α-[(S oder R)-α-(4-Amino-4-methyl-2-oxopentyl)hydrocinnamamido]-N-[(1S,2R,3S)-1-(cyclohexylmethyl)-3-cyclopropyl-2,3-dihydroxypropyl]imidazol-4-propionamid-diacetat als farbloser Festkörper, MS : 610 $(M+H)^+$;
-aus Benzyl cis/trans-4-[[[(S)-α-1-[[(1S,2R,3S)-1-(cyclohexylmethyl)-3-cyclopropyl-2,3-dihydroxypropyl]-carbamoyl-2-imidazol-4-yläthyl]carbamoyl]-p-methoxyphenäthyl]carbamoyl]methyl]cyclohexancarbamat das (S)-α-[(S)-α-[2-(p-Aminophenyl)acetamido]-p-methoxyhydrocinnamamido]-N-[(1S,2R,3S)-1-(cyclohexylmethyl)-3-cyclopropyl-2,3-dihydroxypropyl]imidazol-4-propionamid-diacetat als farbloses, amorphes Pulver, MS : 675 $(M+H)^+$;
- aus Benzyl [(S)-3-[[(S)-α-[[(S)-1-[[(1S,2R,3S)-1-(cyclohexylmethyl)-3-cyclopropyl-2,3-dihydroxypropyl]-carbamoyl]-2-imidazol-4-yläthyl]carbamoyl]-p-methoxyphenäthyl]carbamoyl]-2-hydroxyäthyl]carbamat das (S)-α- [(S)-α-[(S)-4-Amino-2-hydroxybutyramido]-p-methoxyhydrocinnamamido]-N-(1S,2R,3S)-1-

(cyclohexylmethyl)-3-cyclopropyl-2,3-dihydroxypropyl]imidazol-4-propionamid-diacetat als farbloses, amorphes Pulver, MS : 643 $(M+H)^+$;

- aus Benzyl [(S)-4-[[(S)-α-[[(S)-1-[[(1S,2R,3S)-1-(cyclohexylmethyl-3-cyclopropyl-2,3-dihydroxypropyl]-carbamoyl]-2-imidazol-4-yläthyl]carbamoyl]-p-methoxyphenäthyl]carbamoyl]-1-(methoxycarbonyl)butyl]-carbamat das (S)-2-Amino-5-[[(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]-p-methoxyphenäthyl]carbamoyl]-valeriansäuremethylester-diacetat als farbloses, amorphes Pulver, MS : 643 $(M+H)^+$.

## Beispiel 61

In analoger Weise wie in Beispiel 18b) beschrieben, wurde aus tert.Butyl [2-[[(S)-α-[[(R)-1-[[(1S,2R,3S)-1-cyclohexylmethyl)-3-cyclopropyl-2,3-dihydroxypropyl]carbamoyl-2-(methylthio)äthyl]carbamoyl]-p-methoxyphenäthyl]carbamoyl]-1,1-dimethyl]carbamat durch Abspaltung der Boc-Schutzgruppe mittels Salzsäure in Methanol das (S)-α-(3-Amino-3-methylbutyramido)-N-[(S)-1-[[(1S,2R,3S)-1-(cyclohexylmethyl)-3-cyclopropyl-2,3-dihydroxypropyl]carbamoyl]-2-(methylthio)äthyl]-p-methoxyhydrozimtsäureamid als farbloser Festkörper, MS : 621 $(M+H)^+$, erhalten.

## Beispiel A

Eine sterilfiltrierte wässrige Lösung von (S)-α-[(S)-α-[(tert-Butylsulfonyl)methyl]hydrocinnamamido]-N-[-(1S,2R)-1-(cyclohexylmethyl)-2-[(R oder S)-3-cyclopropyl-2-oxo-5-oxazolidinyl]-2-hydroxyäthyl]imidazol-4-propionamid wird unter Erwärmen mit einer sterilen Gelatinelösung, welche als Konservierungsmittel Phenol enthält, unter aseptischen Bedingungen so vermischt, dass 1,0 ml Lösung folgende Zusammensetzung hat:

| | |
|---|---|
| (S)-α-[(S)-α-[(tert-Butylsulfonyl)-methyl]hydrocinnamamido]-N-[(1S,2R)-1-(cyclohexylmethyl)-2-[(R oder S)-3-cyclopropyl-2-oxo-5-oxazolidinyl]-2-hydroxyäthyl]-imidazol-4-propionamid | 3,0 mg |
| Gelatine | 150,0 mg |
| Phenol | 4,7 mg |
| dest. Wasser bis zu | 1,0 ml |

Dis Mischung wird unter aseptischen Bedingungen in Vialen zu 1,0 ml abgefüllt.

Beispiel B

Man löst 5 mg (S)-α-[(S)-α-[(tert-Butylsulfonyl)methyl]hydrocinnamamido]-N-[(1S,2R)-1-(cyclohexylmethyl)-2-[(R oder S)-3-cyclopropyl-2-oxo-5-oxazolidinyl]-2-hydroxyäthyl]imidazol-4-propionamid in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

Beispiel C

In einer mischung von 3,5 ml Myglyol 812 und 0,08 g Benzylalkohol werden 500 mg fein gemahlenes (5,0 µm) (S)-α-[(S)-α-[(tert-Butylsulfonyl)methyl]hydrocinnamamido]-N-[(1S,2R)-1-(cyclohexylmethyl)-2-[(R oder S)-3-cyclopropyl-2-oxo-5-oxazolidinyl]-2-hydroxyäthyl]imidazol-4-propionamid suspendiert. Diese Suspension wird in einen Behälter mit Dosierventil eingefüllt. Es werden 5,0 g Freon 12 unter Druck durch das Ventil in den Behälter abgefüllt. Durch Schütteln wird das Freon in der Myglyol-Benzylalkoholmischung gelöst. Dieser Spraybehälter enthält ca. 100 Einzeldosen, die einzeln appliziert werden können.

Beispiel D

Wenn man nach den in den Beispielen A-C beschriebenen Verfahren arbeitet, können aus den folgenden, ebenfalls bevorzugten Verbindungen entsprechende galenische Präparate hergestellt werden:
(S)-α-[(S)-α-[[[(2-Amino-1,1-dimethyläthyl)sulfonyl]methyl]hydrocinnamamido]-N-[(1S,2R,3S)-1-(cyclohexylmethyl)-3-cyclopropyl-2,3-dihydroxypropyl]imidazol-4-propionamid-dihydrochlorid,
(S)-α-[(S)-α-[[(2-Amino-2-methylpropyl)sulfonyl]methyl]hydrocinnamamido]-N-[(1S,2R,3S)-1-(cyclohexylmethyl)-3-cyclopropyl-2,3-dihydroxypropyl]imidazol-4-propionsäureamiddiacetat,
(S)-α-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]phenäthyl 1-piperidincarboxylat,
(S)-α-[(S)-α-[(tert-Butylsulfonyl)methyl]hydrocinnamamido]-N-[(1S,2R,3S)-1-(cyclohexylmethyl)-3-cyclopropyl-3-fluor-2-hydroxypropyl]imidazol-4-propionamid und
N-[(S)-1-[[(1S,2R,3S)-1-(Cyclohexylmethyl)-3-cyclopropyl-2,3-dihydroxypropyl]carbamoyl]-2-imidazol-4-yläthyl]indol-2-carboxamid.

**Ansprüche**

1. Aminosäurederivate der allgemeinen Formel

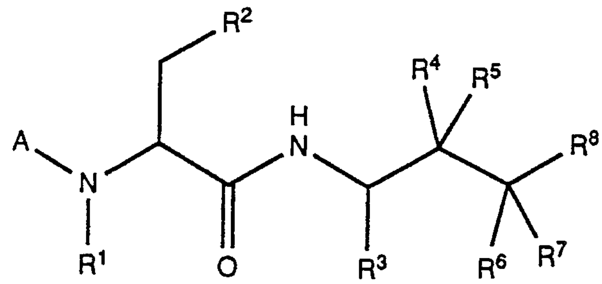

I

worin $R^1$ Wasserstoff oder Methyl, $R^2$ Aethyl, Propyl, Isopropyl,Thioalkyl, Imidazol-2-yl, Imidazol-4-yl, 5-Jodimidazol-4-yl, 5-Cyanimidazol-4-yl, N-Methylimidazol-2-yl, N-Methylimidazol-4-yl, C-methyliertes Imidazol-2-yl, C-methyliertes Imidazol-4-yl, N-substituiertes Imidazol-2-yl, N-substituiertes Imidazol-4-yl, Pyrazol-3-yl, Thiazol-4-yl, Thien-2-yl, Aethoxycarbonyl, Aminocarbonyl, Aminocarbonylmethyl, t-Butoxycarbonylmethyl, Benzyloxycarbonylmethyl oder t-Butoxy, $R^3$ Isobutyl, Cyclohexylmethyl, substituiertes Cyclohexylmethyl, Cyclohexenylmethyl, Cyclohexanonylmethyl, Bicyclo[3.1.0]hexylmethyl, Bicyclo[4.1.0]-heptylmethyl, Cycloalkylalkylthiomethyl, 1,3-Dithiolan-2-ylmethyl, 1,3-Dithian-2-ylmethyl, Halobenzyl oder Benzyl, $R^4$ Wasserstoff und $R^5$ Wasserstoff oder Alkyl und $R^5$ und $R^7$ unabhängig voneinander je Hydroxy, gegebenenfalls durch Amino, Monoalkylamino, Dialkylamino, Alkanoylamino, Alkoxycarbonylamino, Alkylcarbonyloxy, Carboxy, Alkoxy oder Hydroxy ein- oder mehrfach substituiertes Alkylcarbonyloxy, oder Arylcarbonyloxy, Arylalkylcarbonyloxy, Cycloalkylcarbonyloxy, Heteroarylalkylcarbonyloxy, die Gruppe $-OSO_3H$ oder $-PO(OR)_2$, worin R Alkyl bedeutet, oder mit einer O-Schutzgruppe geschütztes Hydroxy, oder $R^5$ Amino oder unter physiologischen Bedingungen leicht spaltbares, durch eine Schutzgruppe substituiertes Amino und $R^7$ Hydroxy, Amino, unter physiologischen Bedingungen leicht spaltbares, durch eine Schutzgruppe substituiertes Amino oder Azido oder $R^5$ und $R^7$ zusammen mit einer cyclischen O-Schutzgruppe geschütztes Hydroxy, oder $R^4$ und $R^5$ zusammen eine Oxogruppe und $R^6$ Wasserstoff oder Fluor und $R^7$ Fluor, oder $R^4$ Wasserstoff, $R^5$ Hydroxy, $R^6$ Wasserstoff und $R^7$ Amino, unter physiologischen Bedingungen leicht spaltbares, durch eine Schutzgruppe substituiertes Amino, Azido oder Fluor oder $R^6$ und $R^7$ je Fluor oder zusammen Oximino oder eine Oxogruppe, $R^8$ Hydroxymethyl, Alkylhydroxymethyl, Cycloalkylhydroxymethyl, Cycloalkylaminomethyl, Cycloalkylcarbonyl, oder eine der Gruppen

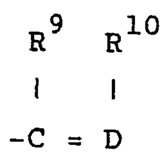

(a)

und

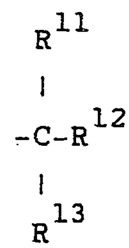

(b)

oder $R^7$ und $R^8$ zusammen 2-Oxo-3-cycloalkyl-oxazolidin-5-yl und A eine der Gruppen

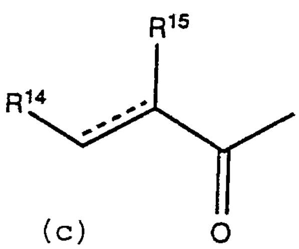

(c)

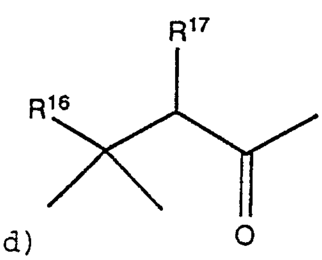

(d)

und $-(Y)_nZ$ (e)

bedeuten, worin D eine Methingruppe oder ein Stickstoffatom, $R^9$ Alkyl, Aryl oder Arylalkyl, $R^{10}$ Wasserstoff, Alkyl, Aryl oder Arylalkyl, oder $R^9$ und $R^{10}$ zusammen mit den beiden sie verbindenden Atomen, Aryl, Heteroaryl, Cycloalkenyl oder Heterocycloalkenyl, $R^{11}$ Wasserstoff oder Alkyl und $R^{12}$ und $R^{13}$ unabhängig voneinander je Alkyl, Aryl, Arylalkyl, Cycloalkyl oder die Gruppe

$-CH_2-X-R^{18}$ (f)

oder zusammen mit dem Kohlenstoffatom, an das sie gebunden sind, Cycloalkyl oder Heterocycloalkyl bedeuten, mit der Massgabe, dass, falls $R^{11}$ Alkyl bedeutet, $R^{12}$ und $R^{13}$ ebenfalls Alkyl bedeuten, die gestrichelte Linie eine zusätzliche Bindung bedeuten kann, $R^{14}$ und $R^{16}$ je Phenyl, substituiertes Phenyl, Benzyl, Naphthyl, Cyclohexyl, Thienyl oder Furyl und $R^{15}$ und $R^{17}$ je Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Cycloalkylsulfonylalkyl, Cycloalkylalkylsulfonylalkyl, substituiertes Phenylsulfonylalkyl, substituiertes Aminocarbonyloxy, Aminoalkylcarbonylalkyl, substituiertes Aminoalkylcarbonylalkyl, Heterocycloalkylcarbonyloxy, Guanidinium-methylsulfonat, substituiertes Aminoalkylsulfonylalkyl oder substituiertes Aminosulfonylalkyl bedeuten, mit der Massgabe, dass $R^{15}$ nicht Alkoxycarbonylamino oder Arylalkoxycarbonylamino bedeuten kann, wenn $R^{14}$ Phenyl, Halophenyl, Hydroxyphenyl, Methoxyphenyl, Benzyl, $\alpha$-Naphthyl, Cyclohexyl, Thienyl oder Furyl bedeutet, Y den N-terminal mit Z verbundenen, bivalenten Rest von gegebenenfalls N- und/oder $\alpha$-methyliertem Phenylalanin, Halophenylalanin, Cyclohexylalanin, Thienylalanin, Furylalanin, Pyridylalanin, Tyrosin, O-Methyltyrosin, $\alpha$-Naphthylalanin, Homophenylalanin oder 2-Hydroxy-3-amino-4-phenylbuttersäure, Z Wasserstoff, Acyl oder 1-Azabicyclo[2.2.2]octan-3-yl, n die Zahl 0 oder 1, X ein Sauerstoff- oder Schwefelatom oder die Gruppe -NH- und $R^{18}$ Wasserstoff, Alkyl, Cycloalkyl, Arylalkyl, Cycloalkylalkyl, Alkylcarbonyl, Arylcarbonyl oder Arylalkylcarbonyl bedeuten, mit den Massgaben, dass

(i) falls $R^4$ und $R^6$ je Wasserstoff und $R^5$ und $R^7$ unabhängig voneinander je Hydroxy, gegebenenfalls durch Amino, Monoalkylamino, Dialkylamino, Alkanoylamino, Alkoxycarbonylamino, Carboxy, Alkoxy oder Hydroxy ein- oder mehrfach substituiertes Alkylcarbonyloxy oder eine O-Schutzgruppe oder zusammen eine cyclische O-Schutzgruppe bedeuten, dann A die Gruppe (c), worin $R^{14}$ Cyclohexyl, Thienyl oder Furyl und/oder $R^{15}$ Cycloalkylsulfonylalkyl, Cycloalkylalkylsulfonylalkyl, substituiertes Phenylsulfonylalkyl, substituiertes Aminocarbonyloxy, Aminoalkylcarbonylalkyl, substituiertes Aminocarbonylalkyl, substituiertes Aminoalkylsulfonylalkyl oder substituiertes Aminosulfonylalkyl bedeuten, die Gruppe (d) oder (e) bedeutet, worin n die Zahl 0 oder Y Thienylalanin, Furylalanin oder Pyridylalanin und/oder Z 1-Azabicyclo[2.2.2]octan-3-yl und/oder $R^2$ Thioalkyl, N-substituiertes Imidazol-2-yl oder N-substituiertes Imidazol-4-yl und/oder $R^3$ substituiertes Cyclohexylmethyl, Cyclohexenylmethyl, Cyclohexanonylmethyl, Bicyclo[3.1.0]hexylmethyl, Bicyclo[4.1.0]heptylmethyl, Cycloalkylalkylthiomethyl, 1,3-Dithiolan-2-ylmethyl oder 1,3-Dithian-2-ylmethyl und/oder $R^8$ Hydroxymethyl, Cycloalkylhydroxymethyl, Cycloalkylaminomethyl oder Cycloalkylcarbonyl bedeuten,

(ii) falls $R^4$ und $R^6$ Wasserstoff, $R^5$ Amino und $R^7$ Hydroxy, Amino oder Azido oder $R^4$ Wasserstoff, $R^5$ Hydroxy, $R^6$ Wasserstoff und $R^7$ Amino, Azido oder Fluor oder $R^6$ und $R^7$ je Fluor oder zusammen eine Oxogruppe oder $R^4$ und $R^5$ zuammen eine Oxogruppe und $R^6$ Wasserstoff oder Fluor und $R^7$ Fluor bedeuten, dann $R^8$ die Gruppe (a) oder (b) bedeutet,

(iii) falls $R^8$ Hydroxymethyl, Alkylhydroxymethyl, Cycloalkylhydroxymethyl, Cycloalkylaminomethyl oder Cycloalkylcarbonyl bedeutet, dann $R^4$ und $R^6$ je Wasserstoff und $R^5$ und $R^7$ je Hydroxy bedeuten, oder falls $R^7$ und $R^8$ zusammen 2-Oxo-3-cycloalkyl-oxazolidin-5-yl bedeuten, dann $R^4$ und $R^6$ je Wasserstoff und $R^5$ Hydroxy bedeuten, und

(iv) falls $R^8$ Cycloalkylaminomethyl bedeutet, dann $R^{15}$ von Alkylthioalkyl, Alkylsulfinylalkyl, Alkylsulfonylalkyl, substituiertem Aminoalkylsulfonylalkyl oder substituiertem Aminosulfonylalkyl verschieden ist,

in Form von optisch reinen Diastereomeren, Diastereomerengemischen, diastereomeren Racematen oder Gemischen von diastereomeren Racematen sowie pharmazeutisch verwendbare Salze dieser Verbindungen.

2. Verbindungen gemäss Anspruch 1, worin $R^2$ von Thioalkyl, Aminocarbonyl und Aminocarbonylmethyl, $R^3$ von Cycloalkylalkylthiomethyl, $R^{15}$ und $R^{17}$ je von Cycloalkylsulfonylalkyl, Cycloalkylalkylsulfonylalkyl, substituiertem Phenylsulfonylalkyl und Aminoalkylcarbonylalkyl, Y von Pyridylalanin und Z von 1-Azabicyclo[2.2.2] octan-3-yl verschieden sind.

3. Verbindungen gemäss Anspruch 1 oder 2, worin $R^1$ Wasserstoff, $R^2$ Imidazol-2-yl, Imidazol-4-yl, Thiazol-4-yl, Aminocarbonyl oder Aminocarbonylmethyl, $R^3$ Cyclohexylmethyl, substituiertes Cyclohexylmethyl oder Cyclohexenylmethyl, $R^4$ und $R^6$ je Wasserstoff, $R^5$ Hydroxy, Amino oder durch Amino monosubstituiertes Alkylcarbonyloxy, $R^7$ Hydroxy, Amino, durch Amino monosubstituiertes Alkylcarbonyloxy, Azido oder Fluor, $R^8$ Alkylhydroxymethyl, Cycloalkylhydroxymethyl oder die Gruppe (b), oder $R^7$ und $R^8$ zusammen 2-Oxo-3-cycloalkyl-oxazolidin-5-yl, A die Gruppe (c) oder (e), $R^{11}$ Wasserstoff und $R^{12}$ und $R^{13}$ je Alkyl oder zusammen mit dem Kohlenstoffatom, an das sie gebunden sind, Cycloalkyl, $R^{14}$ Phenyl oder substituiertes Phenyl, $R^{15}$ Alkylcarbonylalkyl, Heterocycloalkylcarbonylalkyl, substituiertes Aminocarbonylalkyl, Alkylsulfonylalkyl, Cycloalkylsulfonylalkyl, Cycloalkylalkylsulfonylalkyl, substituiertes Aminoalkylcarbonylalkyl, Heterocycloalkylcarbonyloxy, Aminoalkylsulfonylalkyl oder substituiertes Aminosulfonylalkyl, Y den N-terminal mit Z verbundenen, bivalenten Rest von Phenylalanin oder O-Methyltyrosin und Z Gruppe $R^a$-CO- bedeuten, worin $R^a$ einen unsubstituierten oder substituierten, gesättigten, gegebenenfalls mit Hydroxy und/oder Amino, Monoalkylamino, Dialkylamino, Alkanoylamino, Alkoxycarbonylamino, Arylalkoxycarbonylamino oder substituiertem Aminocarbonyl funktionalisierten aliphatischen Kohlenwasserstoffrest mit bis zu 10 Kohlenstoffatomen oder einen unsubstituierten oder substituierten heteraromatischen Kohlenwasserstoffrest mit bis zu 18 Kohlenstoffatomen bedeutet, insbesondere den einwertigen, über die Carboxylgruppe gebundenen Rest von Prolin, Pyroglutaminsäure, α-Methylalanin, Aminoäthylglycin, D-Alanin, β-Alanin oder β,β-Dimethylalanin.

4. Verbindungen gemäss einem der Ansprüche 1-3, worin $R^1$ Wasserstoff, $R^2$ Imidazol-4-yl, $R^3$ Cyclohexylmethyl oder 4,4-Difluorcyclohexylmethyl, $R^4$ und $R^6$ je Wasserstoff, $R^5$ und $R^7$ je Hydroxy oder Aminomethylcarbonyloxy, $R^8$ die Gruppe (b), $R^{11}$ Wasserstoff, $R^{12}$ und $R^{13}$ zusammen mit dem Kohlenstoffatom, an das sie gebunden sind, Cyclopropyl oder Cyclobutyl, $R^{14}$ Phenyl und $R^{15}$ $C_1$-$C_4$-Alkylcarbonylmethyl, Heterocycloalkylcarbonylmethyl, substituiertes Aminocarbonylmethyl, $C_1$-$C_4$-Alkylsulfonylmethyl, $C_3$-$C_6$-Cycloalkylsulfonylmethyl, $C_3$-$C_6$-Cycloalkyl-$C_1$-$C_4$-alkylsulfonylmethyl, substituiertes Amino-$C_1$-$C_4$-alkylcarbonylmethyl, Heterocycloalkylcarbonyloxy, Amino-$C_1$-$C_4$-alkylsulfonylmethyl oder substituiertes Amino-$C_1$-$C_4$-alkylsulfonylmethyl bedeuten.

5. (S)-α-[(S)-α-[[[(2-Amino-1,1-dimethyläthyl)sulfonyl]methyl]hydrocinnamamido]-N-[(1S,2R,3S)-1-(cyclohexylmethyl)-3-cyclopropyl-2,3-dihydroxypropyl]imidazol-4-propionamid-dihydrochlorid.

6. (S)-α-[(S)-α-[(tert-Butylsulfonyl)methyl]hydrocinnamamido]-N-[(1S,2R,3S)-1-(cyclohexylmethyl)-3-cyclopropyl-2,3-dihydroxypropyl]-5-cyanimidazol-4-propionamid.

7. (S)-α-[(S)-α-[(tert-Butylsulfonyl)methyl]hydrocinnamamido]-N-[(1S,2R,3S)-3-cyclopropyl-1-[(4,4-difluorcyclohexyl)methyl]-2,3-dihydroxypropyl]imidazol-4-propionamid.

8. (S)-α-[(S)-α-[(tert-Butylsulfonyl)methyl]hydrocinnamamido]-N-[(1S,2R,3S)-3-cyclopropyl-1-(p-fluorbenzyl)-2,3-dihydroxypropyl]imidazol-4-propionamid.

9. (S)-α-[(S)-α-[[(2-Amino-2-methylpropyl)sulfonyl]methyl]hydrocinnamamido]-N-[(1S,2R,3S)-1-(cyclohexylmethyl)-3-cyclopropyl-2,3-dihydroxypropyl]imidazol-4-propionsäureamiddiacetat.

10. (S)-N-[(1S,2R,3S)-3-Azido-1-(cyclohexylmethyl)-3-cyclopropyl-2-hydroxypropyl]-α-[ (S)-α-(tert-butylsulfonyl)methyl]hydrocinnamamido]imidazol-4-propionamid, (S)-α-( (S)-α-[(tert-Butylsulfonyl)methyl]-hydrocinnamamido]-N-[(1S,2R)-1-(cyclohexylmethyl)-2-[(R oder S)-3-cyclopropyl-2-oxo-5-oxazolidinyl]-2-hydroxyäthyl]imidazol-4-propionamid, (S)-α-[[(S)-1-[[(1S,2R,3S)-1-(cyclohexylmethyl )-3-cyclopropyl-2,3-dihydroxypropyl]carbamoyl ]-2-imidazol-4-yläthyl]carbamoyl]phenäthyl-1-piperidincarboxylat, (S)-α-[[(S)-1-[[(1S,2R,3S)-1-(cyclohexylmethyl)-3-cyclopropyl-2,3-dihydroxypropyl]carbamoyl]-2-imidazol-4-yläthyl]-carbamoyl]phenäthyl 4-morpholincarboxylat, (R)-2-[[(S)-α-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]phenäthyl]carbamoyl]-1-pyrrolidincarbonsäure-tert-butylester, (S)-α-[(S)-α-((tert-Butylsulfonyl)methyl]hydrocinnamamido]-N-[(1S,2R,3S)-1-(cyclohexylmethyl)-3-cyclopropyl-3-fluor-2-hydroxypropyl]imidazol-4-propionamid, tert-Butyl [1-[(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]phenäthyl]carbamoyl]-1-methyläthyl]carbamat, Di-tert-butyl N-[(S)-1-(tert-butoxycarbonyl)-2-([(S)-α-[[(S)-1-[[(1S,2R,3S)-1-(cyclohexylmethyl)-3-cyclopropyl-2,3-dihydroxypropyl]carbamoyl]-2-imidazol-4-yläthyl]carbamoyl]phenäthyl]carbamoyl]äthyl]äthylendicarbamat, (S)-α-[(S)-α-[(tert-Butylsulfonyl)methyl]-hydrocinnamamido]-N-[(1S,2R,3RS)-1-(cyclohexylmethyl)-2,3,4-trihydroxybutyl]imidazol-4-propionamid, (S oder R)-2-[[(S)-1-[[(1S,2R,3S)-1-(Cyclohexylmethyl)-3-cyclopropyl-2,3-dihydroxypropyl]carbamoyl]-2-imidazol-4-yläthyl]carbamoyl]-3-phenylpropansulfonsäure-guanidinsalz, Di-tert-butyl N-[[[(S)-α-[[(S)-1-[[(1S,2R,3S)-1-(cyclohexylmethyl)-3-cyclopropyl-2,3-dihydroxypropyl]carbamoyl]-2-imidazol-4-yläthyl]-carbamoyl]phenäthyl]carbamoyl]methyl]äthylendicarbamat, N-[(S)-1-[[(1S,2R,3S)-1-(Cyclohexylmethyl)-3-cyclopropyl-2,3-dihydroxypropyl]carbamoyl]-2-imidazol- 4-yläthyl]indol-2-carboxamid, (S)-N-[(1S,2R,3S)-1-(Cyclohexylmethyl)-3-cyclopropyl-2,3-dihydroxypropyl]-α-(2,2-dibenzylacetamido)imidazol-4-propionamid, (S)-α-[(S)-α-(2-[(2-Aminoäthyl)amino]acetamido]hydrocinnamamido]-N-[(1S,2R,3S)-1-(cyclohexylmethyl)-3-cyclopropyl-2,3-dihydroxypropyl]imidazol-4-propionamid, N-[(S)-1-[[(1S,2R,3S)-1-(Cyclohexylmethyl)-3-cyclopropyl-2,3-dihydroxypropyl]carbamoyl]-2-imidazol-4-yläthyl]-2-benzimidazolcarboxamid oder (S)-α-[(S)-α-[(tert-Butylsulfonyl)methyl]hydrocinnamamido]-N-[(1S,2R,3R oder S,4R oder S)-1-(cyclohexylmethyl)-2,3,4-trihydroxyhexyl]imidazol-4-propionamid.

11. Aminosäurederivate gemäss einem der Ansprüche 1-10 zur Anwendung als therapeutische Wirkstoffe.

12. Aminosäurederivate gemäss einem der Ansprüche 1-10 zur Anwendung bei der Bekämpfung bzw. Verhütung von Bluthochdruck und Herzinsuffizienz.

13. Verfahren zur Herstellung einer Verbindung gemäss einem der Ansprüche 1-10, dadurch gekennzeichnet, dass man

a) eine Verbindung der allgemeinen Formel

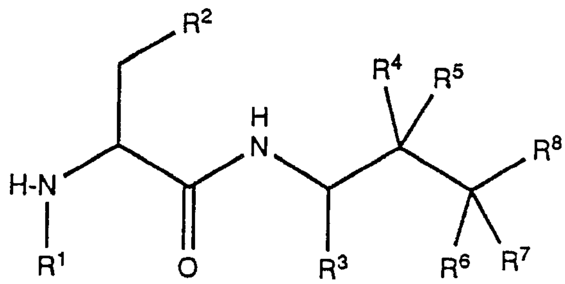

II

worin $R^1$, $R^2$, $R^3$, $R^4$, $R^5$, $R^6$, $R^7$ und $R^8$ die in Anspruch 1 angegebene Bedeutung besitzen, mit einem die Gruppe

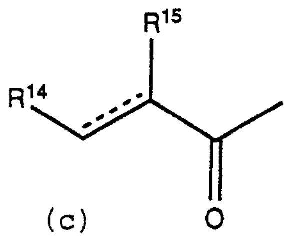

(c)

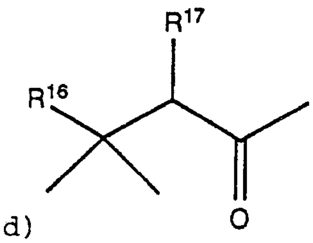

(d) oder $-(Y)_nZ$ (e)

worin $R^{14}$, $R^{15}$, $R^{16}$, $R^{17}$ Y, Z, n und die gestrichelte Linie die in Anpruch 1 angegebene Bedeutung besitzen,

abgebenden Acylierungsmittel umsetzt, oder

b) eine Verbindung der allgemeinen Formel

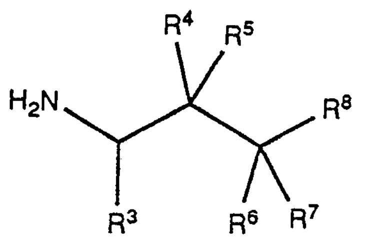

III

worin $R^3$, $R^4$, $R^5$, $R^6$, $R^7$ und $R^8$ die in Anspruch 1 angegebene Bedeutung besitzen, mit einer Verbindung der allgemeinen Formel

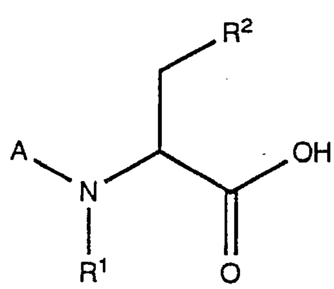

IV

worin $R^1$, $R^2$ und A die in Anpruch 1 angegebene Bedeutung besitzen, oder einem aktivierten Derivat davon umsetzt, oder

c) zur Herstellung einer Verbindung der Formel I, worin A die Gruppe (e) und Z den einwertigen, über die Carboxylgruppe gebundenen Rest einer Aminosäure bedeuten und die übrigen Symbole die in Anpruch 1 angegebene Bedeutung besitzen, eine Verbindung der Formel I, worin Z Wasserstoff bedeutet und die übrigen Symbole die in Anspruch 1 angegebene Bedeutung besitzen, mit einer Aminosäure umsetzt, oder

d) zur Herstellung einer Verbindung der Formel I, worin $R^5$ und/oder $R^7$ gegebenenfalls durch Amino, Monoalkylamino, Dialkylamino, Alkanoylamino, Alkoxycarbonylamino, Alkylcarbonyloxy, Carboxy, Alkoxy oder Hydroxy ein- oder mehrfach substituiertes Alkylcarbonyloxy, oder Arylcarbonyloxy, Arylalkylcarbonyloxy, Cycloalkylcarbonyloxy, Heteroarylalkylcarbonyloxy, die Gruppe $-OSO_3H$ oder $-PO(OR)_2$, worin R Alkyl bedeutet, oder mit einer O-Schutzgruppe geschütztes Hydroxy oder unter physiologischen Bedingungen leicht spaltbares, durch eine Schutzgruppe substituiertes Amino oder zusammen mit einer cyclischen O-Schutzgruppe geschütztes Hydroxy bedeuten und die übrigen Symbole die in Anspruch 1 angegebene Bedeutung besitzen, eine Verbindung der Formel I, worin $R^5$ und/oder $R^7$ Hydroxy oder Amino bedeuten und die übrigen Symbole die in Anspruch 1 angegebene Bedeutung besitzen, mit einem gegebenenfalls durch Amino, Monoalkylamino, Dialkylamino, Alkanoylamino, Alkoxycarbonylamino, Alkylcarbonyloxy, Carboxy, Alkoxy oder Hydroxy ein- oder mehrfach substituierten Alkanoylierungsmittel oder einem Aroylierungs-, Arylalkanoylierungs-, Cycloalkanoylierungs- oder Heteroarylalkanoylierungsmittel, einem Sulfatierungs- oder Phosphorylierungsmittel oder mit einem eine N- und/oder O-Schutzgruppe oder cyclische O-Schutzgruppe bildenden Mittel umsetzt, oder

e) zur Herstellung einer Verbindung der Formel I, worin $R^2$ N-substituiertes, gegebenenfalls C-methyliertes Imidazol-2-yl oder N-substituiertes, gegebenenfalls C-methyliertes Imidazol-4-yl bedeutet und die übrigen Symbole die in Anspruch 1 angegebene Bedeutung besitzen, eine Verbindung der Formel I, worin $R^2$ gegebenenfalls C-methyliertes Imidazol-2-yl oder gegebenenfalls C-methy liertes Imidazol-4-yl bedeutet und die übrigen Symbole die in Anspruch 1 angegebene Bedeutung besitzen, mit einem geeigneten Acylierungs-, Alkylierungs-, Arylalkylierungs- bzw. Arylierungsmittel umsetzt, oder

f) zur Herstellung einer Verbindung der Formel I, worin A eine freie Aminogruppe enthält und/oder $R^2$ Imidazol-2-yl, Imidazol-4-yl, C-methyliertes Imidazol-2-yl, C-methyliertes Imidazol-4-yl oder Pyrazol-3-yl und/oder $R^5$ und/oder $R^7$ Amino bedeuten und/oder $R^8$ eine primäre oder sekundäre Aminogruppe enthält und die übrigen Symbole die in Anspruch 1 angegebene Bedeutung besitzen, aus einer Verbindung der allgemeinen Formel

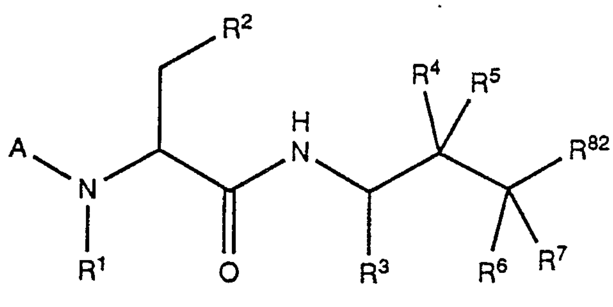

I a

worin $R^{82}$ die gleiche Bedeutung wie $R^8$ hat, jedoch noch zusätzlich N-geschütztes Cycloalkylaminomethyl bedeutet und die übrigen Symbole die in Anspruch 1 angegebene Bedeutung besitzen, mit der Massgabe, dass mindestens einer von A, $R^2$, $R^5$, $R^7$ und $R^{82}$ eine N-Schutzgruppe enthält, die N-Schutzgruppe(n) abspaltet, und

g) erwünschtenfalls ein Gemisch diastereomerer Racemate in die diastereomeren Racemate oder optisch reinen Diastereomeren auftrennt, und/oder

h) erwünschtenfalls ein Diastereomerengemisch in die optisch reinen Diastereomeren auftrennt, und/oder

i) erwünschtenfalls eine erhaltene Verbindung in ein pharmazeutisch verwendbares Salz überführt.

14. Arzneimittel, enthaltend ein Aminosäurederivat gemäss einem der Ansprüche 1-10 und ein therapeutisch inertes Excipiens.

15. Mittel zur Bekämpfung bzw. Verhütung von Bluthochdruck und Herzinsuffizienz, enthaltend ein Aminosäurederivat gemäss einem der Ansprüche 1-10 und ein therapeutisch inertes Excipiens.

16. Verwendung eines Aminosäurederivates gemäss einem der Ansprüche 1-10 bei der Bekämpfung bzw. Verhütung von Krankheiten.

17. Verwendung eines Aminosäurederivates gemäss einem der Ansprüche 1-10 bei der Bekämpfung bzw. Verhütung von Bluthochdruck und Herzinsuffizienz.

18. Verwendung eines Aminosäurederivates gemäss einem der Ansprüche 1-10 zur Herstellung von Mitteln gegen Bluthochdruck und/oder Herzinsuffizienz.

Patentansprüche für folgende Vertragsstaaten: ES, GR.

1. Verfahren zur Herstellung von Aminosäurederivaten der allgemeinen Formel

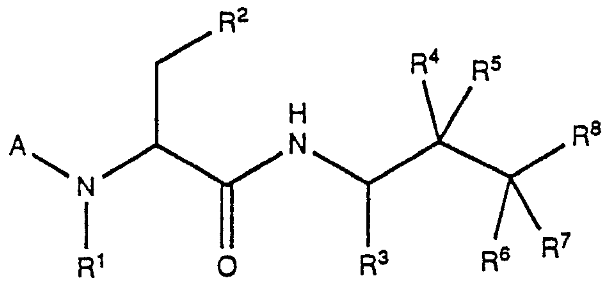

I

worin $R^1$ Wasserstoff oder Methyl, $R^2$ Aethyl, Propyl, Isopropyl, Thioalkyl, Imidazol-2-yl, Imidazol-4-yl, 5-Jodimidazol-4-yl, 5-Cyanimidazol-4-yl, N-Methylimidazol-2-yl, N-Methylimidazol-4-yl, C-methyliertes Imidazol-2-yl, C-methyliertes Imidazol-4-yl, N-substituiertes Imidazol-2-yl, N-substituiertes Imidazol-4-yl, Pyrazol-3-yl, Thiazol-4-yl, Thien-2-yl, Aethoxycarbonyl, Aminocarbonyl, Aminocarbonylmethyl, t-Butoxycarbonylmethyl, Benzyloxycarbonylmethyl oder t-Butoxy, $R^3$ Isobutyl, Cyclohexylmethyl, substituiertes Cyclohexylmethyl, Cyclohexenylmethyl, Cyclohexanonylmethyl, Bicyclo[3.1.0]hexylmethyl, Bicyclo[4.1.0]-heptylmethyl, Cycloalkylalkylthiomethyl, 1,3-Dithiolan-2-ylmethyl, 1,3-Dithian-2-ylmethyl, Halobenzyl oder Benzyl, $R^4$ Wasserstoff und $R^6$ Wasserstoff oder Alkyl und $R^5$ und $R^7$ unabhängig voneinander je Hydroxy, gegebenenfalls durch Amino, Monoalkylamino, Dialkylamino, Alkanoylamino, Alkoxycarbonylamino, Alkylcarbonyloxy, Carboxy, Alkoxy oder Hydroxy ein- oder mehrfach substituiertes Alkylcarbonyloxy, oder Arylcarbonyloxy, Arylalkylcarbonyloxy, Cycloalkylcarbonyloxy, Heteroarylalkylcarbonyloxy, die Gruppe $-OSO_3H$ oder $-PO(OR)_2$, worin R Alkyl bedeutet, oder mit einer O-Schutzgruppe geschütztes Hydroxy, oder $R^5$ Amino oder unter physiologischen Bedingungen leicht spaltbares, durch eine Schutzgruppe substituiertes Amino und $R^7$ Hydroxy, Amino, unter physiologischen Bedingungen leicht spaltbares, durch eine Schutzgruppe substituiertes Amino oder Azido oder $R^5$ und $R^7$ zusammen mit einer cyclischen O-Schutzgruppe geschütztes Hydroxy, oder $R^4$ und $R^5$ zusammen eine Oxogruppe und $R^6$ Wasserstoff oder Fluor und $R^7$ Fluor, oder $R^4$ Wasserstoff, $R^5$ Hydroxy, $R^6$ Wasserstoff und $R^7$ Amino, unter physiologischen Bedingungen leicht spaltbares, durch eine Schutzgruppe substituiertes Amino, Azido oder Fluor oder $R^6$ und $R^7$ je Fluor oder zusammen Oximino oder eine Oxogruppe, $R^8$ Hydroxymethyl, Alkylhydroxymethyl, Cycloalkylhydroxymethyl, Cycloalkylaminomethyl, Cycloalkylcarbonyl, oder eine der Gruppen

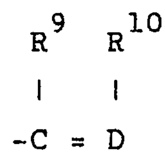

(a)

und

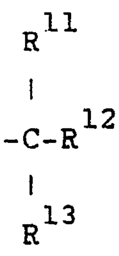

(b)

oder $R^7$ und $R^8$ zusammen 2-Oxo-3-cycloalkyl-oxazolidin-5-yl und A eine der Gruppen

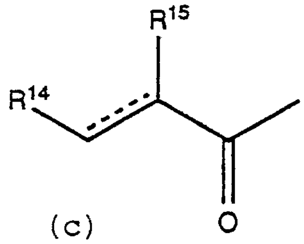

(c)

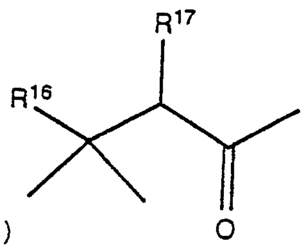

(d)

und $-(Y)_n Z$ (e)

bedeuten, worin D eine Methingruppe oder ein Stickstoffatom, $R^9$ Alkyl, Aryl oder Arylalkyl, $R^{10}$ Wasserstoff, Alkyl, Aryl oder Arylalkyl, oder $R^9$ und $R^{10}$ zusammen mit den beiden sie verbindenden Atomen, Aryl, Heteroaryl, Cycloalkenyl oder Heterocycloalkenyl, $R^{11}$ Wasserstoff oder Alkyl und $R^{12}$ und $R^{13}$ unabhängig voneinander je Alkyl, Aryl, Arylalkyl, Cycloalkyl oder die Gruppe

$-CH_2-X-R^{18}$ (f)

oder zusammen mit dem Kohlenstoffatom, an das sie gebunden sind, Cycloalkyl oder Heterocycloalkyl bedeuten, mit der Massgabe, dass, falls $R^{11}$ Alkyl bedeutet, $R^{12}$ und $R^{13}$ ebenfalls Alkyl bedeuten, die gestrichelte Linie eine zusätzliche Bindung bedeuten kann, $R^{14}$ und $R^{16}$ je Phenyl, substituiertes Phenyl, Benzyl, Naphthyl, Cyclohexyl, Thienyl oder Furyl und $R^{15}$ und $R^{17}$ je Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Alkoxycarbonylhydroxyalkyl Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Cycloalkylsulfonylalkyl, Cycloalkylalkylsulfonylalkyl, substituiertes Phenylsulfonylalkyl, substituiertes Aminocarbonyloxy, Aminoalkylcarbonylalkyl, substituiertes Aminoalkylcarbonylalkyl, Heterocycloalkylcarbonyloxy, Guanidinium-methylsulfonat, substituiertes Aminoalkylsulfonylalkyl oder substituiertes Aminosulfonylalkyl bedeuten, mit der Massgabe, dass $R^{15}$ nicht Alkoxycarbonylamino oder Arylalkoxycarbonylamino bedeuten kann, wenn $R^{14}$ Phenyl, Halophenyl, Hydroxyphenyl, Methoxyphenyl, Benzyl, $\alpha$-Naphthyl, Cyclohexyl, Thienyl oder Furyl bedeutet, Y den N-terminal mit Z verbundenen, bivalenten Rest von gegebenenfalls N- und/oder $\alpha$-methyliertem Phenylalanin, Halophenylalanin, Cyclohexylalanin, Thienylalanin, Furylalanin, Pyridylalanin, Tyrosin, O-Methyltyrosin, $\alpha$-Naphthylalanin, Homophenylalanin oder 2-Hydroxy-3-amino-4-phenylbuttersäure, Z Wasserstoff, Acyl oder 1-Azabicyclo[2.2.2]octan-3-yl, n die Zahl 0 oder 1, X ein Sauerstoff- oder Schwefelatom oder die Gruppe -NH- und $R^{18}$ Wasserstoff, Alkyl, Cycloalkyl, Arylalkyl, Cycloalkylalkyl, Alkylcarbonyl, Arylcarbonyl oder Arylalkylcarbonyl bedeuten, mit den Massgaben, dass

(i) falls $R^4$ und $R^6$ je Wasserstoff und $R^5$ und $R^7$ unabhängig voneinander je Hydroxy, gegebenenfalls durch Amino, Monoalkylamino, Dialkylamino, Alkanoylamino, Alkoxycarbonylamino, Carboxy, Alkoxy oder Hydroxy ein- oder mehrfach substituiertes Alkylcarbonyloxy oder eine O-Schutzgruppe oder zusammen eine cyclische O-Schutzgruppe bedeuten, dann A die Gruppe (c), worin $R^1$ Cyclohexyl, Thienyl oder Furyl und/oder $R^{15}$ Cycloalkylsulfonylalkyl, Cycloalkylalkylsulfonylalkyl, substituiertes Phenylsulfonylalkyl, substituiertes Aminocarbonyloxy, Aminoalkylcarbonylalkyl, substituiertes Aminocarbonylalkyl, substituiertes Aminoalkylsulfonylalkyl oder substituiertes Aminosulfonylalkyl bedeuten, die Gruppe (d) oder (e) bedeutet, worin n die Zahl 0 oder Y Thienylalanin, Furylalanin oder Pyridylalanin und/oder Z 1-Azabicyclo[2.2.2]octan-3-yl und/oder $R^2$ Thioalkyl, N-substituiertes Imidazol-2-yl oder N-substituiertes Imidazol-4-yl und/oder $R^3$ substituiertes Cyclohexylmethyl, Cyclohexenylmethyl, Cyclohexanonylmethyl, Bicyclo[3.1.0]hexylmethyl, Bicyclo-(4.1.0]heptylmethyl, Cycloalkylalkylthiomethyl, 1,3-Dithiolan-2-ylmethyl oder 1,3-Dithian-2-ylmethyl und/oder $R^8$ Hydroxymethyl, Cycloalkylhydroxymethyl, Cycloalkylaminomethyl oder Cycloalkylcarbonyl bedeuten,

(ii) falls $R^4$ und $R^6$ Wasserstoff, $R^5$ Amino und $R^7$ Hydroxy, Amino oder Azido oder $R^4$ Wasserstoff, $R^5$ Hydroxy, $R^6$ Wasserstoff und $R^7$ Amino, Azido oder Fluor oder $R^6$ und $R^7$ je Fluor oder zusammen eine Oxogruppe oder $R^4$ und $R^5$ zuammen eine Oxogruppe und $R^6$ Wasserstoff oder Fluor und $R^7$ Fluor bedeuten, dann $R^8$ die Gruppe (a) oder (b) bedeutet,

(iii) falls $R^8$ Hydroxymethyl, Alkylhydroxymethyl, Cycloalkylhydroxymethyl, Cycloalkylaminomethyl oder Cycloalkylcarbonyl bedeutet, dann $R^4$ und $R^6$ je Wasserstoff und $R^5$ und $R^7$ je Hydroxy bedeuten, oder falls $R^7$ und $R^8$ zusammen 2-Oxo-3-cycloalkyl-oxazolidin-5-yl bedeuten, dann $R^4$ und $R^6$ je Wasserstoff und $R^5$ Hydroxy bedeuten, und

(iv) falls $R^8$ Cycloalkylaminomethyl bedeutet, dann $R^{15}$ von Alkylthioalkyl, Alkylsulfinylalkyl, Alkylsulfonylalkyl, substituiertem Aminoalkylsulfonylalkyl oder substituiertem Aminosulfonylalkyl verschieden ist,

in Form von optisch reinen Diastereomeren, Diastereomerengemischen, diastereomeren Racematen oder Gemischen von diastereomeren Racematen sowie pharmazeutisch verwendbare Salze dieser Verbindungen,

dadurch gekennzeichnet, dass man a) eine Verbindung der allgemeinen Formel

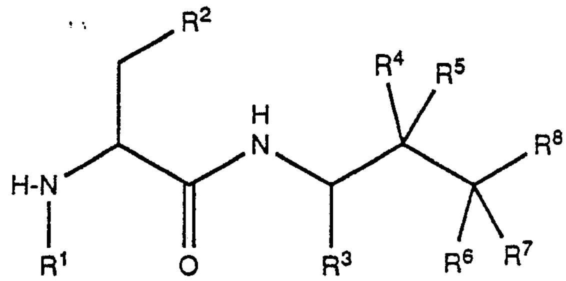

II

worin $R^1$, $R^2$, $R^3$, $R^4$, $R^5$, $R^6$, $R^7$ und $R^8$ die oben angegebene Bedeutung besitzen, mit einem die Gruppe

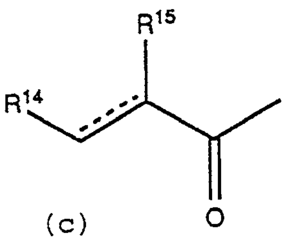

(c)

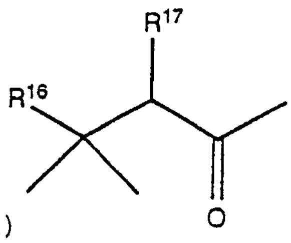

(d)

oder $-(Y)_nZ$ (e)

worin $R^{14}$, $R^{15}$, $R^{16}$, $R^{17}$ Y, Z, n und die gestrichelte Linie die oben angegebene Bedeutung besitzen, abgebenden Acylierungsmittel umsetzt, oder

b) eine Verbindung der allgemeinen Formel

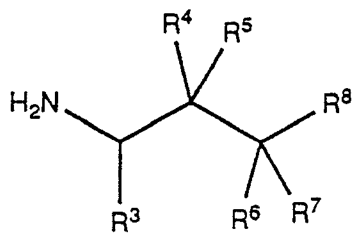

III

worin $R^3$, $R^4$, $R^5$, $R^6$, $R^7$ und $R^8$ die oben angegebene Bedeutung besitzen, mit einer Verbindung der allgemeinen Formel

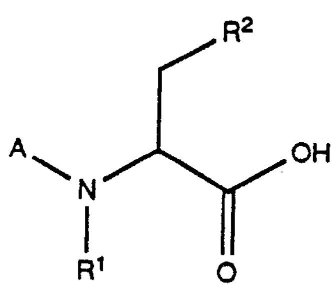

IV

worin $R^1$, $R^2$ und A die oben angegebene Bedeutung besitzen, oder einem aktivierten Derivat davon umsetzt, oder

c) zur Herstellung einer Verbindung der Formel I, worin A die Gruppe (e) und Z den einwertigen, über die Carboxylgruppe gebundenen Rest einer Aminosäure bedeuten und die übrigen Symbole die oben angegebene Bedeutung besitzen, eine Verbindung der Formel I, worin Z Wasserstoff bedeutet und die übrigen Symbole die oben angegebene Bedeutung besitzen, mit einer Aminosäure umsetzt, oder

d) zur Herstellung einer Verbindung der Formel I, worin $R^5$ und/oder $R^7$ gegebenenfalls durch Amino, Monoalkylamino, Dialkylamino, Alkanoylamino, Alkoxycarbonylamino, Alkylcarbonyloxy, Carboxy, Alkoxy oder Hydroxy ein- oder mehrfach substituiertes Alkylcarbonyloxy, oder Arylcarbonyloxy, Arylalkylcarbonyloxy, Cycloalkylcarbonyloxy, Heteroarylalkylcarbonyloxy, die Gruppe $-OSO_3H$ oder $-PO(OR)_2$, worin R Alkyl bedeutet, oder mit einer O-Schutzgruppe geschütztes Hydroxy oder unter physiologischen Bedingungen leicht spaltbares, durch eine Schutzgruppe substituiertes Amino oder zusammen mit einer cyclischen O-Schutzgruppe geschütztes Hydroxy bedeuten und die übrigen Symbole die oben angegebene Bedeutung besitzen, eine Verbindung der Formel I, worin $R^5$ und/oder $R^7$ Hydroxy oder Amino bedeuten und die übrigen Symbole die oben angegebene Bedeutung besitzen, mit einem gegebenenfalls durch Amino, Monoalkylamino, Dialkylamino, Alkanoylamino, Alkoxycarbonylamino, Alkylcarbonyloxy, Carboxy, Alkoxy oder Hydroxy ein- oder mehrfach substituierten Alkanoylierungsmittel oder einem Aroylierungs-, Arylalkanoylierungs-, Cycloalkanoylierungs- oder Heteroarylalkanoylierungsmittel, einem Sulfatierungs- oder Phosphorylierungsmittel oder mit einem eine N- und/oder O-Schutzgruppe oder cyclische O-Schutzgruppe bildenden Mittel umsetzt, oder

e) zur Herstellung einer Verbindung der Formel I, worin $R^2$ N-substituiertes, gegebenenfalls C-methyliertes Imidazol-2-yl oder N-substituiertes, gegebenenfalls C-methyliertes Imidazol-4-yl bedeutet und die übrigen Symbole die oben angegebene Bedeutung besitzen, eine Verbindung der Formel I, worin $R^2$ gegebenenfalls C-methyliertes Imidazol-2-yl oder gegebenenfalls C-methyliertes Imidazol-4-yl bedeutet und die übrigen Symbole die oben angegebene Bedeutung besitzen, mit einem geeigneten Acylierungs-, Alkylierungs-, Arylalkylierungs- bzw. Arylierungsmittel umsetzt, oder

f) zur Herstellung einer Verbindung der Formel I, worin A eine freie Aminogruppe enthält und/oder $R^2$ Imidazol-2-yl, Imidazol-4-yl, C-methyliertes Imidazol-2-yl, C-methyliertes Imidazol-4-yl oder Pyrazol-3-yl und/oder $R^5$ und/oder $R^7$ Amino bedeuten und/oder $R^8$ eine primäre oder sekundäre Aminogruppe enthält und die übrigen Symbole die oben angegebene Bedeutung besitzen, aus einer Verbindung der allgemeinen Formel

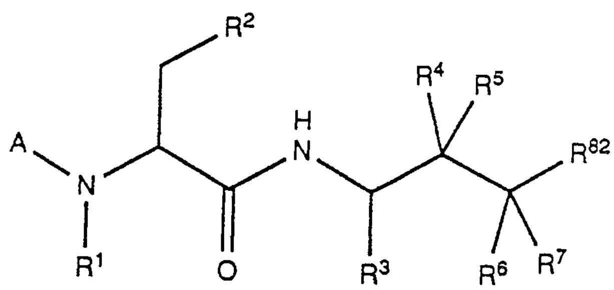

I a

worin $R^{82}$ die gleiche Bedeutung wie $R^8$ hat, jedoch noch zusätzlich N-geschütztes Cycloalkylaminomethyl bedeutet und die übrigen Symbole die oben angegebene Bedeutung besitzen, mit der Massgabe, dass mindestens einer von A, $R^2$, $R^5$, $R^7$ und $R^{82}$ eine N-Schutzgruppe enthält, die N-Schutzgruppe(n) abspaltet, und

g) erwünschtenfalls ein Gemisch diastereomerer Racemate in die diastereomeren Racemate oder optisch reinen Diastereomeren auftrennt, und/oder

h) erwünschtenfalls ein Diastereomerengemisch in die optisch reinen Diastereomeren auftrennt, und/oder

i) erwünschtenfalls eine erhaltene Verbindung in ein pharmazeutisch verwendbares Salz überführt.

2. Verfahren gemäss Anspruch 1, worin $R^2$ von Thioalkyl, Aminocarbonyl und Aminocarbonylmethyl, $R^3$ von Cycloalkylalkylthiomethyl, $R^{15}$ und $R^{17}$ je von Cycloalkylsulfonylalkyl, Cycloalkylalkylsulfonylalkyl, substituiertem Phenylsulfonylalkyl und Aminoalkylcarbonylalkyl, Y von Pyridylalanin und Z von 1-Azabicyclo[2.2.2]-octan-3-yl verschieden sind.

3. Verfahren gemäss Anspruch 1 oder 2, worin $R^1$ Wasserstoff, $R^2$ Imidazol-2-yl, Imidazol-4-yl, Thiazol-4-yl, Aminocarbonyl oder Aminocarbonylmethyl, $R^3$ Cyclohexylmethyl, substituiertes Cyclohexylmethyl oder Cyclohexenylmethyl, $R^4$ und $R^6$ je Wasserstoff, $R^5$ Hydroxy, Amino oder durch Amino monosubstituiertes

Alkylcarbonyloxy, $R^7$ Hydroxy, Amino, durch Amino monosubstituiertes Alkylcarbonyloxy, Azido oder Fluor, $R^8$ Alkylhydroxymethyl, Cycloalkylhydroxymethyl oder die Gruppe (b), oder $R^7$ und $R^8$ zusammen 2-Oxo-3-cycloalkyl-oxazolidin-5-yl, A die Gruppe (c) oder (e), $R^{11}$ Wasserstoff und $R^{12}$ und $R^{13}$ je Alkyl oder zusammen mit dem Kohlenstoffatom, an das sie gebunden sind, Cycloalkyl, $R^{14}$ Phenyl oder substituiertes Phenyl, $R^{15}$ Alkylcarbonylalkyl, Heterocycloalkylcarbonylalkyl, substituiertes Aminocarbonylalkyl, Alkylsulfonylalkyl, Cycloalkylsulfonylalkyl, Cycloalkylalkylsulfonylalkyl, substituiertes Aminoalkylcarbonylalkyl, Heterocycloalkylcarbonyloxy, Aminoalkylsulfonylalkyl oder substituiertes Aminosulfonylalkyl, Y den N-terminal mit Z verbundenen, bivalenten Rest von Phenylalanin oder O-Methyltyrosin und Z die Gruppe $R^a$-CO- bedeuten, worin $R^a$ einen unsubstituierten oder substituierten, gesättigten, gegebenenfalls mit Hydroxy und/oder Amino, Monoalkylamino, Dialkylamino, Alkanoylamino, Alkoxycarbonylamino, Arylalkoxycarbonylamino oder substituiertem Aminocarbonyl funktionalisierten aliphatischen Kohlenwasserstoffrest mit bis zu 10 Kohlenstoffatomen oder einen unsubstituierten oder substituierten heteraromatischen Kohlenwasserstoffrest mit bis zu 18 Kohlenstoffatomen bedeutet, insbesondere den einwertigen, über die Carboxylgruppe gebundenen Rest von Prolin, Pyroglutaminsäure, α-Methylalanin, Aminoäthylglycin, D-Alanin, β-Alanin oder β,β-Dimethylalanin.

4. Verfahren gemäss einem der Ansprüche 1-3, worin $R^1$ Wasserstoff, $R^2$ Imidazol-4-yl, $R^3$ Cyclohexylmethyl oder 4,4-Difluorcyclohexylmethyl, $R^4$ und $R^6$ je Wasserstoff, $R^5$ und $R^7$ je Hydroxy oder Aminomethylcarbonyloxy, $R^8$ die Gruppe (b), $R^{11}$ Wasserstoff, $R^{12}$ und $R^{13}$ zusammen mit dem Kohlenstoffatom, an das sie gebunden sind, Cyclopropyl oder Cyclobutyl, $R^{14}$ Phenyl und $R^{15}$ $C_1$-$C_4$-Alkylcarbonylmethyl, Heterocycloalkylcarbonylmethyl, substituiertes Aminocarbonylmethyl, $C_1$-$C_4$-Alkylsulfonylmethyl, $C_3$-$C_6$-Cycloalkylsulfonylmethyl, $C_3$-$C_6$-Cycloalkyl-$C_1$-$C_4$-alkylsulfonylmethyl, substituiertes Amino-$C_1$-$C_4$-alkylcarbonylmethyl, Heterocycloalkylcarbonyloxy, Amino-$C_1$-$C_4$-alkylsulfonylmethyl oder substituiertes Amino-$C_1$-$C_4$-alkylsul fonylmethyl bedeuten.

5. Verfahren gemäss Anspruch 2, dadurch gekennzeichnet, dass man (S)-α[(S)-α-[[[(2-Amino-1,1-dimethyläthyl)sulfonyl]methyl]hydrocinnamamido]-N-[(1S,2R,3S)-1-(cyclohexylmethyl)-3-cyclopropyl-2,3-dihydroxypropyl]imidazol-4-propionamid-dihydrochlorid herstellt.

6. Verfahren gemäss Anspruch 2, dadurch gekennzeichnet, dass man (S)-α-[(S)-α-[(tert-Butylsulfonyl)-methyl]hydrocinnamamido]-N-[(1S,2R,3S)-1-(cyclohexylmethyl)-3-cyclopropyl-2,3-dihydroxypropyl]-5-cyanimidazol-4-propionamid herstellt.

7. Verfahren gemäss Anspruch 2, dadurch gekennzeichnet, dass man (S)-α-[(S)-α-[(tert-Butylsulfonyl)-methyl]hydrocinnamamido]-N-[(1S,2R,3S)-3-cyclopropyl-1-[(4,4-difluorcyclohexyl)methyl]-2,3-dihydroxypropyl]imidazol-4-propionamid herstellt.

8. Verfahren gemäss Anspruch 2, dadurch gekennzeichnet, dass man (S)-α-((5)-α-[(tert-Butylsulfonyl)-methyl]hydrocinnamamido]-N-[(1S,2R,3S)-3-cyclopropyl-1-(p-fluorbenzyl)-2,3-dihydroxypropyl]imidazol-4-propionamid herstellt.

9. Verfahren gemäss Anspruch 2, dadurch gekennzeichnet, dass man (S)-α-[(S)-α-[[(2-Amino-2-methylpropyl)sulfonyl]methyl]hydrocinnamamido]-N-[(1S, 2R, 3S)-1-(cyclohexylmethyl)-3-cyclopropyl-2,3-dihydroxypropyl]imidazol-4-propionsäureamid-diacetat herstellt.

10. Verfahren zur Herstellung von Arzneimitteln, insbesondere zur Verwendung bei der Bekämpfung bzw. Verhütung von Bluthochdruck und Herzinsuffizienz, dadurch gekennzeichnet, dass man ein Aminosäurederivat der Formel I in Anspruch 1, in Form eines optisch reinen Diastereomeren, eines Diastereomerengemisches, eines diastereomeren Racemates oder eines Gemisches von diastereomeren Racematen, oder ein pharmazeutisch verwendbares Salz einer solchen Verbindung in eine galenische Darreichungsform bringt.

11. Verwendung eines Aminosäurederivates der Formel I in Anspruch 1, in Form eines optisch reinen Diastereomeren, eines Diastereomerengemisches, eines diastereomeren Racemates oder eines Gemisches von diastereomeren Racematen, oder eines pharmazeutisch verwendbaren Salzes einer solchen Verbindung zur Herstellung von Mitteln gegen Bluthochdruck und/oder Herzinsuffizienz.